# EUROPEAN PATENT APPLICATION

(11) **EP 4 008 716 A1**
(43) Date of publication of application: **08.06.2022**
(21) Application number: 20211807.1
(22) Date of filing: 04.12.2020
(51) Int. Cl.: C07D 333/28, C07D 333/38, C07D 239/91, C07D 495/04, C07D 417/12, C07D 409/06, C07C 233/00, C07D 295/15, C07D 333/78, C07D 333/68, C07D 409/12, A61P 35/00, A61K 31/381, A61K 31/517, A61K 31/519, A61K 31/495

(54) **NOVEL INHIBITORS OF INSULIN-LIKE GROWTH FACTOR 2 MRNA BINDING PROTEINS**

(71) Applicant: Martin-Luther-Universität Halle-Wittenberg, 06108 Halle (Saale) (DE)
(72) Inventor: Prof. Dr. HÜTTELMAIER, Stefan, 06114 Halle (Saale) (DE); MÜLLER, Simon, 06108 Halle (Saale) (DE); Dr. BLEY, Nadine, 06108 Halle (Saale) (DE); DAILEN, García Martínez, 06110 Halle (Saale) (DE); Dr. SIPPL, Wolfgang, 06114 Halle (Saale) (DE); GHAZY, Ehab, 06122 Halle (Saale) (DE)
(74) Representative: Maikowski & Ninnemann Patentanwälte Partnerschaft mbB

(57) **Abstract**

The presented invention provides compounds of formula (I), 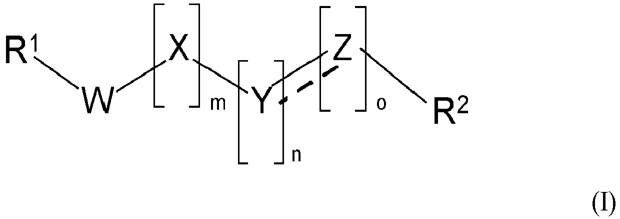 or a pharmaceutically acceptable salt, solvate or polymorph thereof, including all tautomers and stereoisomers thereof, wherein W, X, Y, Z, m, n and o are as defined herein. Said compounds of formula (I) are inhibitors of insulin-like growth factor 2 mRNA binding proteins (IGF2BP). Provided are novel inhibitors of IGF2BPs and processes for manufacturing the same. Efficacy of the new compounds is shown in solid cancer models.

## Description

### Field of the invention

The present invention is concerned with the non-covalent, reversible inhibition of insulin-like growth factor 2 mRNA binding proteins (IGF2BP). Provided are novel inhibitors of IGF2BPs and processes for manufacturing the same. Efficacy of the new compounds is shown in solid cancer models.

### Background of the invention

RNA-binding proteins (RBPs) are critical players in cancer progression and their expression correlates directly with poor patient prognosis. Due to their role in cancer, RBPs have attracted attention in drug development over the years (see US6004749A). Although targeting of RNA binding proteins remains challenging due to the mostly non-enzymatic nature of RNA-protein association, various small molecule inhibitors inhibiting protein-RNA association of pro-oncogenic RBPs (oncoRBPs), which in part promote stemness-like cell properties in cancer, has been reported. Prominent examples are inhibitors of the oncoRBPs IGF2BP1, LIN28B, and MSI2 (1-4).

The human IGF2 mRNA binding protein family (IGF2BPs) comprises a group of three canonical RBPs (5). Two members, IGF2BP1 and 3, are considered *bona fide* oncofetal RBPs, characterized by substantial expression during embryogenesis, residual expression in some stem and progenitor cells in adult life and severe *de novo* synthesis in various cancers (5). IGF2BP2 is upregulated in various cancers as well, but shows ubiquitous, although moderate expression in healthy adult tissues as well (5). IGF2BP1 is the most conserved and potent pro-oncogenic RBP of the IGF2BP family, promoting tumor cell vitality and invasion as well as tumor growth and metastasis in experimental mouse tumor models (6-13). The IGF2BP1-directed control of tumor cell properties largely settles on the mRNA-binding properties of the protein. RNA-association of IGF2BP1 results in the recruitment of transcripts in mRNPs impairing mRNA turnover, resulting in enhanced protein output (7,13,14). Collectively these findings suggest IGF2BP1, in particular its RNA-binding activity as a *bona fide* target for cancer therapy. The only to date reported small molecule inhibitor impairing IGF2BP1 mRNA-association and tumor cell vitality *in vitro* is BTYNB (3). In recent studies, we demonstrated that BTYNB impairs the mRNA stabilizing role of IGF2BP1 in various solid cancer models (15). In these studies, we furthermore reveal that IGF2BP1 is a conserved oncoRBP promoting tumor cell cycle proliferation and stemness-like properties by shortening the G1 cell cycle phase. This regulation settles on a post-transcriptional super-enhancement of E2F-driven gene expression by impairing downregulation of transcriptional regulators E2F1-3 and transcripts driven by these, e.g. the proliferation marker MKI67. BTYNB efficiently impairs this regulatory role of IGF2BP1 by interfering with IGF2BP1 target mRNA association. In Müller et al., we demonstrate that BTYNB impairs E2F1 and E2F-driven gene expression by interfering with m⁶A- (N⁶-methyladenosine) and 3'UTR (untranslated region) dependent stabilization of the E2F1 mRNA as well as other regulators of the G1/S cell cycle checkpoint, including CDK4/6 (15). In agreement, BTYNB shows additive or synergistic inhibitory activity with the CDK4/6 inhibitor Palbociclib (15). Consistent with a conserved pro-oncogenic role of IGF2BP1 in essentially all solid cancers (6,7,10,15), BTYNB shows inhibitory potential in a broad variety of solid cancer cell models in vitro and impairs tumor growth and metastatic spread in the peritoneum in ovarian cancer mouse models (15). However, like observed for other small molecule inhibitors of oncoRBPs, e.g. LIN28B inhibitors, the potency of inhibitors, as indicated by EC₅₀ (half maximal effective concentration) or ICso (half maximal inhibitory concentration) remain in the micromolar range and require improvement to increase compound potency and pave the way for clinical evaluation.

### Summary of the invention

In order to overcome the obstacles of the prior art, the invention provides a compound of formula (I) 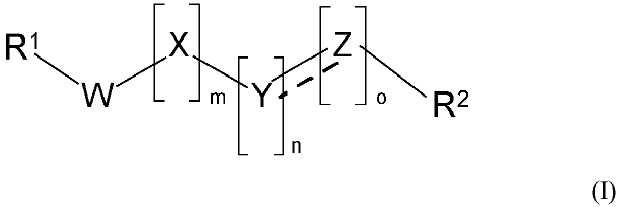 or a pharmaceutically acceptable salt, solvate or polymorph thereof, including all tautomers and stereoisomers thereof, wherein R¹, R², W, X, Y, Z, m, n and o are described herein.

The invention further provides methods for manufacturing the compounds formula (I).

In further embodiments, the invention relates to pharmaceutical compositions comprising at least one compound of formula (I) and to the use of the compounds of formula (I) and the pharmaceutical compositions comprising the same in methods for treating cancer.

### Detailed description of the invention

The "EC₅₀" value, which reflects the inhibitor concentration, which results in 50 % cell mortality in a cancer cell model.

The term "IGF2BP inhibitor" or "inhibitor of IGF2 mRNA binding proteins" is generally known to a person skilled in the art and means inhibitors, which inhibit the pharmacological activity of IGF2 mRNA binding proteins, especially the proliferative activity of IGF2BPs.

The term "IGF2BP activity" refers to binding of IGF2BPs to their pharmacological targets in a subject, thereby initiating the development of certain proliferative diseases.

### Potency of QC inhibition

In light of the correlation with IGF2BP inhibition, in preferred embodiments, the subject method and medical use utilize a compound of formula (I) with an EC₅₀ for IGF2BP inhibition of 10 µM or less, more preferably of 5 µM or less, even more preferably of 3 µM or less or 1 µM or less, or most preferably 0.8 µM or less, 0.6 µM or less or 0.4 µM or less. Thus, while the active agents are described herein, for convenience, as "IGF2BP inhibitors", it will be understood that such nomenclature is not intending to limit the subject of the invention to a particular mechanism of action.

### Molecular weight of IGF2BP inhibitors

In general, the IGF2BP inhibitors of the subject method or medical use will be small molecules, e.g., with molecular weights of 550 g/mole or less, 500 g/mole or less, 400 g/mole or less, preferably of 350 g/mole or less, and even more preferably of 300 g/mole or less and even of 250 g/mole or less.

The term "subject" as used herein, refers to an animal, preferably a mammal, most preferably a human, who has been the object of treatment, observation or experiment.

The term "therapeutically effective amount" as used herein, means that amount of active compound or pharmaceutical agent that elicits the biological or medicinal response in a tissue system, animal or human being sought by a researcher, veterinarian, medical doctor or other clinician, which includes alleviation of the symptoms of the disease or disorder being treated.

As used herein, the term "pharmaceutically acceptable" embraces both human and veterinary use: For example the term "pharmaceutically acceptable" embraces a veterinary acceptable compound or a compound acceptable in human medicine and health care.

Throughout the description and the claims the expression "alkyl", unless specifically limited, denotes a C₁₋₁₂ alkyl group, suitably a C₁₋₈ alkyl group, e.g. C₁₋₆ alkyl group, e.g. C₁₋₄ alkyl group. Alkyl groups may be straight chain or branched. Suitable alkyl groups include, for example, methyl, ethyl, propyl (e.g. n-propyl and isopropyl), butyl (e.g n-butyl, iso-butyl, sec-butyl and tert-butyl), pentyl (e.g. n-pentyl), hexyl (e.g. n-hexyl), heptyl (e.g. n-heptyl) and octyl (e.g. n-octyl). The expression "alk", for example in the expressions "alkoxy", "haloalkyl" and "thioalkyl" should be interpreted in accordance with the definition of "alkyl". Exemplary alkoxy groups include methoxy, ethoxy, propoxy (e.g. n-propoxy), butoxy (e.g. n-butoxy), pentoxy (e.g. n-pentoxy), hexoxy (e.g. n-hexoxy), heptoxy (e.g. n-heptoxy) and octoxy (e.g. n-octoxy). Exemplary thioalkyl groups include methylthio-. Exemplary haloalkyl groups include fluoroalkyl e.g. CF₃, fluoroethyl, fluropropyl, fluorobutyl, difluoroethyl, difluoropropyl and difluorobutyl.

The expression "alkylene" denotes a chain of formula -(CH₂)ₙ- wherein n is an integer e.g. 2-5, unless specifically limited.

The expression "cycloalkyl" or "carbocyclyl", unless specifically limited, denotes a C₃₋₁₀ cycloalkyl group (i.e. 3 to 10 ring carbon atoms), more suitably a C₃₋₈ cycloalkyl group, e.g. a C₃₋₆ cycloalkyl group. Exemplary cycloalkyl groups include cyclopropyl, cyclobutyl, cyclopentyl, cyclohexyl, cycloheptyl and cyclooctyl. A most suitable number of ring carbon atoms is three to six.

The expression "heterocyclyl", unless specifically limited, refers to a carbocyclyl group wherein one or more (e.g. 1, 2 or 3) ring atoms are replaced by heteroatoms selected from N, S and O. A specific example of a heterocyclyl group is a cycloalkyl group (e.g. cyclopentyl or more particularly cyclohexyl) wherein one or more (e.g. 1, 2 or 3, particularly 1 or 2, especially 1) ring atoms are replaced by heteroatoms selected from N, S or O. Exemplary heterocyclyl groups containing one hetero atom include pyrrolidine, tetrahydrofuran and piperidine, and exemplary heterocyclyl groups containing two hetero atoms include morpholine, piperazine, dioxolane and dioxane. A further specific example of a heterocyclyl group is a cycloalkenyl group (e.g. a cyclohexenyl group) wherein one or more (e.g. 1, 2 or 3, particularly 1 or 2, especially 1) ring atoms are replaced by heteroatoms selected from N, S and O. Examples of such a group are piperazinyl (e.g. piperazine-1-yl-) and piperidinyl.

The expression "aryl", unless specifically limited, denotes a C₆₋₁₂ aryl group, suitably a C₆₋₁₀ aryl group, more suitably a C₆₋₈ aryl group. Aryl groups will contain at least one aromatic ring (e.g. one, two or three rings). An example of a typical aryl group with one aromatic ring is phenyl. An example of a typical aryl group with two aromatic rings is naphthyl.

The expression "heteroaryl", unless specifically limited, denotes an aryl residue, wherein one or more (e.g. 1, 2, 3, or 4, suitably 1, 2 or 3) ring atoms are replaced by heteroatoms selected from N, S and O, or else a 5-membered aromatic ring containing one or more (e.g. 1, 2, 3, or 4, suitably 1, 2 or 3) ring atoms selected from N, S and O. Exemplary monocyclic heteroaryl groups having one heteroatom include: five membered rings (e.g. pyrrole, furan, thiophene); and six membered rings (e.g. pyridine, such as pyridin-2-yl, pyridin-3-yl and pyridin-4-yl). Exemplary monocyclic heteroaryl groups having two heteroatoms include: five membered rings (e.g. pyrazole, oxazole, isoxazole, thiazole, isothiazole, imidazole, such as imidazol-1-yl, imidazol-2-yl imidazol-4-yl); six membered rings (e.g. pyridazine, pyrimidine, pyrazine). Exemplary monocyclic heteroaryl groups having three heteroatoms include: 1,2,3-triazole and 1,2,4-triazole. Exemplary monocyclic heteroaryl groups having four heteroatoms include tetrazole. Exemplary bicyclic heteroaryl groups include: indole (e.g. indol-6-yl), benzofuran, benzthiophene, quinoline, isoquinoline, indazole, benzimidazole, benzthiazole, quinazoline and purine.

The expression "-alkylaryl", unless specifically limited, denotes an aryl residue which is connected via an alkylene moiety e.g. a C₁₋₄alkylene moiety.

The expression "-alkylheteroaryl", unless specifically limited, denotes a heteroaryl residue which is connected via an alkylene moiety e.g. a C₁₋₄alkylene moiety.

The term "halogen" or "halo" comprises fluorine or fluoro (F), chlorine or chloro (Cl) and bromine or bromo (Br).

### Stereoisomers:

All possible stereoisomers of the claimed compounds are included in the present invention.

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention.

### Preparation and isolation of stereoisomers:

Where the processes for the preparation of the compounds according to the invention give rise to a mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their components enantiomers by standard techniques, such as the formation of diastereomeric pairs by salt formation with an optically active acid, such as (-)-di-p-toluoyl-d-tartaric acid and/or (+)-di-p-toluoyl-1-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

### Pharmaceutically acceptable salts:

In view of the close relationship between the free compounds and the compounds in the form of their salts or solvates, whenever a compound is referred to in this context, a corresponding salt, solvate or polymorph is also intended, provided such is possible or appropriate under the circumstances.

Salts and solvates of the compounds of formula (I) and physiologically functional derivatives thereof which are suitable for use in medicine are those wherein the counter-ion or associated solvent is pharmaceutically acceptable. However, salts and solvates having non-pharmaceutically acceptable counter-ions or associated solvents are within the scope of the present invention, for example, for use as intermediates in the preparation of other compounds and their pharmaceutically acceptable salts and solvates.

Suitable salts according to the invention include those formed with both organic and inorganic acids or bases. Pharmaceutically acceptable acid addition salts include those formed from hydrochloric, hydrobromic, sulfuric, nitric, citric, tartaric, phosphoric, lactic, pyruvic, acetic, trifluoroacetic, triphenylacetic, sulfamic, sulfanilic, succinic, oxalic, fumaric, maleic, malic, mandelic, glutamic, aspartic, oxaloacetic, methanesulfonic, ethanesulfonic, arylsulfonic (for example p-toluenesulfonic, benzenesulfonic, naphthalenesulfonic or naphthalenedisulfonic), salicylic, glutaric, gluconic, tricarballylic, cinnamic, substituted cinnamic (for example, phenyl, methyl, methoxy or halo substituted cinnamic, including 4-methyl and 4-methoxycinnamic acid), ascorbic, oleic, naphthoic, hydroxynaphthoic (for example 1- or 3-hydroxy-2-naphthoic), naphthaleneacrylic (for example naphthalene-2-acrylic), benzoic, 4 methoxybenzoic, 2- or 4-hydroxybenzoic, 4-chlorobenzoic, 4-phenylbenzoic, benzeneacrylic (for example 1,4-benzenediacrylic), isethionic acids, perchloric, propionic, glycolic, hydroxyethanesulfonic, pamoic, cyclohexanesulfamic, salicylic, saccharinic and trifluoroacetic acid. Pharmaceutically acceptable base salts include ammonium salts, alkali metal salts such as those of sodium and potassium, alkaline earth metal salts such as those of calcium and magnesium and salts with organic bases such as dicyclohexylamine and N-methyl-D-glucamine.
All pharmaceutically acceptable acid addition salt forms of the compounds of the present invention are intended to be embraced by the scope of this invention.

### Polymorph crystal forms:

Furthermore, some of the crystalline forms of the compounds may exist as polymorphs and as such are intended to be included in the present invention. In addition, some of the compounds may form solvates with water (i.e. hydrates) or common organic solvents, and such solvates are also intended to be encompassed within the scope of this invention. The compounds, including their salts, can also be obtained in the form of their hydrates, or include other solvents used for their crystallization.

### Prodrugs:

The present invention further includes within its scope prodrugs of the compounds of this invention. In general, such prodrugs will be functional derivatives of the compounds which are readily convertible in vivo into the desired therapeutically active compound. Thus, in these cases, the methods of treatment of the present invention, the term "administering" shall encompass the treatment of the various disorders described with prodrug versions of one or more of the claimed compounds, but which converts to the above specified compound in vivo after administration to the subject. Conventional procedures for the selection and preparation of suitable prodrug derivatives are described, for example, in "Design of Prodrugs", ed. H. Bundgaard, Elsevier, 1985.

### Protective Groups:

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, fully incorporated herein by reference. The protecting groups may be removed at a convenient subsequent stage using methods known from the art.

A protecting group or protective group is introduced into a molecule by chemical modification of a functional group in order to obtain chemoselectivity in a subsequent chemical reaction. Protecting groups are e.g. alcohol protecting groups, amine protecting groups, carbonyl protecting groups, carboxylic acid protecting groups and phosphate protecting groups.

Examples for alcohol protecting groups are acetyl (Ac), benzoyl (Bz), benzyl (Bn, Bnl) β-methoxyethoxymethyl ether (MEM), mimethoxytrityl [bis-(4-methoxyphenyl)phenylmethyl, DMT], methoxymethyl ether (MOM), methoxytrityl [(4-methoxyphenyl)diphenylmethyl, MMT), p-methoxybenzyl ether (PMB), methylthiomethyl ether, pivaloyl (Piv), tetrahydropyranyl (THP), trityl (triphenylmethyl, Tr), silyl ethers (such as trimethylsilyl ether (TMS), tert-butyldimethylsilyl ether (TBDMS), tert-butyldimethylsilyloxymethyl ether (TOM), and triisopropylsilyl ether (TIPS)); methyl ethers and ethoxyethyl ethers (EE). Suitable amine protecting groups are selected from carbobenzyloxy (Cbz), p-methoxybenzyl carbonyl (Moz or MeOZ), tert-butyloxycarbonyl (BOC), 9-fluorenylmethyloxycarbonyl (FMOC), acetyl (Ac), benzoyl (Bz), benzyl (Bn), p-methoxybenzyl (PMB), 3,4-dimethoxybenzyl (DMPM), p-methoxyphenyl (PMP), tosyl (Ts), and other sulfonamides (Nosyl & Nps).
Suitable carbonyl protecting groups are selected from acetals and ketals, acylals and dithianes. Suitable carboxylic acid protecting groups are selected from methyl esters, benzyl esters, tert-butyl esters, silyl esters, orthoesters, and oxazoline.
Examples for phosphate protecting groups are 2-cyanoethyl and methyl (Me).

As used herein, the term "composition" is intended to encompass a product comprising the claimed compounds in the therapeutically effective amounts, as well as any product which results, directly or indirectly, from combinations of the claimed compounds.

### Carriers and Additives for galenic formulations:

Thus, for liquid oral preparations, such as for example, suspensions, elixirs and solutions, suitable carriers and additives may advantageously include water, glycols, oils, alcohols, flavoring agents, preservatives, coloring agents and the like; for solid oral preparations such as, for example, powders, capsules, gelcaps and tablets, suitable carriers and additives include starches, sugars, diluents, granulating agents, lubricants, binders, disintegrating agents and the like.

Carriers, which can be added to the mixture, include necessary and inert pharmaceutical excipients, including, but not limited to, suitable binders, suspending agents, lubricants, flavorants, sweeteners, preservatives, coatings, disintegrating agents, dyes and coloring agents.

Soluble polymers as targetable drug carriers can include polyvinylpyrrolidone, pyran copolymer, polyhydroxypropylmethacrylamidephenol, polyhydroxyethylaspartamide-phenol, or polyethyleneoxidepolyllysine substituted with palmitoyl residue. Furthermore, the compounds of the present invention may be coupled to a class of biodegradable polymers useful in achieving controlled release of a drug, for example, polyactic acid, polyepsilon caprolactone, polyhydroxy butyeric acid, polyorthoesters, polyacetals, polydihydropyrans, polycyanoacrylates and cross-linked or amphipathic block copolymers of hydrogels.

Suitable binders include, without limitation, starch, gelatin, natural sugars such as glucose or betalactose, corn sweeteners, natural and synthetic gums such as acacia, tragacanth or sodium oleate, sodium stearate, magnesium stearate, sodium benzoate, sodium acetate, sodium chloride and the like.

Disintegrators include, without limitation, starch, methyl cellulose, agar, bentonite, xanthan gum and the like.
The invention provides a compound of formula (I) 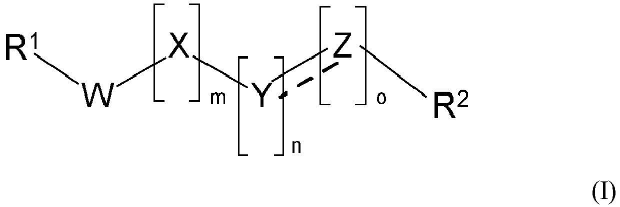 or a pharmaceutically acceptable salt, solvate or polymorph thereof, including all tautomers and stereoisomers thereof,
wherein:
W is selected from the group consisting of NH, O, and CR³R⁴;
Y is selected from the group consisting of C=O, CR³R⁴ and CR³;
Z is CR³ or CR³R⁴,
any of m, n, and o is independently selected from 0 and 1;
R¹ is selected from the group consisting of aryl, heteroaryl and heteroaryl fused to carbocyclyl;
   wherein R¹ may be optionally substituted by one or more groups selected from the group consisting of alkyl, alkoxy, haloalkyl, thioalkyl, carbamoyl, aryl, alkylaryl, heteroaryl fused to aryl, heteroaryl fused to heteroaryl and halo; wherein said carbamoyl group may optionally be further substituted by alkyl and wherein said thioalkyl and heteroaryl groups may optionally be further substituted by one or more groups selected from oxo, alkylaryl, and heteroaryl.
R² is selected from the group consisting of alkyl, heteroalkyl, aryl, aryl fused to heterocyclyl, heteroaryl and heterocyclyl;
   wherein R² may be optionally substituted by one or more groups selected from the group consisting of alkyl, alkoxy, aryl, alkylaryl, alkoxy-aryl, aryl-amido, alkoxy-heteroaryl, halo and nitro, wherein said aryl and heteroaryl groups may optionally be further substituted by one or more groups selected from halo and alkoxy;
R³ is hydrogen;
R⁴ is hydrogen or alkyl, such as methyl;

In a preferred embodiment, when R¹ is aryl or heteroaryl, R1 is substituted by one or more substituents independently selected from the group consisting of benzyl, alkyl, and amido.
More preferably, when R¹ is aryl, said aryl group is further substituted by amido.
Furthermore preferably, when R¹ is aryl, said aryl group is further substituted by amido and alkyl, such as isopropyl.
Furthermore preferably, when R¹ is aryl, said aryl group is further substituted by amido and benzyl.
Furthermore preferably, when R¹ is heteroaryl, said heteroaryl group is further substituted by amido.
Furthermore preferably, when R¹ is heteroaryl, said heteroaryl group is further substituted by amido and alkyl, such as isopropyl.
Furthermore preferably, when R¹ is heteroaryl, said heteroaryl group is further substituted by amido and benzyl.
Most preferably, said aryl is phenyl. Further most preferably, said heteroaryl is thiophenyl.

In a further preferred embodiment, the bond between Y and Z is a double bond, when m, n and o are each 1.

According to preferred embodiments, the definitions of W, X, Y, Z, m, n and o in the compounds of formula (I) are selected from the group consisting of:
i. W is O, X is CH₂, m is 1, n is 0 and o is 0.
ii. W is CH₂, X is O, m is 1, n is 0 and o is 0.
iii. W is CR³R⁴, X is S, m is 1, n is 0 and o is 0, wherein R³ is hydrogen and R⁴ is methyl.
iv. W is CH₂, X is 0, m is 1, n is 0 and o is 0.
v. W is NH, X is C=O, m is 1, n is 0 and o is 0.
vi. W is O, X is CH₂, Y is C=O, m is 1, n is 1 and o is 0.
vii. W is NH, X is C=O, Y is CH₂, m is 1, n is 1 and o is 0.
viii. W is NH, X is C=O, Y is CH, Z is CH, m is 1, n is 1 and o is 1.

More preferably, the compound of formula (I) is one of formulae (Ia) to (Ih) or a pharmaceutically acceptable salt form, solvate or polymorph thereof, including all tautomers and stereoisomers thereof:

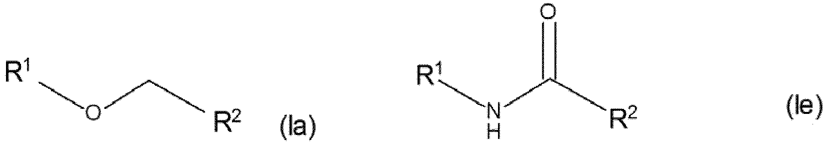

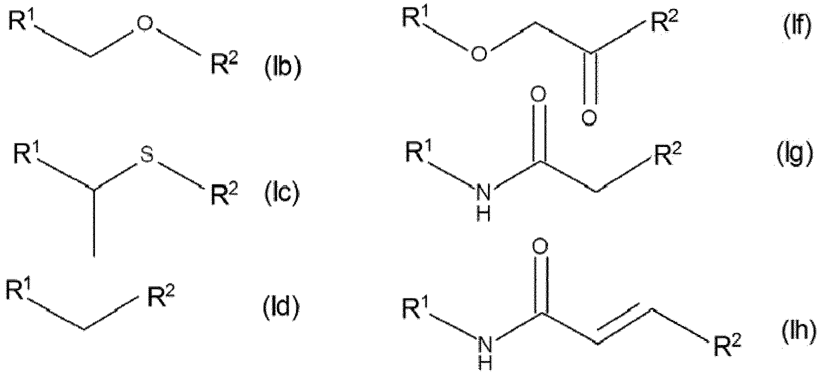

Most preferably, the compound of formula (I) is one of formulae (Ia, Ie, If and Ig) or a pharmaceutically acceptable salt form, solvate or polymorph thereof, including all tautomers and stereoisomers thereof:

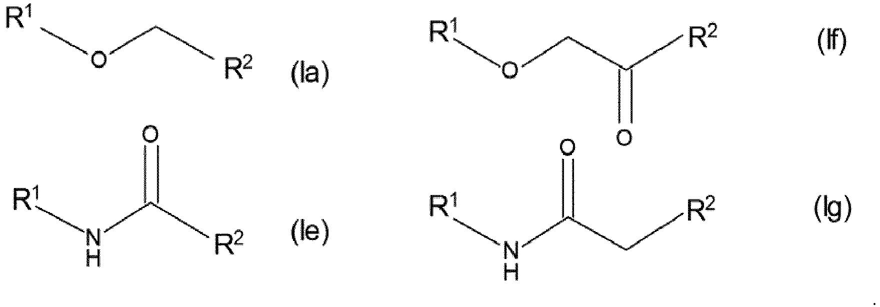

In an especially preferred embodiment, the compound of formula (I) is one of formulae (Ie) and (Ig) or a pharmaceutically acceptable salt form, solvate or polymorph thereof, including all tautomers and stereoisomers thereof: 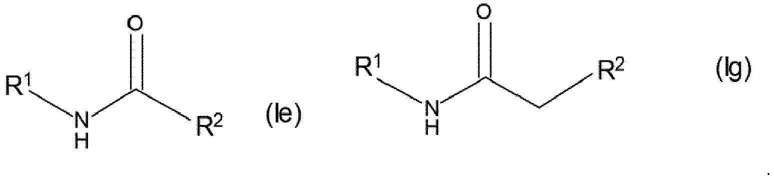 R¹ is suitably selected from the group consisting of phenyl, thiophenyl, thiadiazolyl, such as 1,3,4-thiadiazolyl, 4,5,6,7-tetrahydro-1-benzothiophene, thieno[2,3-d]pyrimidin-4(3H)-one and 5,6-dihydro-4H-cyclopenta[b]thiophene, which groups may optionally be further substituted as described above.

Preferably, R¹ is thiophenyl, phenyl, 4,5,6,7-tetrahydro-1-benzothiophene, any of which may optionally be further substituted by one or more groups selected from isopropyl, carbamoyl, methylcarbamoyl, chloro, benzyl and 4(3H)-quinazolinone, thieno[2,3-d]pyrimidinone which may further be substituted by one or more of methoxy and benzyl.

In more preferred are compounds of formula (I), is selected from the group consisting of benzamide, 5-chlorobenzamide, 3-(aminocarbonyl)-5-benzyl-2-thienyl, phenyl-4(3H)-quinazolinone, 3-methoxyphenyl-4(3H)-quinazolinone, 6-benzyl-2-(4-methoxyphenyl)thieno[2,3-d]pyrimidin-4(3H)-one, 4,5,6,7-tetrahydro-1-benzothiophene-3-carboxamide, 5-isopropyl-3-thiophenecarboxamide, 2-(methylcarbamoyl)phenyl and 6-methylthieno[2,3-d]pyrimidin-4(3H)-one.

Most preferred are compounds of formula (I), wherein R¹ is selected from the group consisting of benzamide which may optionally be further substituted as desctibed above, thiophene-3-carboxamide which may be optionally further substituted as described above, 3-(aminocarbonyl)-5-benzyl-2-thienyl and 3-methoxyphenyl-4(3H)-quinazolinone.

R² is suitably selected from the group consisting of phenyl, benozodioxole, piperazinyl, furanyl and thiophenyl, which groups may optionally be further substituted as claimed in claim 1.

Preferably, R² is selected from the group consisting of phenyl, 1,3- benzodioxole, piperazin-1-yl, furan-2-yl, thiophen-2-yl and thiophen-3-yl, which groups may optionally be substituted by one or more groups selected from alkoxy, aryl, halo, nitro, alkylaryl, alkoxyaryl, wherein said aryl groups may optionally be further substituted by one or more halo groups.

More preferably, R² is selected from the group consisting of 3-[(2,4-dichlorophenoxy)methyl]phenyl, 2-fluorobenzyl, 3-(2,4-dichlorophenoxymethyl)-4-methoxyphenyl, 3-(3-chlorophenoxymethyl)-4-methoxy-phenyl, 3-(2,3-dichlorophenoxymethyl)-4-methoxy-phenyl, 3-(2-chlorophenoxymethyl)-4-methoxy-phenyl, 2-nitrophenyl, 5-[(2,4-dichlorophenoxy)methyl]thiophen-2-yl, thiophen-2-yl, 3-chlorophenoxy-benzyl, 4-(3,4-dichlorobenzyl)piperazin-1-yl, 2,4-dichlorobenzyl-1-piperazinyl, 3-(4-chlorophenoxymethyl)-4-methoxy-phenyl, 3-(2-chlorophenoxymethyl)-4-methoxy-phenyl, 4-methoxyphenyl, thiophen-2-yl, (4-bromophenoxy)-benzyl, 1,3-benzodioxole, 5-chloro-2-thiophenyl, 5-methyl-2-furanyl, 4-(benzyloxy)-3-methoxyphenyl, 3,4-dichlorophenyl, 5-ethyl-3-thiophenyl, 4-methoxy-phenyl, 5-methyl-2-thiophenyl, phenyl, 3-(2,6-dichlorophenoxymethyl)-4-methoxyphenyl, 4,5-dimethyl-thiophen-2-yl, 4-bromophenyl, 4-chlorobenzyl-1-piperazinyl, 2-chloro-4-fluorobenzyl-1-piperazinyl, 3-[(2-bromophenoxy)methyl]phenyl, 4-ethyl-5-methyl-3-thiophenyl, 3,5-dichlorophenyl, 2-thienylcarbonyl-piperidine, 3-chloro-4-methoxyphenyl, 2,6-dichlorobenzyl-1-piperazinyl, 3-[(4-bromophenoxy)methyl]phenyl and 2-chlorobenzyl-1-piperazinyl.

Preferred compounds of formula (I) that show an EC₅₀ of 10 µM or below, more preferably of 5 µM or below, most preferably of 1 µM or below, in the call-based proliferation test, are compounds of formula (II) or a pharmaceutically acceptable salt form, solvate or polymorph thereof, including all tautomers and stereoisomers thereof: 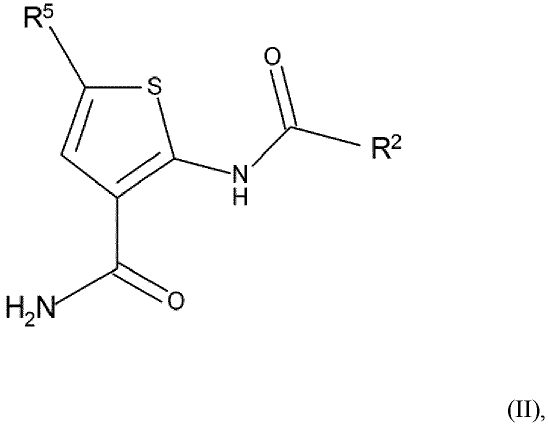 wherein R² is defined in one of the preceding claims and R⁵ is selected from hydrogen, benzyl and alkyl.
In a preferred embodiment, R⁵ is hydrogen.
In a further preferred embodiment, R⁵ is benzyl.
In yet a further preferred embodiment, R⁵ is alkyl, most preferably isopropyl.

Further preferred compounds of formula (I) that show an EC₅₀ of 10 µM or below, more preferably of 5 µM or below, most preferably of 1 µM or below, in the call-based proliferation test, are compounds of formula (IIa) or a pharmaceutically acceptable salt form, solvate or polymorph thereof, including all tautomers and stereoisomers thereof: 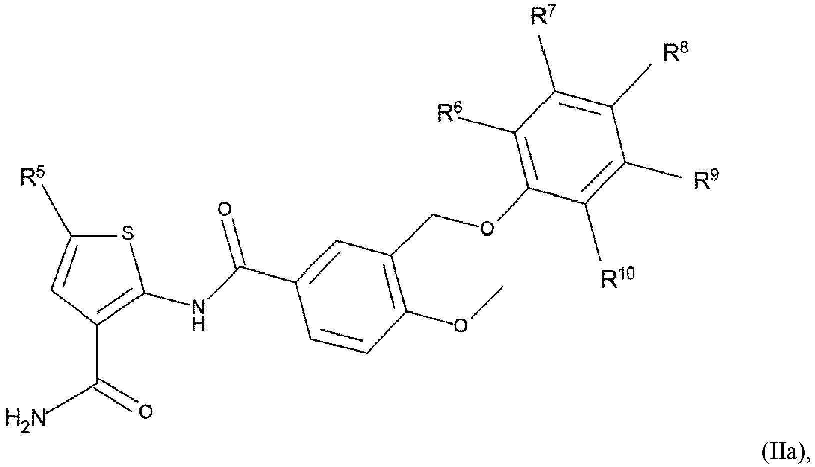 wherein
R⁵ is selected from hydrogen, benzyl and alkyl, such as isopropyl; and
R⁶, R⁷, R⁸, R⁹, and R¹⁰ are independently selected from the group consisting of hydrogen, nitro and halo, such as bromo, chloro or fluoro.
In a preferred embodiment, R⁵ is benzyl, R⁶ is halo, preferably chloro, and R⁷, R⁸, R⁹ and R¹⁰ are hydrogen.
In a further preferred embodiment, R⁵ is benzyl, R⁶ and R⁷ are halo, preferably chloro, and R⁸, R⁹ and R¹⁰ are hydrogen.
In a further preferred embodiment, R⁵ is alkyl, preferably isopropyl, R⁶ is halo, preferably chloro, and R⁷, R⁸, R⁹ and R¹⁰ are hydrogen.
In a further preferred embodiment, R⁵ is benzyl, R⁶ and R⁸ are halo, preferably chloro, and R⁷, R⁹ and R¹⁰ are hydrogen.
In a further preferred embodiment, R⁵ is benzyl, R⁶ is halo, preferably chloro, and R⁷, R⁸, R⁹ and R¹⁰ are hydrogen.
In a further preferred embodiment, R⁵ is benzyl, R⁷ is halo, preferably chloro, and R⁶, R⁸, R⁹ and R¹⁰ are hydrogen.
In a further preferred embodiment, R⁵ is benzyl, R⁸ is halo, preferably chloro, and R⁶, R⁷, R⁹ and R¹⁰ are hydrogen.

In a further preferred embodiment, R⁵ is benzyl, R⁶ and R¹⁰ are halo, preferably chloro, and R⁷, R⁸ and R⁹ are hydrogen.
In a further preferred embodiment, R⁵ is alkyl, preferably isopropyl, R⁸ is halo, preferably bromo, and R⁶, R⁷, R⁹ and R¹⁰ are hydrogen.

Further preferred compounds of formula (I) that show an EC₅₀ of 10 µM or below, more preferably of 5 µM or below, most preferably of 1 µM or below, in the call-based proliferation test, are compounds of formula (III) or a pharmaceutically acceptable salt form, solvate or polymorph thereof, including all tautomers and stereoisomers thereof: 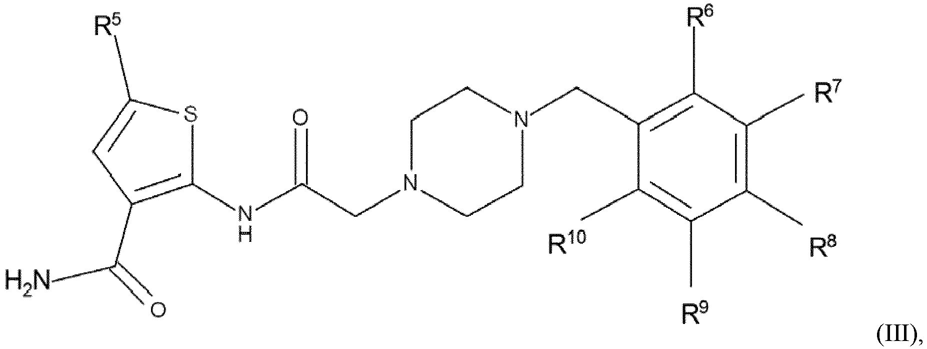 wherein
R⁵ is selected from hydrogen and benzyl; and
R⁶, R⁷, R⁸, R⁹, and R¹⁰ are independently selected from the group consisting of hydrogen and halo, such as bromo, chloro or fluoro.
In a preferred embodiment, R⁵ is benzyl, R⁶ is halo, preferably choro, and R⁷, R⁸, R⁹, and R¹⁰ are hydrogen.
In a further preferred embodiment, R⁵ is benzyl, R⁶, R⁹, and R¹⁰ are hydrogen, and R⁷ and R⁸ are both halo, preferably choro.
In a further preferred embodiment, R⁵ is hydrogen, R⁶, R⁹, and R¹⁰ are hydrogen, and R⁷ and R⁸ are both halo, preferably choro.

Further preferred compounds of formula (I) that show an EC₅₀ of 10 µM or below, more preferably of 5 µM or below, most preferably of 1 µM or below, in the call-based proliferation test, are compounds of formula (IV) or a pharmaceutically acceptable salt form, solvate or polymorph thereof, including all tautomers and stereoisomers thereof: 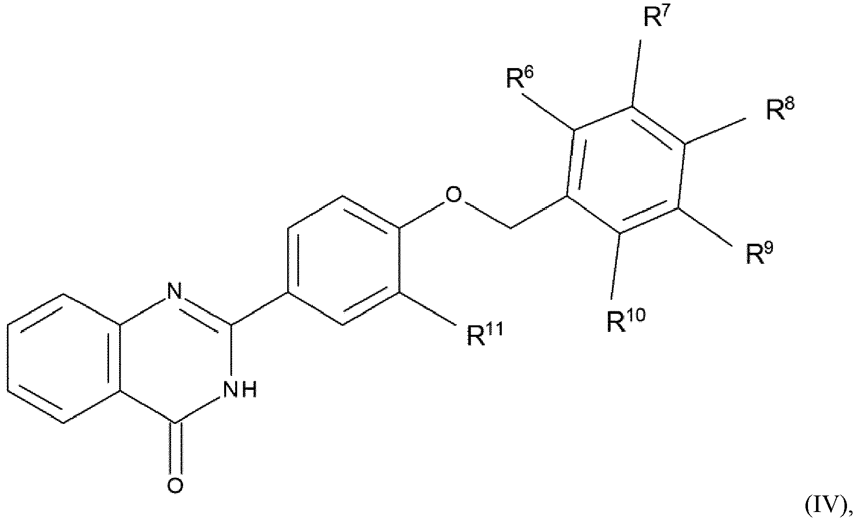 wherein
R⁶, R⁷, R⁸, R⁹, and R¹⁰ are independently selected from the group consisting of hydrogen, nitro and halo, such as bromo, chloro or fluoro; and
R¹¹ is selected from hydrogen and alkoxy.
In a preferred embodiment, R¹¹ is methoxy, R¹ to R⁹ are hydrogen and R¹⁰ nitro.
In a preferred embodiment, R¹¹ is hydrogen, R¹ to R⁹ are hydrogen, R¹⁰ is halo, preferably fluoro,
Further preferred compounds of formula (I) that show an EC₅₀ of 10 µM or below, more preferably of 5 µM or below, most preferably of 1 µM or below, in the call-based proliferation test, are compounds of formula (V) or a pharmaceutically acceptable salt form, solvate or polymorph thereof, including all tautomers and stereoisomers thereof: 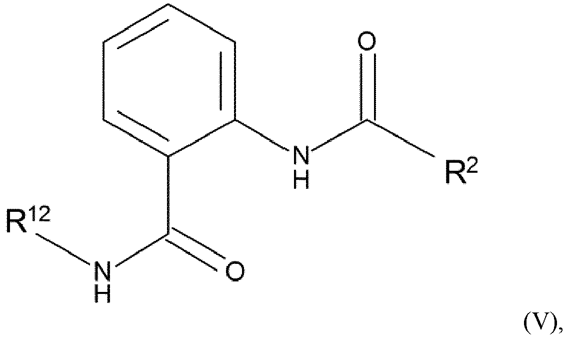 wherein
R² is as defined in claim 1; and
R¹² is hydrogen or alkyl.
R¹² is methyl and R² is 4,5-dimethyl-thiophen-2-yl.
In a further preferred embodiment, R¹² is hydrogen and R² is 2-thienylcarbonyl-piperidine.
In a further preferred embodiment, R¹² is hydrogen and R² is 3,4-dichlorophenyl.
In a further preferred embodiment, R¹² is hydrogen and R² is (3-chlorophenoxy)benzyl.
In a further preferred embodiment, R¹² is hydrogen and R² is 3-(2-chlorophenoxymethyl)-4-methoxy-phenyl.

Further preferred compounds of formula (I) that show an EC₅₀ of 10 µM or below, more preferably of 5 µM or below, most preferably of 1 µM or below, in the call-based proliferation test, are compounds of formula (VI) or a pharmaceutically acceptable salt form, solvate or polymorph thereof, including all tautomers and stereoisomers thereof: 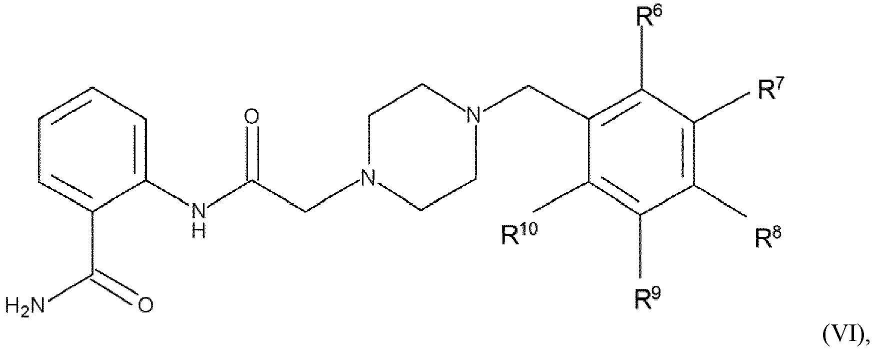 wherein
R⁶, R⁷, R⁸, R⁹, and R¹⁰ are independently selected from the group consisting of hydrogen and and halo, such as bromo, chloro or fluoro.
In a preferred embodiment, R⁶, R⁹ and R¹⁰ are hydrogen and R⁷ and R⁸ are halo, preferably chloro.
In a further preferred embodiment, R⁷, R⁹ and R¹⁰ are hydrogen and R⁶ and R⁸ are halo, preferably chloro.
In a further preferred embodiment, R⁷, R⁸ and R⁹ are hydrogen and R⁶ and R¹⁰ are halo, preferably chloro.
In a further preferred embodiment, R⁶, R⁷, R⁹ and R¹⁰ are hydrogen and R⁸ is halo, preferably chloro.

In a further preferred embodiment, R⁷, R⁸, R⁹ and R¹⁰ are hydrogen and R⁶ is halo, preferably chloro.
In a further preferred embodiment, R⁷, R⁹ and R¹⁰ are hydrogen and R⁶ and R⁸ are halo, preferably chloro or fluoro. Most preferably, R⁶ is chloro and R⁸ is fluoro.

Further preferred compounds of formula (I) that show an EC₅₀ of 10 µM or below, more preferably of 5 µM or below, most preferably of 1 µM or below, in the call-based proliferation test, are compounds of formula (VII) or a pharmaceutically acceptable salt form, solvate or polymorph thereof, including all tautomers and stereoisomers thereof: 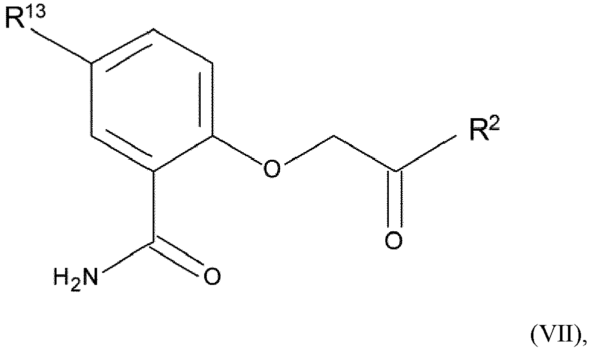 wherein
R² is as defined in claim 1; and
R¹³ is hydrogen or halo.
In a preferred embodiment, R¹³ is halo, preferably chloro, R² is thiophenyl substituted by halo, preferably 5-chlorothiophen-2-yl. In a preferred embodiment, R¹³ is hydrogen and R² is phenyl substituted by halo, preferably 4-bromophenyl.

A suitable compound of formula (I) that shows an EC₅₀ of 10 µM or below in the call-based proliferation test, is a compound or a pharmaceutically acceptable salt form, solvate or polymorph thereof, including all tautomers and stereoisomers thereof, selected from the group consisting of:
2-[(5-chlorothiophen-2-yl)methoxy]benzamide;
N-[2-(aminocarbonyl)phenyl]-5-methyl-2-thiophenecarboxamide;
2-{3-methoxy-4-[(2-nitrobenzyl)oxy]phenyl}-4(3H)-quinazolinone;
2-{4-[(2-fluorobenzyl)oxy]phenyl}-4(3H)-quinazolinone;
6-benzyl-2-{4-methoxy-3-[(4-methoxyphenoxy)methyl]phenyl}thieno[2,3-d]pyrimidin-4(3H)-one;
N-(5-{[1-(4-oxo-3,4-dihydro-2-quinazolinyl)ethyl]thio}-1,3,4-thiadiazol-2-yl)benzamide;
2-benzyl-6-methylthieno[2,3-d]pyrimidin-4(3H)-one;
4,5-dimethyl-N-[2-(methylcarbamoyl)phenyl]thiophene-2-carboxamide;
5-chloro-2-[2-(5-chlorothiophen-2-yl)-2-oxoethoxy]benzamide;
N-[3-(aminocarbonyl)-2-thienyl]-2-thiophenecarboxamide;
N-[2-(aminocarbonyl)phenyl]-1-(2-thienylcarbonyl)-4-piperidinecarboxamide;
N-[2-(aminocarbonyl)phenyl]-3,4-dichlorobenzamide;
N-(2-carbamoylphenyl)-5-chlorothiophene-2-carboxamide;
N-(2-carbamoylphenyl)benzamide;
2-{[(5-ethyl-3-thienyl)carbonyl]amino}-3-thiophenecarboxamide;
N-[2-(aminocarbonyl)phenyl]-3 -chloro-4-methoxybenzamide;
2-({3-[(3-chlorophenoxy)methyl]benzoyl}amino)benzamide;
N-[2-(aminocarbonyl)phenyl]-3,5-dichlorobenzamide;
2-[2-(4-bromophenyl)-2-oxoethoxy]benzamide;
2-({[4-(3,4-dichlorobenzyl)-1-piperazinyl]acetyl}amino)benzamide;
2-({[4-(2,4-dichlorobenzyl)-1-piperazinyl]acetyl}amino)benzamide;
2-({[4-(2,6-dichlorobenzyl)-1-piperazinyl]acetyl}amino)benzamide;
2-({[4-(4-chlorobenzyl)-1-piperazinyl]acetyl}amino)benzamide;
2-({[4-(2-chlorobenzyl)-1-piperazinyl]acetyl}amino)benzamide;
2-({[4-(2-chloro-4-fluorobenzyl)-1-piperazinyl]acetyl}amino)benzamide;
2-{3-[(2-chlorophenoxy)methyl]-4-methoxybenzamido}benzamide;
2-{3-[(2-chlorophenoxy)methyl]-4-methoxybenzamido}thiophene-3-carboxamide;
2-({3-[4-(benzyloxy)-3-methoxyphenyl]acryloyl}amino)benzamide;
2-{[(5-chloro-2-thienyl)carbonyl]amino}-5,6-dihydro-4H-cyclopenta[b]thiophene-3-carboxamide;
2-{[(5-chloro-2-thienyl)carbonyl]amino}-4,5,6,7-tetrahydro-1-benzothiophene-3-carboxamide;
N-[3-(aminocarbonyl)-5-benzyl-2-thienyl]-2-thiophenecarboxamide;
5-benzyl-2-{[(5-ethyl-3-thienyl)carbonyl]amino}-3-thiophenecarboxamide;
5-benzyl-2-{[(4-ethyl-5-methyl-3-thienyl)carbonyl]amino}-3-thiophenecarboxamide;
5-benzyl-2-[(2-thienylacetyl)amino]-3-thiophenecarboxamide;
N-[3-(aminocarbonyl)-5-benzyl-2-thienyl]-5-methyl-2-furamide;
N-[3-(aminocarbonyl)-5-benzyl-2-thienyl]-5-chloro-2-thiophenecarboxamide;
2-(benzoylamino)-5-benzyl-3-thiophenecarboxamide;
N-[3-(aminocarbonyl)-5-benzyl-2-thienyl]-1,3-benzodioxole-5-carboxamide;
5-benzyl-2-[(3-phenylpropanoyl)amino]-3-thiophenecarboxamide;
5-benzyl-2-[(phenoxyacetyl)amino]-3-thiophenecarboxamide;
5-benzyl-2-[(4-methoxybenzoyl)amino]-3-thiophenecarboxamide;
2-{[(3,4-dichlorophenyl)acetyl]amino}-5-isopropyl-3-thiophenecarboxamide;
5-benzyl-2-({4-[(4-bromophenoxy)methyl]benzoyl}amino)-3-thiophenecarboxamide;
5-benzyl-2-({3-[(2-chlorophenoxy)methyl]-4-methoxybenzoyl}amino)-3-thiophenecarboxamide;
5-benzyl-2-({3-[(2,3-dichlorophenoxy)methyl]-4-methoxybenzoyl}amino)-3-thiophenecarboxamide;
2-({3-[(2-chlorophenoxy)methyl]-4-methoxybenzoyl}amino)-5-isopropyl-3-thiophenecarboxamide;
5-benzyl-2-{3-[(2,4-dichlorophenoxy)methyl]-4-methoxybenzamido}thiophene-3-carboxamide;
2-[3-(2-chlorobenzyloxy)-4-methoxybenzamido]-5-benzylthiophene-3-carboxamide;
N-(5-benzyl-3-carbamoylthiophen-2-yl)-5-[(2,4-dichlorophenoxy)methyl]thiophene-2-carboxamide;
5-benzyl-2-{3-[(2,4-dichlorophenoxy)methyl]benzamido}thiophene-3-carboxamide;
5-benzyl-2-{3-[(3-chlorophenoxy)methyl]-4-methoxybenzamido}thiophene-3-carboxamide;
5-benzyl-2-{3-[(4-chlorophenoxy)methyl]-4-methoxybenzamido}thiophene-3-carboxamide;
5-benzyl-2-{3-[(2,6-dichlorophenoxy)methyl]-4-methoxybenzamido}thiophene-3-carboxamide;
2-{2-[4-(2-Chlorobenzyl)piperazin-1-yl]acetamido}-5-benzylthiophene-3-carboxamide;
2-{2-[4-(3,4-Dichlorobenzyl)piperazin-1-yl]acetamido}-5-benzylthiophene-3-carboxamide;
2-{2-[4-(3,4-Dichlorobenzyl)piperazin-1-yl]acetamido}thiophene-3-carboxamide;
2-({4-[(2-bromophenoxy)methyl]benzoyl}amino)-5-isopropyl-3-thiophenecarboxamide; and
2-({3-[(4-bromophenoxy)methyl]-4-methoxybenzoyl}amino)-5-isopropyl-3-thiophenecarboxamide.

A more preferred compound of formula (I) of formula (I) that shows an EC₅₀ of 5 µM or below in the call-based proliferation test, is a compound, or a pharmaceutically acceptable salt form, solvate or polymorph thereof, including all tautomers and stereoisomers thereof, selected from the group consisting of:
5-benzyl-2-{3-[(2,4-dichlorophenoxy)methyl]benzamido}thiophene-3-carboxamide;
2-{4-[(2-fluorobenzyl)oxy]phenyl}-4(3H)-quinazolinone;
5-benzyl-2-{3-[(2,4-dichlorophenoxy)methyl]-4-methoxybenzamido}thiophene-3-carboxamide;
5-benzyl-2-{3-[(3-chlorophenoxy)methyl]-4-methoxybenzamido}thiophene-3-carboxamide;
5-benzyl-2-({3-[(2,3-dichlorophenoxy)methyl]-4-methoxybenzoyl}amino)-3-thi ophenecarboxamide;
5-benzyl-2-({3-[(2-chlorophenoxy)methyl]-4-methoxybenzoyl}amino)-3-thiophenecarboxamide;
2-{3-methoxy-4-[(2-nitrobenzyl)oxy]phenyl}-4(3H)-quinazolinone;
N-(5-benzyl-3-carbamoylthiophen-2-yl)-5-[(2,4-dichlorophenoxy)methyl]thiophene-2-carboxamide;
N-[3-(aminocarbonyl)-5-benzyl-2-thienyl]-2-thiophenecarboxamide;
2-({3-[(3-chlorophenoxy)methyl]benzoyl}amino)benzamide;
2-{2-[4-(3,4-Dichlorobenzyl)piperazin-1-yl]acetamido}thiophene-3-carboxamide;
2-({[4-(2,4-dichlorobenzyl)-1-piperazinyl]acetyl}amino)benzamide;
5-benzyl-2-{3-[(4-chlorophenoxy)methyl]-4-methoxybenzamido}thiophene-3-carboxamide;
2-[3-(2-chlorobenzyloxy)-4-methoxybenzamido]-5-benzylthiophene-3-carboxamide;
2-{3-[(2-chlorophenoxy)methyl]-4-methoxybenzamido}benzamide;
6-benzyl-2-{4-methoxy-3-[(4-methoxyphenoxy)methyl]phenyl}thieno[2,3-d]pyrimidin-4(3H)-one;
5-benzyl-2-[(2-thienylacetyl)amino]-3-thiophenecarboxamide;
5-benzyl-2-({4-[(4-bromophenoxy)methyl]benzoyl}amino)-3-thiophenecarboxamide;
2-({[4-(3,4-dichlorobenzyl)-1-piperazinyl]acetyl}amino)benzamide;
N-[3-(aminocarbonyl)-5-benzyl-2-thienyl]-1,3-benzodioxole-5-carboxamide;
5-chloro-2-[2-(5-chlorothiophen-2-yl)-2-oxoethoxy]benzamide;
N-[3-(aminocarbonyl)-5-benzyl-2-thienyl]-5-methyl-2-furamide;
2-{[(5-chloro-2-thienyl)carbonyl]amino}-4,5,6,7-tetrahydro-1-benzothiophene-3-carboxamide;
N-[3-(aminocarbonyl)-5-benzyl-2-thienyl]-5-chloro-2-thiophenecarboxamide;
2-{3-[(2-chlorophenoxy)methyl]-4-methoxybenzamido}thiophene-3-carboxamide;
2-({3-[4-(benzyloxy)-3-methoxyphenyl]acryloyl}amino)benzamide;
N-[2-(aminocarbonyl)phenyl]-3,4-dichlorobenzamide;
5-benzyl-2-{[(5-ethyl-3-thienyl)carbonyl]amino}-3-thiophenecarboxamide;
5-benzyl-2-[(4-methoxybenzoyl)amino]-3-thiophenecarboxamide;
N-[2-(aminocarbonyl)phenyl]-5-methyl-2-thiophenecarboxamide;
2-({3-[(2-chlorophenoxy)methyl]-4-methoxybenzoyl}amino)-5-isopropyl-3-thiophenecarboxamide;
2-(benzoylamino)-5-benzyl-3-thiophenecarboxamide;
N-(2-carbamoylphenyl)-5-chlorothiophene-2-carboxamide;
5 -benzyl-2- [(phenoxyacetyl)amino]-3 -thiophenecarboxamide;
2-{[(3,4-dichlorophenyl)acetyl]amino}-5-isopropyl-3-thiophenecarboxamide;
5-benzyl-2-{3-[(2,6-dichlorophenoxy)methyl]-4-methoxybenzamido}thiophene-3-carboxamide;
4,5-dimethyl-N-[2-(methylcarbamoyl)phenyl]thiophene-2-carboxamide;
2-[2-(4-bromophenyl)-2-oxoethoxy]benzamide;
2-{[(5-ethyl-3-thienyl)carbonyl]amino}-3-thiophenecarboxamide;
2-({[4-(4-chlorobenzyl)-1-piperazinyl]acetyl}amino)benzamide;
2-({[4-(2-chloro-4-fluorobenzyl)-1-piperazinyl]acetyl}amino)benzamide;
N-[3-(aminocarbonyl)-2-thienyl]-2-thiophenecarboxamide;
2-({4-[(2-bromophenoxy)methyl]benzoyl}amino)-5-isopropyl-3-thiophenecarboxamide;
N-(5-{[1-(4-oxo-3,4-dihydro-2-quinazolinyl)ethyl]thio}-1,3,4-thiadiazol-2-yl)benzamide;
2-{2-[4-(3,4-Dichlorobenzyl)piperazin-1-yl]acetamido}-5-benzylthiophene-3-carboxamide; and
2-benzyl-6-methylthieno[2,3-d]pyrimidin-4(3H)-one.

A most preferred compound of formula (I) that shows an EC₅₀ of 1 µM or below in the call-based proliferation test, is a compound, or a pharmaceutically acceptable salt form, solvate or polymorph thereof, including all tautomers and stereoisomers thereof, selected from the group consisting of:
5-benzyl-2-{3-[(2,4-dichlorophenoxy)methyl]benzamido}thiophene-3-carboxamide;
2-{4-[(2-fluorobenzyl)oxy]phenyl}-4(3H)-quinazolinone;
5-benzyl-2-{3-[(2,4-dichlorophenoxy)methyl]-4-methoxybenzamido}thiophene-3-carboxamide;
5-benzyl-2-{3-[(3-chlorophenoxy)methyl]-4-methoxybenzamido}thiophene-3-carboxamide;
5-benzyl-2-({3-[(2,3-dichlorophenoxy)methyl]-4-methoxybenzoyl}amino)-3-thiophenecarboxamide;
5-benzyl-2-({3-[(2-chlorophenoxy)methyl]-4-methoxybenzoyl}amino)-3-thiophenecarboxamide;
2-{3-methoxy-4-[(2-nitrobenzyl)oxy]phenyl}-4(3H)-quinazolinone;
N-(5-benzyl-3-carbamoylthiophen-2-yl)-5-[(2,4-dichlorophenoxy)methyl]thiophene-2-carboxamide;
N-[3-(aminocarbonyl)-5-benzyl-2-thienyl]-2-thiophenecarboxamide;
2-({3-[(3-chlorophenoxy)methyl]benzoyl}amino)benzamide;
2-{2-[4-(3,4-Dichlorobenzyl)piperazin-1-yl]acetamido}thiophene-3-carboxamide; and
2-({[4-(2,4-dichlorobenzyl)-1-piperazinyl]acetyl}amino)benzamide.

In a preferred embodiment, the compound of formula (I) is not a compound selected from the group consisting of:
- BTY_A14 (CB7930921), N-(5-{[1-(4-oxo-3,4-dihydro-2-quinazolinyl)ethyl]thio}-1,3,4-thiadiazol-2-yl)benzamide;
- BTY_A18 (CB7764964), 2-benzyl-6-methylthieno[2,3-d]pyrimidin-4(3H)-one;
- BTY_A3.11 (CB7948757), N-[3-(aminocarbonyl)-2-thienyl]-2-thiophenecarboxamide;
- BTY_A3.34 (CB7949579), 2-{[(5-ethyl-3-thienyl)carbonyl]amino}-3-thiophenecarboxamide;
- BTY_A3.57 (CB6890496), 2-({3-[4-(benzyloxy)-3-methoxyphenyl]acryloyl}amino)-benzamide;
- BTY A3.63 (CB9085496), 2-{[(5-chloro-2-thienyl)carbonyl]amino}-5,6-dihydro-4H-cyclopenta[b]thiophene-3-carboxamide;
- BTY_A3.64 (CB9085855), 2-{[(5-chloro-2-thienyl)carbonyl]amino}-4,5,6,7-tetrahydro-1-benzothiophene-3-carboxamide;
- BTY_A3.14.14 (CB8803228), 2-{[(3,4-dichlorophenyl)acetyl]amino}-5-isopropyl-3-thiophenecarboxamide;
- BTY_A2, 2-[(5-chlorothiophen-2-yl)methoxy]benzamide;
- BTY_A3, N-[2-(aminocarbonyl)phenyl]-5-methyl-2- thiophenecarboxamide;
- 5-bromo-N-(2-nitrophenyl)-2-thiophenecarboxamide;
- N-[3-(aminocarbonyl)-5-benzyl-4-methyl-2-thienyl]-2-thiophenecarboxamide.

The compounds CB7930921, CB7764964, CB7948757, CB7949579, CB6890496, CB9085496, CB9085855, CB8803228 are obtainable from Chembridge, Inc., San Diego, USA and are disclosed in the Chembridge Catalogue as screening compounds without indication of a function or pharmaceutical use.

The compound BTY_A2 is disclosed on the PubChem website (ID-No. 45843355) without indication of a function or pharmaceutical use.

The compound BTY_A3 is disclosed on the CHEMSTEP website (CAS-No. 717866-93-8) without indication of a function or pharmaceutical use.

The compound 5-bromo-N-(2-nitrophenyl)-2-thiophenecarboxamide is disclosed on the PubChem website (ID-No. 54878060) without indication of a function or pharmaceutical use.

The compound N-[3-(aminocarbonyl)-5-benzyl-4-methyl-2-thienyl]-2-thiophenecarboxamide is disclosed on the Chemical Book website (ID-No. 315248-67-0) without indication of a function or pharmaceutical use.

In a further embodiment, the compound o formula (I) is not the compound 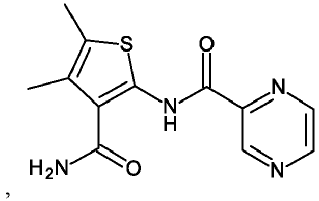 which is disclosed in US8377639 B2 as a compound with anti-helmintic activity.

### Pharmaceutical uses

The compounds of formula (I) as described herein bind non-covalently to an IGF2 (insulin-like growth factor 2) mRNA-binding protein and thereby inhibit the proliferative activity of said IGF2 mRNA-binding protein.

Accordingly, the invention further provides the compounds of formula (I) as described herein for use as a medicament.

The compounds of formula (I) as described herein may also be comprised in a pharmaceutical composition. In further embodiment, the present invention provides a pharmaceutical composition comprising a compound of formula (I) as described herein, optionally in combination with further excipients and diluents.

As shown in the working examples below, the compounds of the present invention are useful for the treatment of proliferative diseases, such as cancer, tumor metastasis or tumor recurrence in a subject. Accordingly, in a preferred embodiment, the invention provides the compounds of formula (I) or the pharmaceutical composition comprising said compound of formula (I) for use in the prevention or treatment of cancer, tumor metastasis or tumor recurrence in a subject.

In a further embodiment, the invention provides a method of treating a proliferative diseases, such as cancer, tumor metastasis or tumor recurrence in a subject in need thereof, comprising administering to said subject in need thereof a therapeutically effective amount of a compound of formula (I) or a pharmaceutical composition comprising said compound of formula (I).

Said proliferative disease is preferably selected from malignant tumors of epithelial, mesenchymal, (neuro)-endocrine or hematopoietic origin.

In a more preferred embodiment, said proliferative disease is cancer and is selected from the group consisting of malignant tumors of epithelial, mesenchymal or haemopoietic cells, breast cancer, prostate cancer, pancreatic cancer, adrenal cancer, melanoma, lung cancer, colon cancer, leukemia (a liquid or non-solid tumor), soft tissue and bone sarcomas, neuroendocrine tumors such as islet cell carcinoma or medullary carcinoma of the thyroid, squamous carcinomas (particularly of the head and neck), adenocarcinomas, gliosarcomas such as glioblastoma multiforme, liver cancer, kidney cancer, bladder cancer, stomach cancer, thyroid carcinomas of follicular origin, neuroblastoma, teratoma and ovarian carcinoma; preferably ovarian carcinoma, lung adenocarcinoma, melanoma, renal carcinoma, hepatocellular carcinoma, pancreas carcinoma and childhood-specific neuroblastoma.

### Processes

According to a further aspect of the invention there are provided processes for preparing compounds of general formula (I) which comprises:
(a) Preparing a compound of formula (IIa): 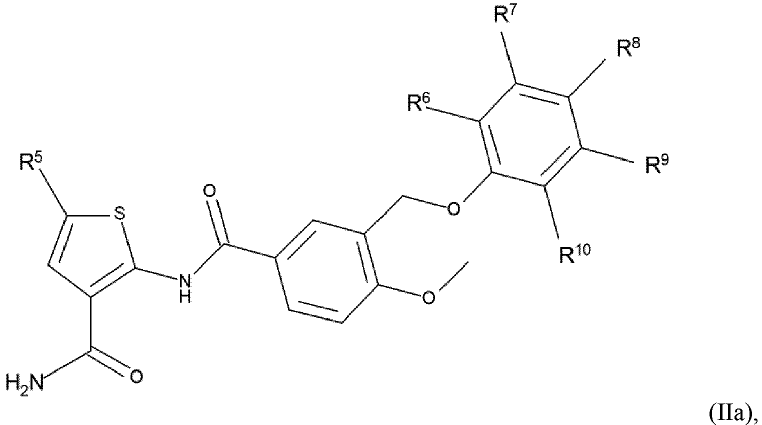 wherein
   R⁵, R⁶, R⁷, R⁸, R⁹, and R¹⁰ are as defined herein for compounds of formula (IIa).
   The process typically involves reacting the suitable phenol with the suitable alkyl bromide in presence of potassium carbonate followed by hydrolysis of the resulting ester using lithium hydroxide, and the resulting acid is coupled with the suitable amine using thionyl chloride. A non-limiting example of the methodologies of process (a) is described in methods A, B and C herein.
(b) Preparing a compound of formula (III): 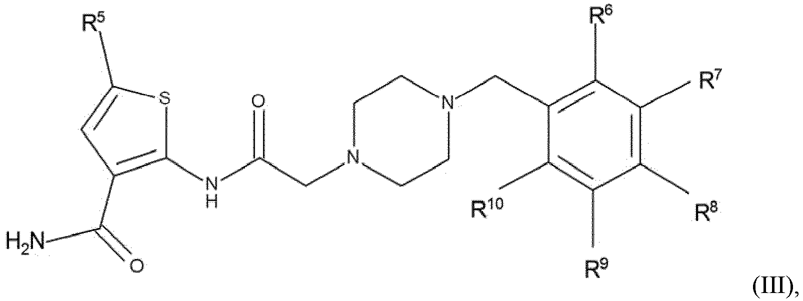 wherein
   R⁵, R⁶, R⁷, R⁸, R⁹, and R¹⁰ are as defined herein for compounds of formula (III).
   The process typically involves reacting the suitable amine with chloroacetyl chloride in presence of triethylamine followed by reacting the product with the suitable piperazine derivative using potassium carbonate. A non-limiting example of the methodologies of process (b) is described in the preparation of general intermediates and method E herein.
(c) Preparing a compound of formula (IV): 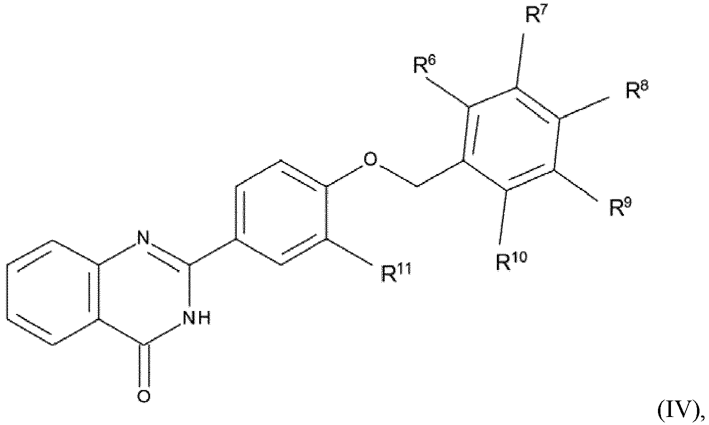 wherein
   R⁶, R⁷, R⁸, R⁹, and R¹⁰ and R¹¹ are as defined herein for compounds of formula (IV).
   The process typically involves reacting the suitable phenol with the suitable alkyl bromide in presence of potassium carbonate followed by reacting the resulting aldehyde with the suitable amine in dimethylsulfoxide to form the quinazolinone ring. A non-limiting example of the methodologies of process (c) is described in methods B and F herein.
(d) Preparing a compound of formula (V): 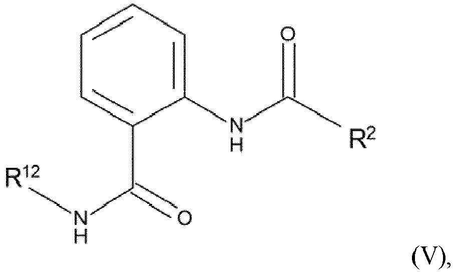 wherein R², R¹², R¹² are as defined herein for compounds of formula (V).
   The process typically involves reacting the suitable carboxylic acid with the suitable amine using thionyl chloride. A non-limiting example of the methodology of process (d) is described in method A herein.
(e) Preparing a compound of formula (VI): 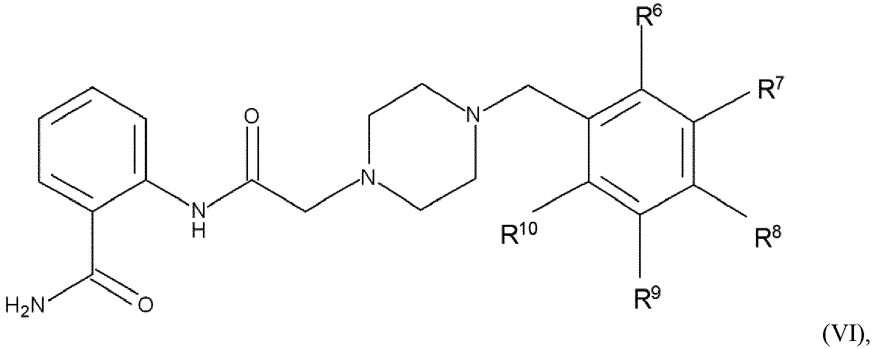 wherein
   R⁶, R⁷, R⁸, R⁹, and R¹⁰ are as defined herein for compounds of formula (VI).
   The process typically involved reacting 2-aminobenzamide with chloroacetyl chloride in presence of triethylamine followed by reacting the product with the suitable piperazine derivative using potassium carbonate. A non-limiting example of the methodologies of precess (e) is described in the preparation of general intermediates and method E herein.
(f) Preparing a compound of formula (VII): 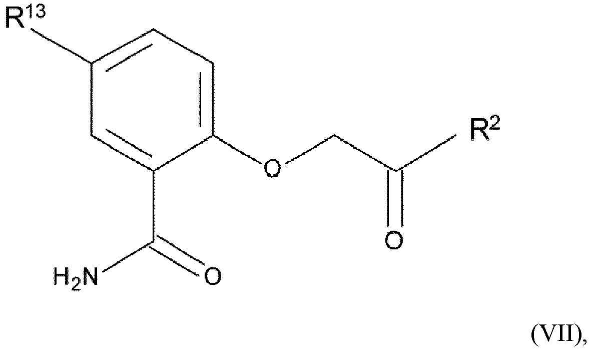 wherein
   R² and R¹³ are as defined herein for compounds of formula (VII).

The process typically involves reacting the suitable phenol with the suitable alkyl bromide in presence of potassium carbonate. A non-limiting examples of the methodology of process (of) is describes in method B herein.

### Pharmaceutical compositions

To prepare the pharmaceutical compositions of this invention, at least one compound of formula (I) optionally in combination with at least one of the other aforementioned agents can be used as the active ingredient(s). The active ingredient(s) is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, though other ingredients, for example, for purposes such as aiding solubility or for preservation, may be included.

Injectable suspensions may also prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient(s) necessary to deliver an effective dose as described above. The pharmaceutical compositions herein will contain, per dosage unit, e.g., tablet, capsule, powder, injection, suppository, teaspoonful and the like, from about 0.03 mg to 100 mg/kg (preferred 0.1 - 30 mg/kg) and may be given at a dosage of from about 0.1 - 300 mg/kg per day (preferred 1 - 50 mg/kg per day) of each active ingredient or combination thereof. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 500 mg of each active ingredient or combinations thereof of the present invention.

The tablets or pills of the compositions of the present invention can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

This liquid forms in which the compositions of the present invention may be incorporated for administration orally or by injection include, aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinylpyrrolidone or gelatin.

The pharmaceutical composition may contain between about 0.01 mg and 100 mg, preferably about 5 to 50 mg, of each compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixirs, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or betalactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitable flavored suspending or dispersing agents such as the synthetic and natural gums, for example, tragacanth, acacia, methyl-cellulose and the like. For parenteral administration, sterile suspensions and solutions are desired. Isotonic preparations which generally contain suitable preservatives are employed when intravenous administration is desired.

The compounds or combinations of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine or phosphatidylcholines.

Compounds or combinations of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamidephenol, polyhydroxyethylaspartamid-ephenol, or polyethyl eneoxidepolyllysine substituted with palmitoyl residue. Furthermore, the compounds of the present invention may be coupled to a class of biodegradable polymers useful in achieving controlled release of a drug, for example, polyactic acid, polyepsilon caprolactone, polyhydroxy butyeric acid, polyorthoesters, polyacetals, polydihydropyrans, polycyanoacrylates and cross-linked or amphipathic block copolymers of hydrogels.

Compounds or combinations of this invention may be administered in any of the foregoing compositions and according to dosage regimens established in the art whenever treatment of the addressed disorders is required.

The daily dosage of the products may be varied over a wide range from 0.01 to 1.000 mg per mammal per day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250 and 500 milligrams of each active ingredient or combinations thereof for the symptomatic adjustment of the dosage to the patient to be treated. An effective amount of the drug is ordinarily supplied at a dosage level of from about 0.1 mg/kg to about 300 mg/kg of body weight per day. Preferably, the range is from about 1 to about 50 mg/kg of body weight per day. The compounds or combinations may be administered on a regimen of 1 to 4 times per day.

Optimal dosages to be administered may be readily determined by those skilled in the art, and will vary with the particular compound used, the mode of administration, the strength of the preparation, the mode of administration, and the advancement of disease condition. In addition, factors associated with the particular patient being treated, including patient age, weight, diet and time of administration, will result in the need to adjust dosages.

In a further aspect, the invention also provides a process for preparing a pharmaceutical composition comprising at least one compound of formula (I), optionally in combination with at least one of the other aforementioned agents and a pharmaceutically acceptable carrier.

The compositions are preferably in a unit dosage form in an amount appropriate for the relevant daily dosage.

Suitable dosages, including especially unit dosages, of the the compounds of the present invention include the known dosages including unit doses for these compounds as described or referred to in reference text such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) or the above mentioned publications.

### Examples of the invention

### Examples of compounds of formula (I)

| **No.** | **Compound** | **Code** | **IUPAC name** | **Formula**/ **MW** |
|---|---|---|---|---|
| 1 | 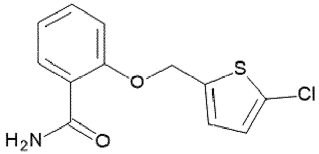 | BTY_A2 | 2-[(5-chlorothiophen-2-yl)methoxy]benzami de | C₁₂H₁₀ClNO₂S/ 267.7313 |
| 2 | 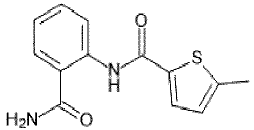 | BTY_A3 | N-[2-(aminocarbonyl)phe nyl]-5-methyl-2-thiophenecarboxami de | C₁₃H₁₂N₂O₂S/ 260.3116 |
| 3 | 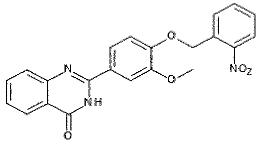 | BTY_A8 | 2-{3-methoxy-4-[(2-nitrobenzyl)oxy]phe nyl}-4(3H)-quinazolinone | C₂₂H₁₇N₃O₅/ 403.3875 |
| 4 | 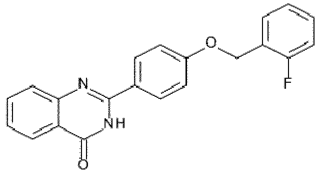 | BTY_A1 0 | 2-{4-[(2-fluorobenzyl)oxy]ph enyl}-4(3H)-quinazolinone | C₂₁H₁₅FN₂O₂/ 346.3544 |
| 5 | 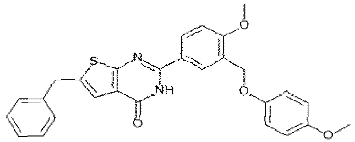 | BTY_A1 3 | 6-benzyl-2-{4-methoxy-3-[(4-methoxyphenoxy)m ethyl]phenyl}thieno[ 2,3-d]pyrimidin-4(3H)-one | C₂₈H₂₄N₂O₄S/ 484.5662 |
| 6 | 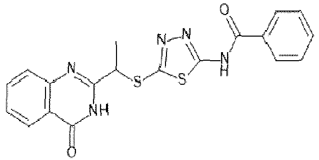 | BTY_A1 4 | N-(5-{ [1-(4-oxo-3,4-dihydro-2-quinazolinyl)ethyl]t hio}-1,3,4-thiadiazol-2-yl)benzamide | C₁₉H₁₅N₅O₂S₂/ 409.4847 |
| 7 | 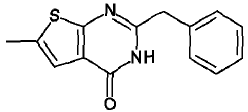 | BTY_A1 8 | 2-benzyl-6-methylthieno [2,3 - d]pynmidin-4(3H)-one | C₁₄H₁₂N₂OS/ 256.3229 |
| 8 | 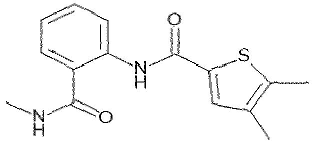 | BTY_A3. 2 | 4,5-dimethyl-N-[2-(methylcarbamoyl)p henyl]thiophene-2-carboxamide | C₁₅H₁₆N₂O₂S/ 288.3647 |
| 9 | 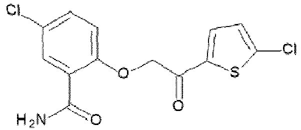 | BTY_A3. 5 | 5-chloro-2-[2-(5-chlorothiophen-2-yl)-2-oxoethoxy]benzami de | C₁₃H₉Cl₂NO₃S/ 330.1865 |
| 10 | 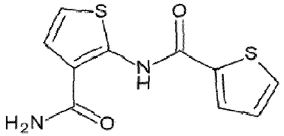 | BTY_A3. 11 | N-[3-(aminocarbonyl)-2-thienyl]-2-thiophenecarboxami de | C₁₀H₈N₂O₂S₂/ 252.3100 |
| 11 | 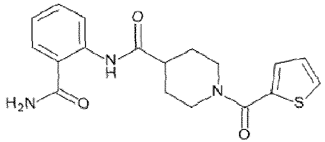 | BTY_A3. 22 | N-[2-(aminocarbonyl)phe nyl]-1-(2-thienylcarbonyl)-4-piperidinecarboxami de | C₁₈H₁₉N₃O₃S/ 357.4268 |
| 12 | 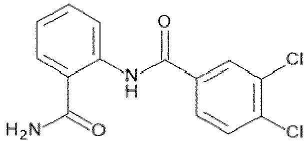 | BTY_A3. 26 | N-[2-(aminocarbonyl)phe nyl]-3,4-dichlorobenzamide | C₁₄H₁₀Cl₂N₂O₂/ 309.1478 |
| 13 | 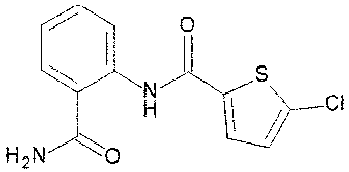 | BTY_A3. 29 | N-(2-carbamoylphenyl)-5-chlorothiophene-2-carboxamide | C₁₂H₉ClN₂O₂S/ 280.7301 |
| 14 | 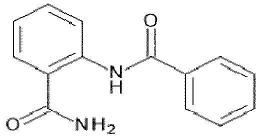 | BTY_A3. 30 | N-(2-carbamoylphenyl)be nzamide | C₁₄H₁₂N₂O₂/ 240.2573 |
| 15 | 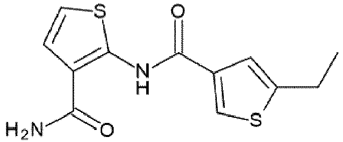 | BTY_A3. 34 | 2-{[(5-ethyl-3-thienyl)carbonyl]am ino}-3-thiophenecarboxami de | C₁₂H₁₂N₂O₂S₂/ 280.3659 |
| 16 | 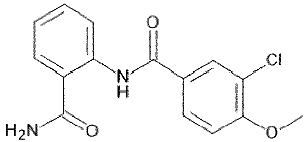 | BTY_A3. 35 | N-[2-(aminocarbonyl)phe nyl]-3-chloro-4-methoxybenzamide | C₁₅H₁₃ClN₂O₃/ 304.7283 |
| 17 | 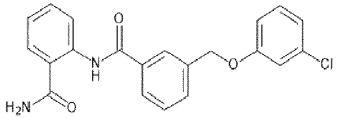 | BTY_A3. 39 | 2-({3-[(3-chlorophenoxy)meth yl]benzoyl}amino)b enzamide | C₂₁H₁₇ClN₂O₃/ 380.8243 |
| 18 | 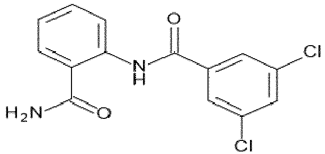 | BTY_A3. 40 | N-[2-(aminocarbonyl)phe nyl]-3,5-dichlorobenzamide | C₁₄H₁₀Cl₂N₂O₂/ 309.1474 |
| 19 | 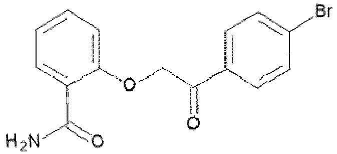 | BTY_A3. 44 | 2-[2-(4-bromophenyl)-2-oxoethoxy]benzami de | C₁₅H₁₂BrNO₃/ 334.1647 |
| 20 | 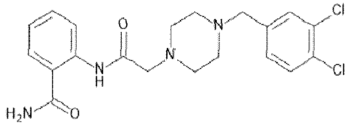 | BTY_A3. 46 | 2-({[4-(3,4-dichlorobenzyl)-1-piperazinyl]acetyl}a mino)benzamide | C₂₀H₂₂Cl₂N₄O₂/ 421.3203 |
| 21 | 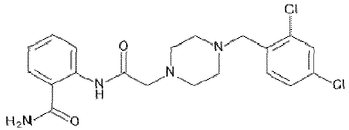 | BTY_A3. 47 | 2-({[4-(2,4-dichlorobenzyl)-1-piperazinyl]acetyl}a mino)benzamide | C₂₀H₂₂Cl₂N₄O₂/ 421.3203 |
| 22 | 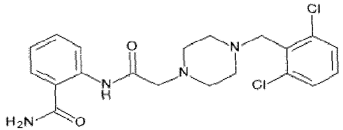 | BTY_A3. 49 | 2-({[4-(2,6-dichlorobenzyl)-1-piperazinyl]acetyl}a mino)benzamide | C₂₀H₂₂Cl₂N₄O₂/ 421.3203 |
| 23 | 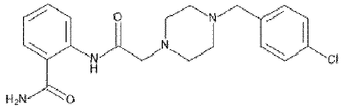 | BTY_A3. 50 | 2-({[4-(4-chlorobenzyl)-1-piperazinyl]acetyl}a mino)benzamide | C₂₀H₂₃ClN₄O₂/ 386.8752 |
| 24 | 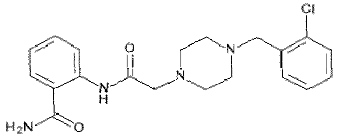 | BTY_A3. 51 | 2-({[4-(2-chlorobenzyl)-1-piperazinyl]acetyl}a mino)benzamide | C₂₀H₂₃ClN₄O₂/ 386.8752 |
| 25 | 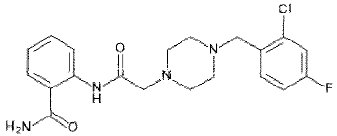 | BTY_A3. 52 | 2-({[4-(2-chloro-4-fluorobenzyl)-1-piperazinyl]acetyl}a mino)benzamide | C₂₀H₂₂ClFN₄O₂/ 404.8657 |
| 26 | 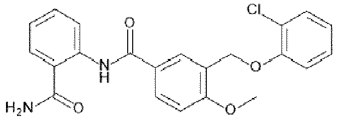 | BTY_A3. 54 | 2-{3-[(2-chlorophenoxy)meth yl]-4-methoxybenzamido } benzamide | C₂₂H₁₉ClN₂O₄/ 410.8503 |
| 27 | 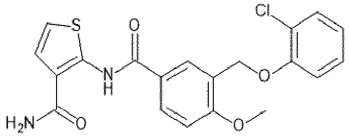 | BTY_A3. 55 | 2-{3-[(2-chlorophenoxy)meth yl]-4-methoxybenzamido} thiophene-3-carboxamide | C₂₀H₁₇ClN₂O₄S/ 416.8780 |
| 28 | 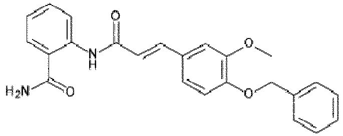 | BTY_A3. 57 | 2-({3-[4-(benzyloxy)-3-methoxyphenyl]acry loyl}amino)benzami de | C₂₄H₂₂N₂O₄/ 402.4435 |
| 29 | 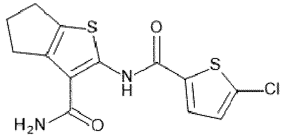 | BTY_A3. 63 | 2-{[(5-chloro-2-thienyl)carbonyl]am ino}-5,6-dihydro-4H-cyclopenta[b]thioph ene-3 -carboxamide | C₁₃H₁₁ClN₂O₂S₂/ 326.8216 |
| 30 | 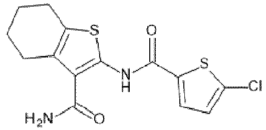 | BTY_A3. 64 | 2-{[(5-chloro-2-thienyl)carbonyl]am ino}-4,5,6,7-tetrahydro-1-benzothiophene -3- carboxamide | C₁₄H₁₃ClN₂O₂S₂/ 340.8482 |
| 31 | 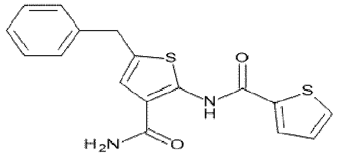 | BTY_A3. 14 | N-[3-(aminocarbonyl)-5-benzyl-2-thienyl]-2-thiophenecarboxami de | C₁₇H₁₄N₂O₂S₂/ 342.4353 |
| 32 | 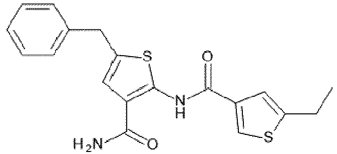 | BTY_A3. 14.2 | 5-benzyl-2-{[(5-ethyl-3-thienyl)carbonyl]am ino}-3-thiophenecarboxami de | C₁₉H₁₈N₂O₂S₂/ 370.4884 |
| 33 | 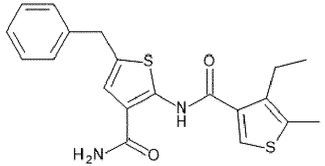 | BTY_A3. 14.3 | 5-benzyl-2-{[(4-ethyl-5-methyl-3-thienyl)carbonyl]am ino}-3-thiophenecarboxami de | C₂₀H₂₀N₂O₂S₂/ 384.5150 |
| 34 | 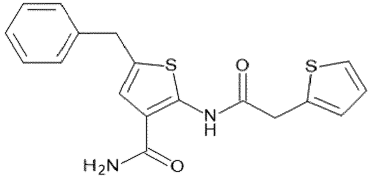 | BTY_A3. 14.4 | 5-benzyl-2-[(2-thienylacetyl)amino] -3-thiophenecarboxami de | C₁₈H₁₆N₂O₂S₂/ 356.4618 |
| 35 | 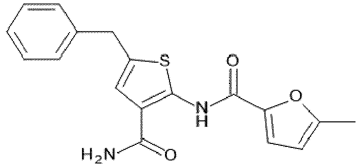 | BTY_A3. 14.5 | N-[3-(aminocarbonyl)-5-benzyl-2-thienyl]-5-methyl-2-furamide | C₁₈H₁₆NO₃S/ 340.3895 |
| 36 | 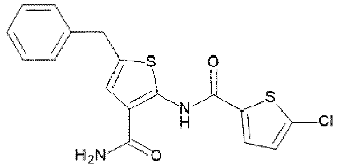 | BTY_A3. 14.6 | N-[3-(aminocarbonyl)-5-benzyl-2-thienyl]-5-chloro-2-thiophenecarboxami de | C₁₇H₁₃ClN₂O₂S₂/ 376.8803 |
| 37 | 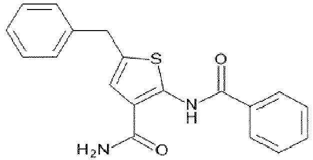 | BTY_A3. 14.36 | 2-(benzoylamino)-5-benzyl-3-thiophenecarboxami de | C₁₉H₁₆N₂O₂S/ 336.4075 |
| 38 | 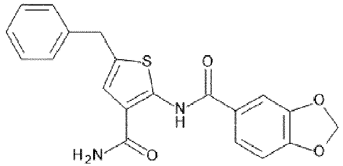 | BTY_A3. 14.9 | N-[3-(aminocarbonyl)-5-benzyl-2-thienyl]-1,3 -benzodioxole-5 - carboxamide | C₂₀H₁₆N₂O₄S/ 380.4170 |
| 39 | 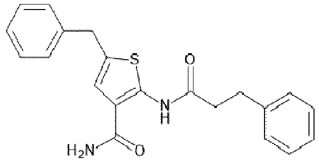 | BTY_A3. 14.10 | 5-benzyl-2-[(3-phenylpropanoyl)am ino]-3-thiophenecarboxami de | C₂₁H₂₀N₂O₂S/ 364.4607 |
| 40 | 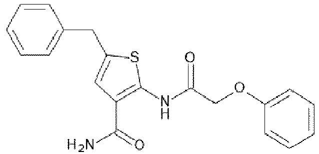 | BTY_A3. 14.11 | 5-benzyl-2-[(phenoxyacetyl)ami no]-3-thiophenecarboxami de | C₂₀H₁₈N₂O₃S/ 366.4335 |
| 41 | 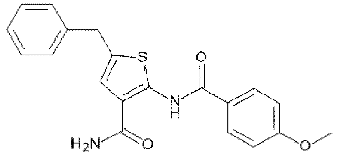 | BTY_A3. 14.12 | 5-benzyl-2-[(4-methoxybenzoyl)am ino]-3-thiophenecarboxami de | C₂₀H₁₈N₂o₃S/ 366.4335 |
| 42 | 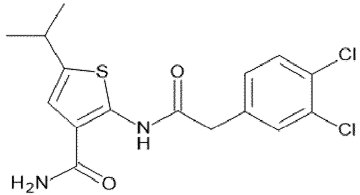 | BTY_A3. 14.14 | 2-{[(3,4-dichlorophenyl)acet yl]amino}-5-isopropyl-3-thiophenecarboxami de | C₁₆H₁₆Cl₂N₂O₂S/ 371.2814 |
| 43 | 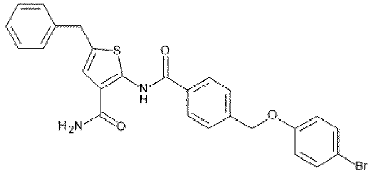 | BTY_A3.1 4.15 | 5-benzyl-2-({4-[(4-bromophenoxy)met hyl]benzoyl}amino) -3-thiophenecarboxami de | C₂₆H₂₁BrN₂O₃S/ 521.4255 |
| 44 | 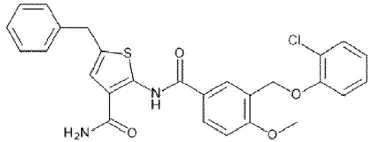 | BTY_A3. 14.16 | 5-benzyl-2-({3-[(2-chlorophenoxy)meth yl]-4-methoxybenzoyl }am ino)-3-thiophenecarboxami de | C₂₇H₂₃ClN₂O₄S 507.0005 |
| 45 | 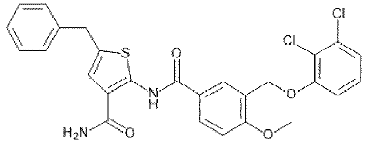 | BTY_A3. 14.17 | 5-benzyl-2-({3-[(2,3-dichlorophenoxy)me thyl]-4-methoxybenzoyl}am ino)-3-thiophenecarboxami de | C₂₇H₂₂Cl₂N₂O₄S/ 541.4456 |
| 46 | 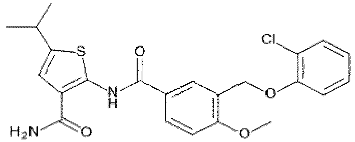 | BTY_A3. 14.21 | 2-({3-[(2-chlorophenoxy)meth yl]-4-methoxybenzoyl }am ino)-5 -isopropyl-3 - thiophenecarboxami de | C₂₃H₂₃ClN₂O₄S/ 458.9577 |
| 47 | 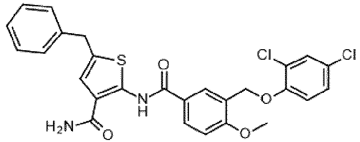 | BTY_A3. 14.22 | 5-benzyl-2-{3-[(2,4-dichlorophenoxy)me thyl]-4-methoxybenzamido } thiophene-3-carboxamide | C₂₇H₂₂Cl₂N₂O₄S/ 541.4456 |
| 48 | 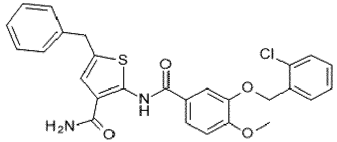 | BTY_A3. 14.23 | 2-[3-(2-chlorobenzyloxy)-4-methoxybenzamido] -5 -benzylthiophene-3 -carboxamide | C₂₂H₂₃ClN₂O₄S/ 507.0005 |
| 49 | 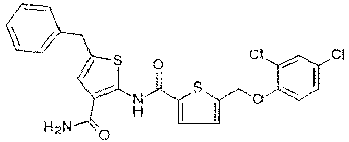 | BTY_A3. 14.24 | N-(5-benzyl-3-carbamoylthiophen-2-yl)-5-[(2,4-dichlorophenoxy)me thyl]thiophene-2-carboxamide | C₂₄H₁₈Cl₂N₂O₃S₂/ 517.4473 |
| 50 | 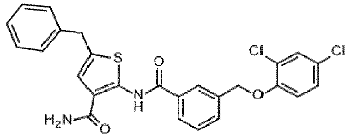 | BTY_A3. 14.25 | 5-benzyl-2-{3-[(2,4-dichlorophenoxy)me thyl]benzamido}thio phene-3-carboxamide | C₂₆H₂₀Cl₂N₂O₃S/ 511.4960 |
| 51 | 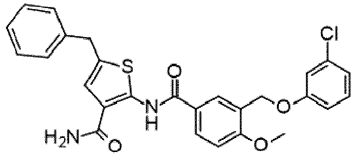 | BTY_A3. 14.27 | 5-benzyl-2-{3-[(3-chlorophenoxy)meth yl]-4-methoxybenzamido} thiophene-3-carboxamide | C₂₂H₂₃ClN₂O₄S/ 507.0050 |
| 52 | 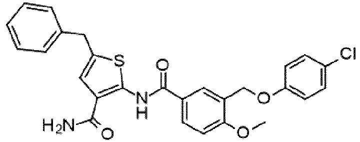 | BTY_A3. 14.28 | 5-benzyl-2-{3-[(4-chlorophenoxy)meth yl]-4-methoxybenzamido} thiophene- 3-carboxamide | C₂₂H₂₃ClN₂O₄S/ 507.0050 |
| 53 | 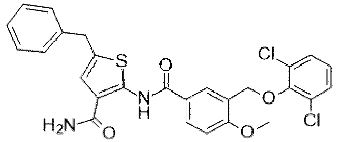 | BTY_A3. 14.30 | 5-benzyl-2-{3-[(2,6-dichlorophenoxy)me thyl]-4-methoxybenzamido } thiophene-3-carboxamide | C₂₇H₂₂Cl₂N₂O₄S/ 541.4456 |
| 54 | 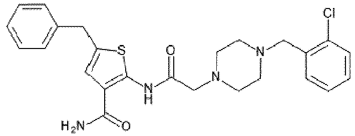 | BTY_A3.1 4.33 | 2-{2-[4-(2-Chlorobenzyl)pipera zin-1-yl]acetamido}-5-benzylthiophene-3-carboxamide | C₂₅H₂₇ClN₄O₂S/ 483.0255 |
| 55 | 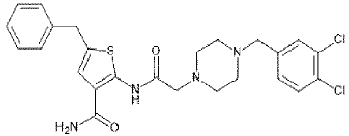 | BTY_A3.1 4.34 | 2-{2-[4-(3,4-Dichlorobenzyl)pipe razin-1-yl]acetamido}-5-benzylthiophene-3-carboxamide | C₂₅H₂₆Cl₂N₄O₂S/ 517.4705 |
| 56 | 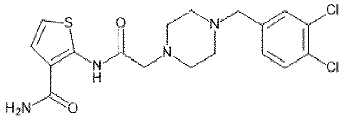 | BTY_A3.1 4.35 | 2-{2-[4-(3,4-Dichlorobenzyl)pipe razin-1-yl]acetamido}thioph ene-3 -carboxamide | C₁₈H₂₀Cl₂N₄O₂S/ 427.3480 |
| 57 | 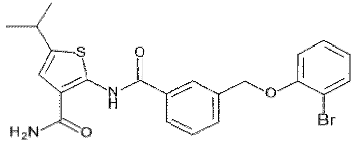 | BTY_A3. 14.37 | 2-({4-[(2-bromophenoxy)met hyl]benzoyl}amino) -5-isopropyl-3-thiophenecarboxami de | C₂₃H₂₃BrN₂O₄S/ 473.3827 |
| 58 | 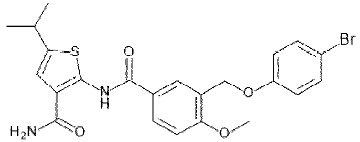 | BTY_A3. 14.39 | 2-({3-[(4-bromophenoxy)met hyl]-4-methoxybenzoyl }am ino)-5-isopropyl-3 - thiophenecarboxami de | C₂₂H₂₁BrN₂O₃S/ 503.3827 |

The invention further relates to the racemates, S-stereoisomers and R-stereoisomers of the compounds of the present invention as shown in the table above.

The chemical details of prior art compound BTYNB are as follows:

| **Compound** | **MW** | **IUPAC name** | **Formula** |
|---|---|---|---|
| 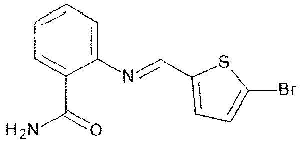 | 309.1817 | 2-{[(5-bromo-2-thienyl)methylene]amino}be nzamide (BTYNB) | C₁₂H₉BrN₂OS |

### Synthesis of the Compounds of the Invention

### General synthesis description:

All materials and reagents were purchased from Sigma-Aldrich Co. Ltd. and abcr GmbH. All solvents were analytically pure and dried before use. Thin layer chromatography was carried out on aluminum sheets coated with silica gel 60 F254 (Merck, Darmstadt, Germany).

The following compounds were purchased from Chembridge, Inc., San Diego, USA:
BTY_A14 (CB7930921), N-(5-{[1-(4-oxo-3,4-dihydro-2-quinazolinyl)ethyl]thio}-1,3,4-thiadiazol-2-yl)benzamide;
BTY_A18 (CB7764964), 2-benzyl-6-methylthieno[2,3-d]pyrimidin-4(3H)-one;
BTY_A3.11 (CB7948757), N-[3-(aminocarbonyl)-2-thienyl]-2-thiophenecarboxamide;
BTY_A3.34(CB7949579),2-{[(5-ethyl-3-thienyl)carbonyl]amino}-3-thiophenecarboxamide;
BTY_A3.57 (CB6890496), 2-({3-[4-(benzyloxy)-3-methoxyphenyl]acryloyl}amino)-benzamide;
BTY_A3.63 (CB9085496), 2-{[(5-chloro-2-thienyl)carbonyl]amino}-5,6-dihydro-4H-cyclopenta[b]thiophene-3-carboxamide;
BTY_A3.64 (CB9085855), 2-{[(5-chloro-2-thienyl)carbonyl]amino}-4,5,6,7-tetrahydro-1-benzothiophene-3-carboxamide;
BTY_A3.14.14 (CB8803228), 2-{[(3,4-dichlorophenyl)acetyl]amino}-5-isopropyl-3-thiophenecarboxamide.

### Analytical methods

Medium pressure liquid chromatography (MPLC) was performed in normal phase with an Isolera Flash chromatography System (Biotage INC) and SNAP Ultra columns (Biotage INC, particle size 25 µm) of different sizes (10, 25 and 50 g).

The purity of the final compounds was determined using HPLC based on UV absorbance at 254 nm. The HPLC consists of an XTerra RP18 column (3.5 µm, 3.9 mm × 100 mm) from the manufacturer Waters (Milford, MA, USA) and two LC-10AD pumps, a SPD-M10A VP PDA detector, and a SIL-HT autosampler, all from the manufacturer Shimadzu (Kyoto, Japan).

Mass spectrometry analyses were performed with a Finnigan MAT710C (Thermo Separation Products, San Jose, CA, USA) for the ESIMS spectra. ¹H NMR spectra were taken on a Varian Inova 400 using deuterated chloroform or deuterated dimethylsulfoxid (DMSO) as solvents. Chemical shifts are referenced to the residual solvent signals.

### Method A: amide coupling using thionyl chloride

The appropriate carboxylic acid (2.2 mmol) was refluxed in thionyl chloride (2 ml) for 2-3 hours. The solvent was then evaporated under reduced pressure, and the residue was dissolved in dry tetrahydrofuran. This solution was then added dropwise with stirring to a solution of the appropriate amine (2 mmol) and N,N-diisopropylethylamine (6 mmol) in dry tetrahydrofuran at 0 °C. The reaction mixture was then left stirring at room temperature overnight. The mixture was then diluted with ethyl acetate and washed with 2M aqueous solution of ammonium chloride and brine. The organic layer was then dried over sodium sulfate, evaporated under reduced pressure, and the mixture was purified with MPLC using chloroform/methanol as eluents.

### Method B: Williamson's etherification

A mixture of the appropriate phenol (1 mmol), alkyl halide (1 mmol) and potassium carbonate (3 mmol) in acetonitrile was refluxed until TLC showed total consumption of the starting materials (usually after 2-4 hours). The reaction mixture was then diluted with water, and the precipitated product was collected with filtration, washed with water and dried. The products were pure enough for the next step.

### Method C: ester hydrolysis with LiOH

A mixture of the appropriate ester (1 mmol) and lithium hydroxide monohydrate (5 mmol) in 1:1 mixture of tetrahydrofuran and water (5 ml) was heated at 50-60 °C in water bath until TLC confirmed total hydrolysis of the ester (usually after 2 hours). Tetrahydrofuran was then evaporated under reduced pressure, and the remaining aqueous solution was acidified with 1M hydrochloric acid. The precipitated acid was collected with filtration, washed with water and dried. The products were pure enough for the next step.

### Method D: esterification of carboxylic acids

The appropriate carboxylic acid (1 mmol) was dissolved in 50 ml methanol and cooled to 0 °C, then thionyl chloride (3 mmol) was added dropwise and the reaction mixture was refluxed for 3 hours. The solvent was then evaporated under reduced pressure, and the crude product was dissolved in ethyl acetate and washed with aqueous sodium hydroxide solution. The organic layer was then dried over sodium sulfate and evaporated under reduced pressure. The obtained products were pure enough for the next step of the synthesis.

### Method E: alkylation of piperazine

A mixture of piperazine (4 mmol), the appropriate alkyl halide (1 mmol) and potassium carbonate (2 mmol) in acetone or acetonitrile was refluxed for 2-4 hours. The solvent was evaporated and the residue was diluted with water. The aqueous layer was extracted with ethyl acetate, and the organic layer was then dried over sodium sulfate and evaporated under reduced pressure. The obtained products were pure enough and required no further purification.

### Method F: synthesis of quinazolinones

An equimolar mixture (1 mmol) of the appropriate aldehyde and amide derivatives was dissolved in dimethylsulfoxid (5 ml). The reaction mixture was stirred at 100 °C in an open flask until the product precipitated. The reaction mixture was cooled to room temperature, and the precipitated product was collected with filtration, washed with dimethylsulfoxid and methanol, and then dried. The obtained products were pure enough and required no further purification.

### Method G: bromination using N-bromosuccinimide

The appropriate methyl derivative (1 mmol) and N-bromosuccinimide (1.05 mmol) were refluxed in carbon tetrachloride in the presence of a catalytic amount of benzoyl peroxide overnight. The reaction was then cooled to 0 °C and filtered. The filtrate was evaporated under reduced pressure and purified with MPLC using hexane/ethyl acetate as eluents.

### Synthesis of common intermediates

### 2-Amino-5-benzylthiophene-3-carboxamide

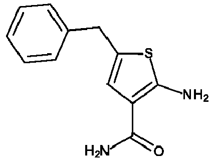

To a mixture of 3-phenylpropanal (2 mmol), 2-cyanoacetamide (2 mmol) and sulfur (2 mmol) in dimethylformamide (8 ml) was added dropwise triethylamine (2 mmol). The mixture was stirred at room temperature overnight, then added to ice/water mixture and vigorously stirred. The precipitated product was collected with filtration, washed with water and dried. The product was further purified with MPLC using chloroform/methanol as eluents.
MS m/z: 233.10 [M+H]⁺
¹H NMR (400 MHz, DMSO) δ 7.31-7.25 (m, 2H), 7.22-7.16 (m, 3H), 7.13-6.89 (m, 3H), 6.84 -6.56 (m, 2H), 3.83 (s, 2H).

### 2-(2-Chloroacetamido)benzamide ¹⁶

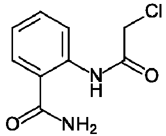

To a mixture of anthranilamide (1 mmol) and triethylamine (1 mmol) in tetrahydrofuran at 0 °C was added drop-wise chloroacetyl chloride (1.1 mmol). The reaction mixture was then stirred at room temperature until TLC confirmed total consumption of the starting material. The mixture was then diluted with ethyl acetate and washed with 2M aqueous solution of ammonium chloride and brine. The organic layer was then dried over sodium sulfate, evaporated under reduced pressure, and the product didn't require further purification.
MS m/z: 213.10 [M+H]⁺
¹H NMR (400 MHz, DMSO) δ 12.26 (s, 1H), 8.45 (dd, *J* = 8.4, 1.0 Hz, 1H), 8.27 (s, 1H), 7.80 (dd, *J* = 7.9, 1.4 Hz, 1H), 7.74 (s, 1H), 7.54 - 7.48 (m, 1H), 7.17 (td, *J* = 7.8, 1.2 Hz, 1H), 4.38 (s, 2H).

### 5-Benzyl-2-(2-chloroacetamido)thioi)hene-3-carboxamide

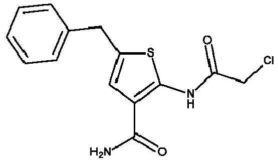

This compound was prepared using the same method as 2-(2-chloroacetamido)benzamide starting from 2-amino-5-benzylthiophene-3-carboxamide.
MS m/z: 309.20 [M+H]⁺
¹H NMR (400 MHz, DMSO) δ 12.76 (s, 1H), 7.87 (s, 1H), 7.48 (s, 1H), 7.33 - 7.28 (m, 2H), 7.27 - 7.18 (m, 4H), 4.49 (s, 2H), 4.03 (s, 2H).

### 2-(2-Chloroacetamido)thiophene-3-carboxamide

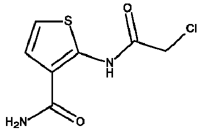

This compound was prepared using the same method as 2-(2-chloroacetamido)benzamide starting from 2-amino-3-thiophenecarboxamide.
MS m/z: 217 [M-H]⁻
¹H NMR (400 MHz, DMSO) δ 12.83 (s, 1H), 7.95 (s, 1H), 7.54 (s, 1H), 7.43 (d, *J* = 5.8 Hz, 1H), 7.02 (dd, *J* = 5.8, 0.7 Hz, 1H), 4.54 (s, 2H).

### Methyl 3-(bromomethyl)-4-methoxybenzoate ¹⁷

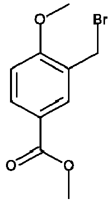

The compound was prepared from 4-methoxy-3-methylbenzoic acid methyl ester using method G.
MS m/z: 259.20 [M+H]⁺
¹H NMR (400 MHz, CDCl₃) δ 8.04 - 7.98 (m, 2H), 6.92 - 6.89 (m, 1H), 4.54 (s, 2H), 3.95 (s, 3H), 3.89 (s, 3H).

### Methyl 5-(bromomethyl)thiophene-2-carboxylate ¹⁸

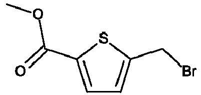

The compound was prepared from methyl 5-methylthiophene-2-carboxylate using method G. MS m/z: 234.80 [M+H]⁺
¹H NMR (400 MHz, CDCl₃) δ 7.62 (d, *J* = 3.8 Hz, 1H), 7.08 (d, *J* = 3.8 Hz, 1H), 4.66 (s, 2H), 3.87 (s, 3H).

### 3-(Bromomethyl)-4-methoxybenzaldehyde

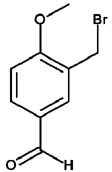

4-Methoxybenzaldehyde (10 mmol) was dissolved in a mixture of 10 ml 47% hydrobromic acid and 5 ml acetic acid, then 0.3 g paraformaldehyde (10 mmol) was added and the reaction was stirred at 60 °C for 24 h. The mixture was then cooled and diluted with water. The formed precipitate was filtered, washed with water and dried. The product was further purified with MPLC using hexane/ethyl acetate as eluents.
MS m/z: 227.90 [M+H]⁺
¹H NMR (400 MHz, CDCl₃) δ 9.90 (s, 1H), 7.90 (s, 1H), 7.80 (d, *J* = 8.00 Hz, 1H), 7.00 (d, *J* = 8.00 Hz, 1H), 4.66 (s, 2H), 3.87 (s, 3H).

### Synthetic schemes

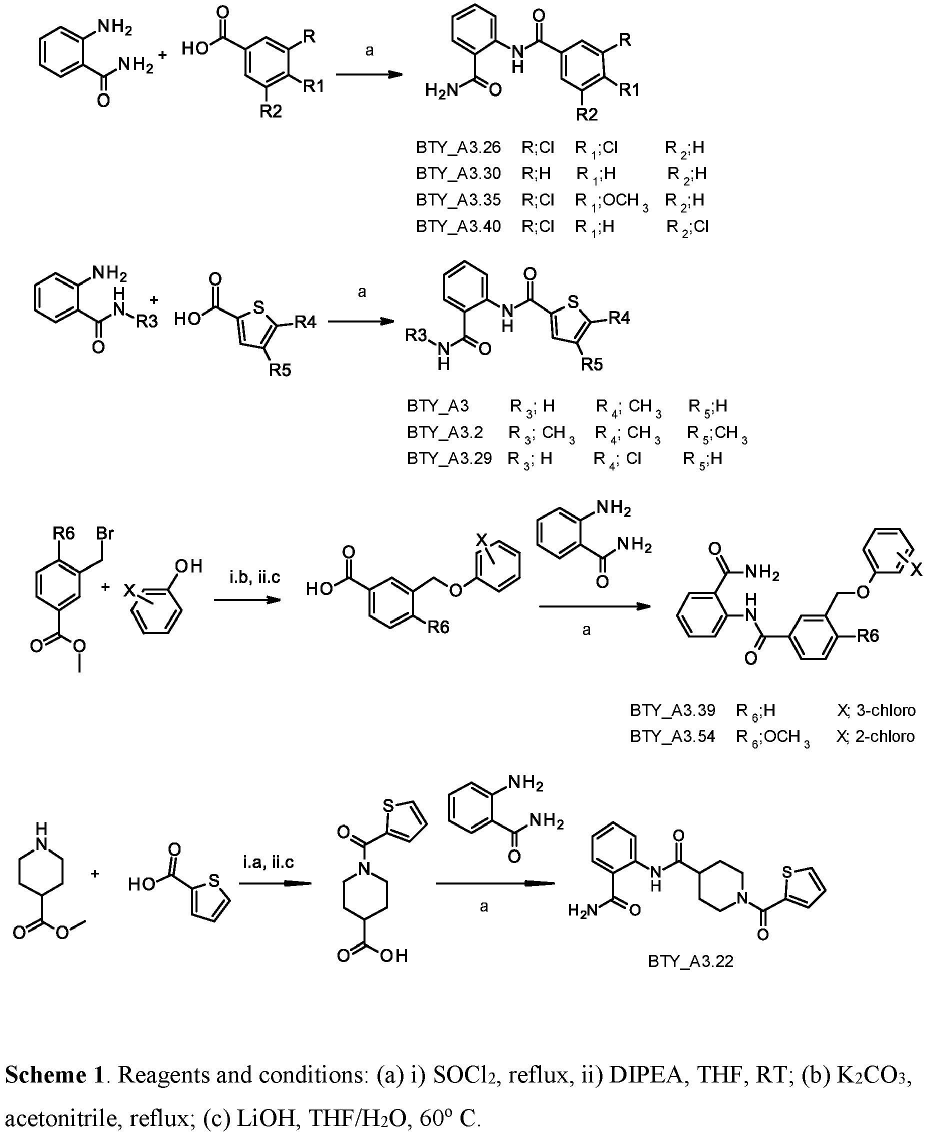

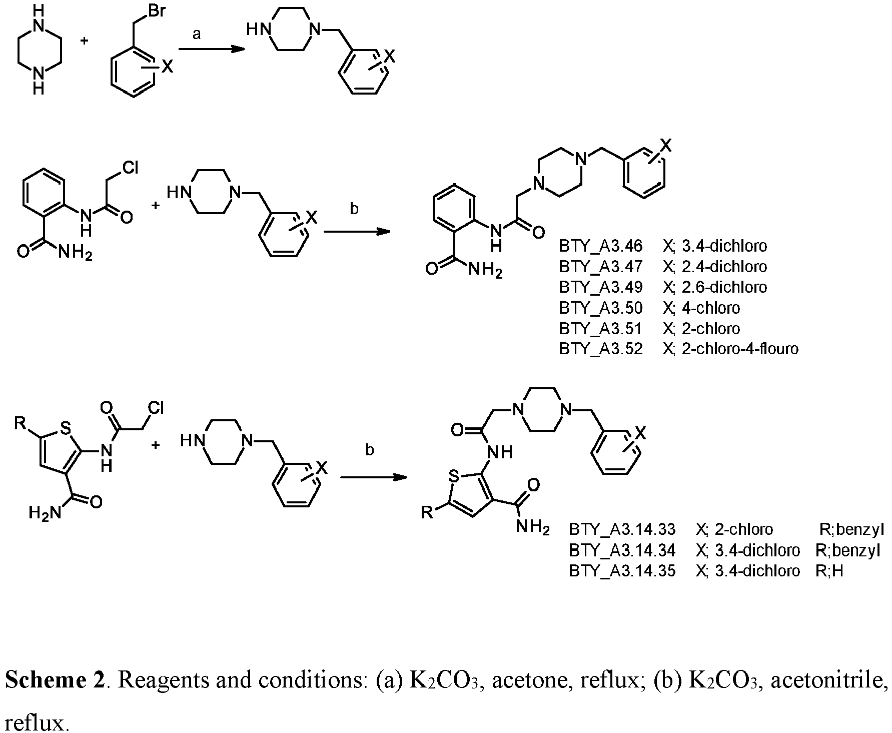

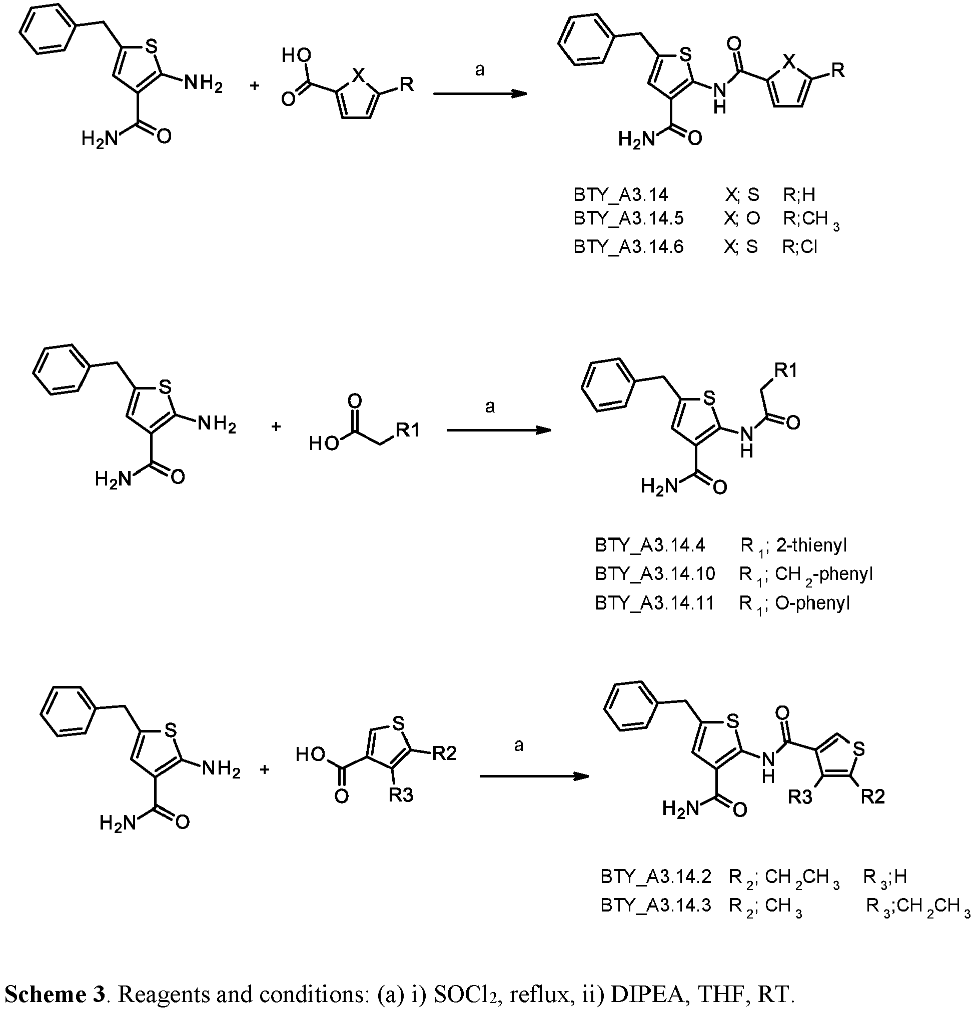

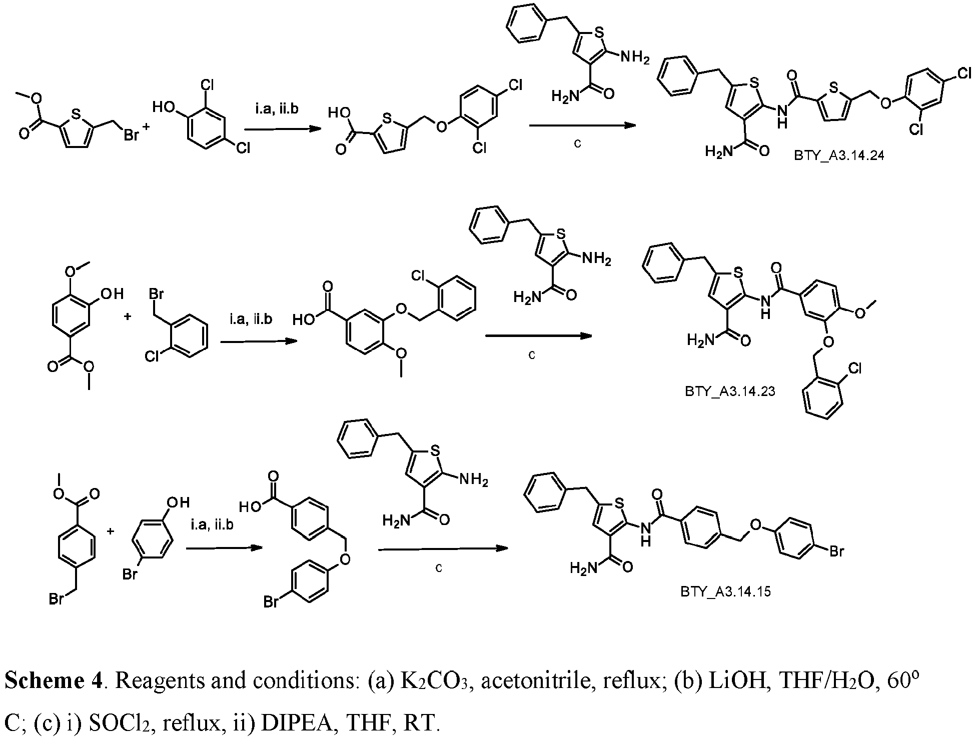

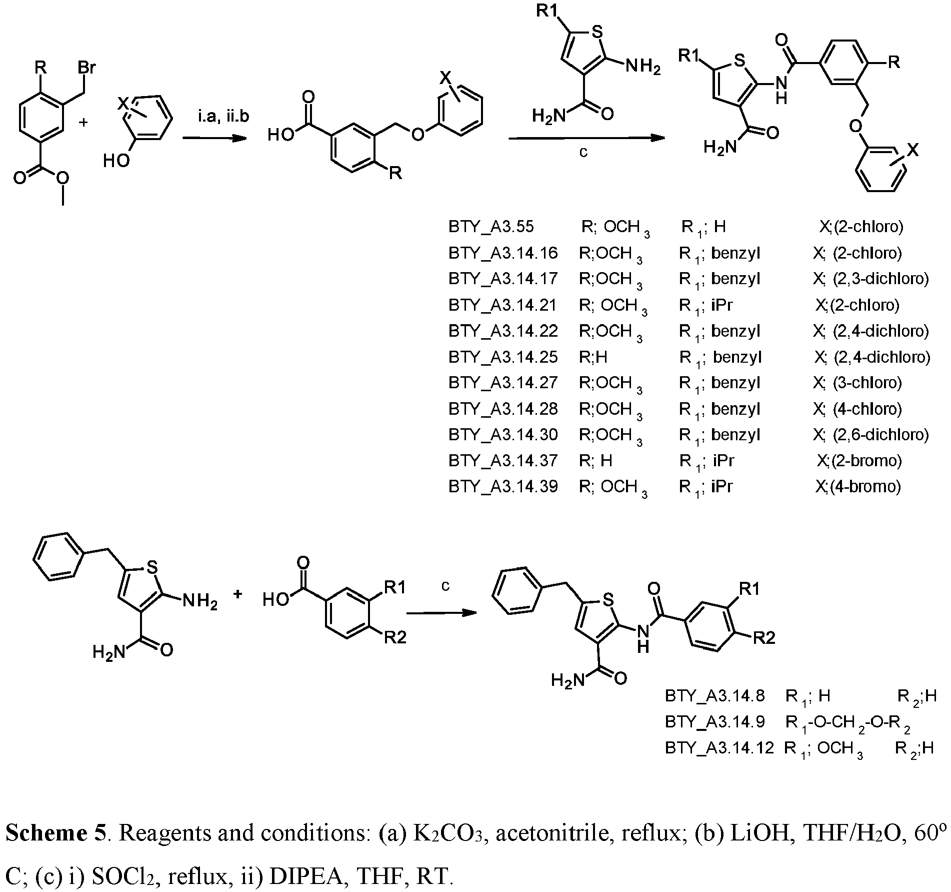

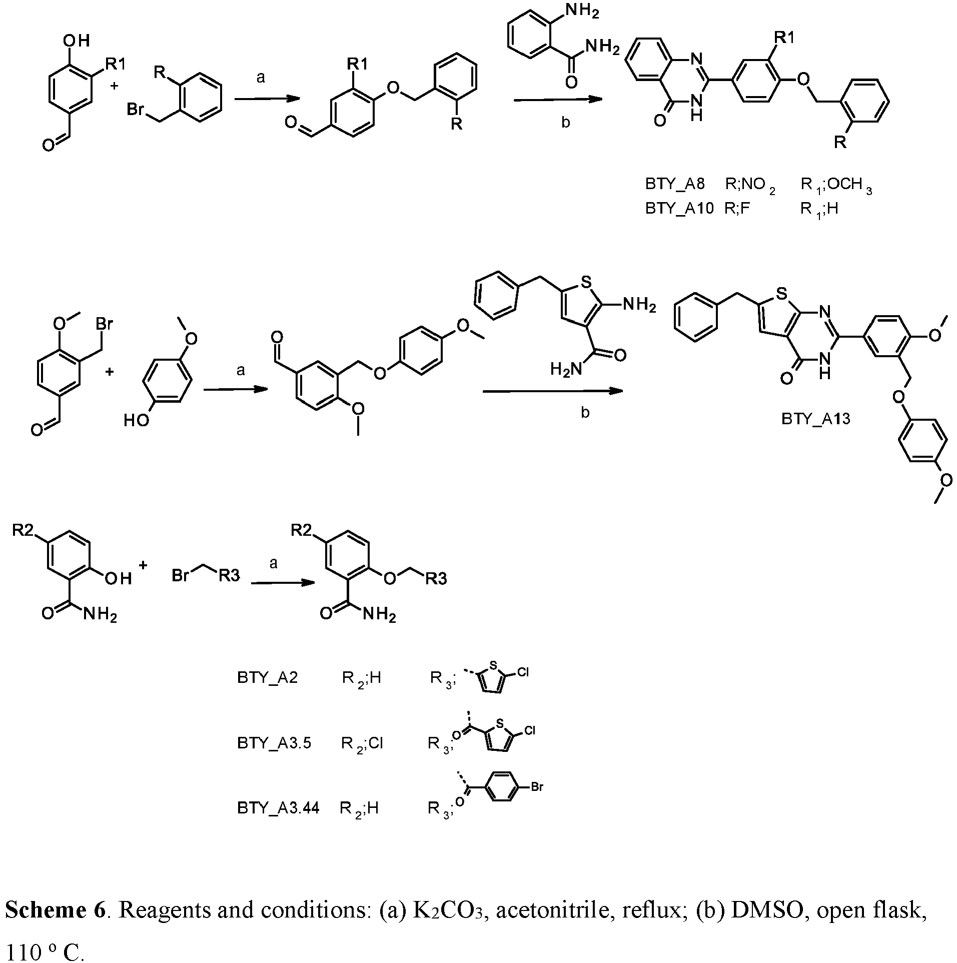

### Example of compounds

### BTY A2

### 2-[(5-chlorothiophen-2-yl)methoxy]benzamide

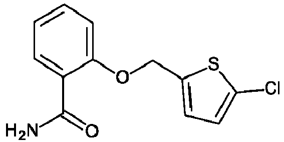

The title compound was synthesized from 2-(bromomethyl)-5-chlorothiophene and salicylamide using method B.
MS m/z: 268.00 [M+H]⁺
¹H NMR (400 MHz, DMSO) δ 7.75 (dt, *J* = 6.5, 3.3 Hz, 1H), 7.57 - 7.40 (m, 3H), 7.23 (d, *J* = 7.8 Hz, 1H), 7.13 (d, *J* = 3.8 Hz, 1H), 7.08 - 7.00 (m, 2H), 5.36 (s, 2H).
Purity 95% (HPLC)

### BTY A3

### N-[2-(aminocarbonyl)phenyl]-5-methyl-2-thiophenecarboxamide

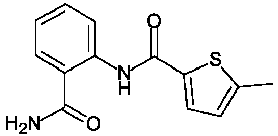

The title compound was synthesized from 5-methyl-2-thiophenecarboxylic acid and anthranilamide using method A.
MS m/z: 259.25 [M-H]⁻
¹H NMR (400 MHz, DMSO) δ 12.87 (s, 1H), 8.54 (dd, *J* = 8.4, 1.1 Hz, 1H), 8.39 (s, 1H), 7.87 (dd, *J* = 8.0, 1.4 Hz, 1H), 7.81 (s, 1H), 7.56 - 7.47 (m, 2H), 7.16-7.10 (m, 1H), 6.94 (dd, *J* = 3.7, 1.1 Hz, 1H), 2.50 (d, *J* = 0.8 Hz, 3H).
Purity 100% (HPLC)

### BTY A8

### 2-{3-Methoxy-4-[(2-nitrobenzyl)oxy]phenyl}-4(3H)-quinazolinone

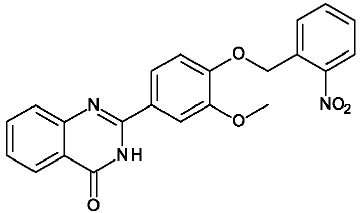

The title compound was synthesized starting from vanillin which reacted with 2-nitrobenzyl bromide using method B to afford 4-(2-nitrobenzyloxy)-3-methoxybenzaldehyde. This aldehyde was converted to the final compound using method F.
MS m/z: 402.40 [M-H]⁻
NMR (400 MHz, DMSO) δ 12.40 (s, 1H), 8.15-8.10 (m, 2H), 7.90-7.75 (m, 5H), 7.70-7.60 (m, 2H), 7.45 (t, *J* = 7.0 Hz, 1H), 7.10 (d, *J* = 8.0 Hz, 1H), 5.60 (s, 2H), 4.05 (s, 3H).
Purity 94% (HPLC)

### BTY A10

### 2-{4-[(2-Fluorobenzyl)oxy]phenyl}-4(3H)-quinazolinone

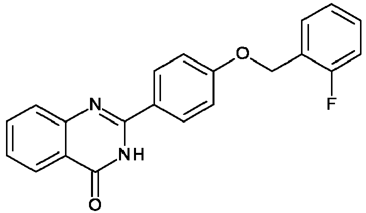

The title compound was synthesized starting from 4-hydroxybenzaldehyde which reacted with 2-fluorobenzyl bromide using method B to afford 4-(2-fluorobenzyloxy)benzaldehyde. This aldehyde was converted to the final compound using method F.
MS m/z: 345.36 [M-H]-
¹H NMR (400 MHz, DMSO) δ 12.50 (s, 1H), 8.20-8.10 (m, 3H), 7.80-7.65 (m, 3H), 7.45-7.40 (m, 2H), 7.25-7.10 (m, 4H), 5.30 (s, 2H).
Purity 95% (HPLC)

### BTY A13

### 6-Benzyl-2-{4-methoxy-3-[(4-methoxyphenoxy)methyl]phenyl}thieno[2,3-d]pyrimidin-4(3H)-one

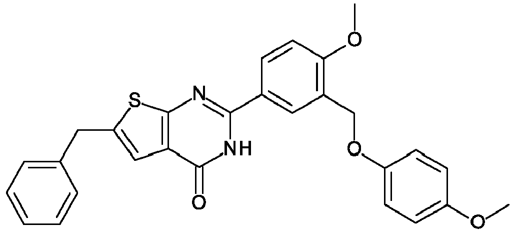

The title compound was synthesized starting from 3-(bromomethyl)-4-methoxybenzaldehyde which reacted with 4-methoxyphenol using method B to afford 4-methoxy-3-[(4-methoxyphenoxy)methyl]benzaldehyde. This aldehyde was converted to the final compound using method F.
MS m/z: 483.58 [M-H]-
¹H NMR (400 MHz, DMSO) δ 12.50 (s, 1H), 8.20-8.10 (m, 2H), 7.30-6.80 (m, 11H), 5.00 (s, 2H), 4.20 (s, 2H), 4.00 (s, 3H), 3.80 (s, 3H).
Purity 95% (HPLC)

### BTY_A14 (CB 7930921)

### N-(5-{[1-(4-oxo-3,4-dihydro-2-quinazolinyl)ethyl]thio}-1,3,4-thiadiazol-2-yl)benzamide

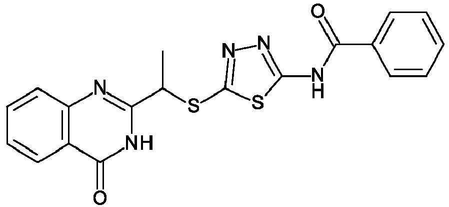

This compound was purchased from Chembridge Inc, San Diego, USA.
MS m/z: 408.49 [M-H]⁻
¹H NMR (400 MHz, DMSO) δ 13.10 (s, 1H), 12.50 (s, 1H), 8.20-8.10 (m, 3H), 7.80-7.65 (m, 1H), 7.65-7.45 (m, 5H), 4.85-4.80 (m, 1H), 1.80 (s, 3H).
Purity 95% (HPLC)

### BTY_A18 (CB 7764964)

### 2-Benzyl-6-methylthieno[2,3-d]pyrimidin-4(3H)-one

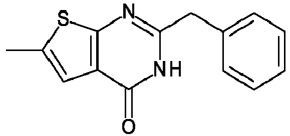

This compound was purchased from Chembridge Inc, San Diego, USA.
MS m/z: 255.33 [M-H]⁻
¹H NMR (400 MHz, DMSO) δ 12.30 (s, 1H), 7.35-7.20 (m, 5H), 6.95 (d, *J* = 1.0 Hz, 1H), 3.90 (s, 2H), 3.20 (s, 3H).
Purity 94% (HPLC)

### BTY_A3.2

### 4,5-Dimethyl-N-[2-(methylcarbamoyl)phenyl]thiophene-2-carboxamide

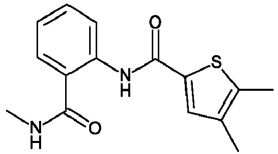

The title compound was synthesized from 4,5-dimethylthiophene-2-carboxylic acid and 2-amino-*N*-methylbenzamide using method A.
MS m/z: 287.19 [M-H]⁻
¹H NMR (400 MHz, DMSO) δ 12.55 (s, 1H), 8.83 (d, *J* = 4.1 Hz, 1H), 8.52 (dd, *J* = 8.4, 1.1 Hz, 1H), 7.79 (dd, *J* = 8.0, 1.4 Hz, 1H), 7.54 - 7.48 (m, 1H), 7.41 (s, 1H), 7.14 (td, *J* = 7.9, 1.2 Hz, 1H), 2.82 (d, *J* = 4.5 Hz, 3H), 2.36 (s, 3H), 2.15 (s, 3H).
Purity 95% (HPLC)

### BTY_A3.5

### 5-chloro-2-[2-(5-chlorothiophen-2-yl)-2-oxoethoxy]benzamide

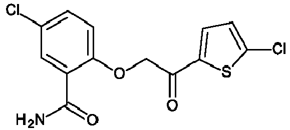

The title compound was synthesized from 2-bromo-1-(5-chlorothiophen-2-yl)ethanone and 5-chloro-2-hydroxybenzamide using method B.
MS m/z: 329.90 [M+H]⁺
¹H NMR (400 MHz, DMSO) δ 8.15 (s, 1H), 8.05 (d, *J* = 4.2 Hz, 1H), 7.85 (d, *J* = 2.8 Hz, 1H), 7.82 (s, 1H), 7.55 (dd, *J* = 8.8, 2.9 Hz, 1H), 7.39 (d, *J* = 4.1 Hz, 1H), 7.26 - 7.22 (m, 1H), 5.64 (s, 2H).
Purity 95% (HPLC)

### BTY_A3.11 (CB 7948757)

### N-[3-(aminocarbonyl)-2-thienyl]-2-thiophenecarboxamide

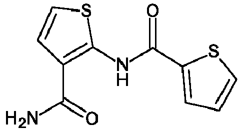

This compound was purchased from Chembridge Inc, San Diego, USA.
MS m/z: 251.30 [M-H]⁻
¹H NMR (400 MHz, DMSO) δ 13.23 (s, 1H), 7.98-7.90 (m, 2H), 7.85 (dd, *J* = 7.2, 1.2 Hz, 1H), 7.70 (dd, *J* = 5.0, 1.2 Hz, 1H), 7.55 (d, *J* = 8.8 Hz, 1H), 7.42 (d, *J* = 8.8 Hz, 1H), 7.21 (dd, *J* = 7.2, 5.0 Hz, 1H).
Purity 90% (HPLC)

### BTY_A3.22

### N-[2-(aminocarbonyl)phenyl]-1-(2-thienylcarbonyl)-4-piperidinecarboxamide

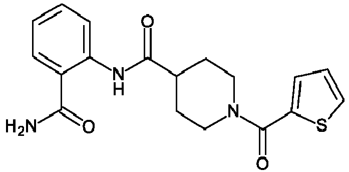

The title compound was synthesized starting from thiophene-2-carboxylic acid through the reaction with methyl isonipecotate using method A, followed by hydrolysis of the ester product using method C to afford 1-(thiophene-2-carbonyl)piperidine-4-carboxylic acid. This acid was reacted with anthranilamide using method A to give the final compound.
MS m/z: 358.00 [M+H]⁺
¹H NMR (400 MHz, DMSO) δ 11.84 (s, 1H), 8.46 (dd, *J* = 8.4, 1.0 Hz, 1H), 8.26 (s, 1H), 7.79 (dd, *J* = 7.9, 1.4 Hz, 1H), 7.75 - 7.67 (m, 2H), 7.50 - 7.44 (m, 1H), 7.38 (dd, *J* = 3.6, 1.1 Hz, 1H), 7.13 - 7.06 (m, 2H), 4.30-4.22 (m, 2H), 3.18-3.03 (m, 2H), 2.68-2.59 (m, 1H), 1.97-1.90 (m, 2H), 1.66-1.53 (m, 2H).
Purity 93% (HPLC)

### BTY_A3.26

### N-[2-(aminocarbonyl)phenyl]-3,4-dichlorobenzamide

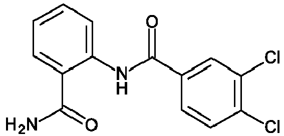

The title compound was synthesized from 3,4-dichlorobenzoic acid and anthranilamide using method A.
MS m/z: 308.15 [M-H]⁻
¹H NMR (400 MHz, DMSO) δ 12.92 (s, 1H), 8.65 (dd, *J* = 8.4, 1.0 Hz, 1H), 8.42 (s, 1H), 7.97 - 7.86 (m, 3H), 7.82 (s, 1H), 7.86 (dd, J = 1.8, 0.5 Hz, 1H), 7.76 (dd, J = 8.6, 0.5 Hz, 1H), 7.70 (dd, J = 8.6, 1.8 Hz, 1H), 7.62 - 7.54 (m, 4H).
Purity 90% (HPLC)

### BTY A3.29

### N-(2-carbamoylphenyl)-5-chlorothiophene-2-carboxamide

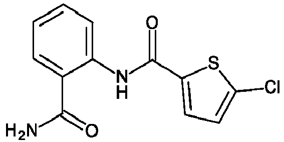

The title compound was synthesized from 5-chlorothiophene-2-carboxylic acid and anthranilamide using method A.
MS m/z: 279.14 [M-H]⁻
¹H NMR (400 MHz, DMSO) δ 13.03 (s, 1H), 8.49 (dd, *J* = 8.4, 1.0 Hz, 1H), 8.42 (s, 1H), 7.91 - 7.82 (m, 2H), 7.57 - 7.50 (m, 2H), 7.28 (d, *J* = 4.1 Hz, 1H), 7.17 (td, *J* = 7.9, 1.2 Hz, 1H).
Purity 99% (HPLC)

### BTY_A3.30

### N-(2-carbamoylphenyl)benzamide

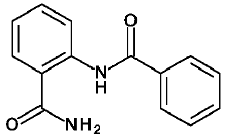

The title compound was synthesized from benzoic acid and anthranilamide using method A.
MS m/z: 239.25 [M-H]⁻
¹H NMR (400 MHz, DMSO) δ 12.94 (s, 1H), 8.69 (dd, *J* = 8.4, 1.0 Hz, 1H), 8.40 (s, 1H), 7.97 - 7.86 (m, 3H), 7.82 (s, 1H), 7.64 - 7.52 (m, 4H), 7.16 (td, *J* = 7.9, 1.2 Hz, 1H).
Purity 100% (HPLC)

### BTY_A3.34

### 2-{[(5-Ethyl-3-thienyl)carbonyl]amino}-3-thiophenecarboxamide

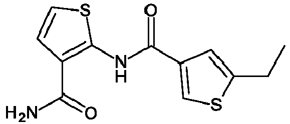

This compound was purchased from Chembridge Inc, San Diego, USA.
MS m/z: 279.37 [M-H]⁻
¹H NMR (400 MHz, DMSO) δ 13.10 (s, 1H), 8.00-7.80 (m, 2H), 7.50-7.35 (m, 2H), 7.2 (d, *J* = 1.1 Hz, 1H), 6.80 (d, *J* = 8.4 Hz, 1H), 2.90 (q, *J* = 7.0 Hz, 2H), 1.32 (t, J = 7.0 Hz, 3H).
Purity 95% (HPLC)

### BTY_A3.35

### N-[2-(aminocarbonyl)phenyl]-3-chloro-4-methoxybenzamide

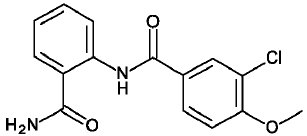

The title compound was synthesized from 3-chloro-4-methoxybenzoic acid and anthranilamide using method A.
MS m/z: 303.74 [M-H]⁻
¹H NMR (400 MHz, DMSO) δ 13.05 (s, 1H), 8.70 (d, *J* = 8.0 Hz, 1H), 8.40 (s, 1H), 8.00-7.90 (m, 3H), 7.80 (s, 1H), 7.55 (t, J = 8.0 Hz, 1H), 7.30 (d, *J* = 8.0 Hz, 1H), 7.15 (t, J = 8.0 Hz, 1H), 4.00 (s, 3H).
Purity 95% (HPLC)

### BTY_A3.39

### 2-({3-[(3-Chlorophenoxy)methyl]benzoyl}amino)benzamide

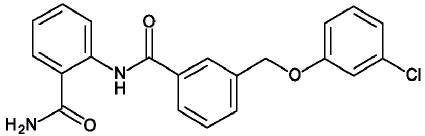

The title compound was synthesized starting from methyl 3-(bromomethyl)benzoate and 3-chlorophenol using method B followed by hydrolysis of the ester product using method C to afford 3-[(3-chlorophenoxy)methyl]benzoic acid. This acid was reacted with anthranilamide using method A to give the final compound.
MS m/z: 379.83 [M-H]⁻
¹H NMR (400 MHz, DMSO) δ 13.00 (s, 1H), 8.80 (d, *J* = 2.3 Hz, 1H), 8.20-8.00 (m, 2H), 7.90-7.85 (m, 2H), 7.70-7.40 (m, 4H), 7.30 (t, *J* = 7.0 Hz, 1H), 7.20-6.90 (m, 4H), 5.19 (s, 2H). Purity 95% (HPLC)

### BTY_A3.40

### N-[2-(aminocarbonyl)phenyl]-3,5-dichlorobenzamide

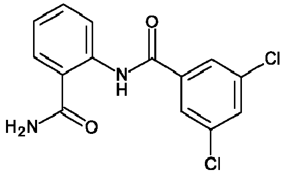

The title compound was synthesized from 3,5-dichlorobenzoic acid and anthranilamide using method A.
MS m/z: 308.15 [M-H]⁻
¹H NMR (400 MHz, DMSO) δ 13.20 (s, 1H), 8.65 (d, *J* = 8.0 Hz, 1H), 8.40 (s, 1H), 7.90-7.70 (m, 5H), 7.55 (t, *J* = 8.0 Hz, 1H), 7.15 (t, *J* = 8.0 Hz, 1H).
Purity 93% (HPLC)

### BTY_A3.44

### 2-[2-(4-Bromophenyl)-2-oxoethoxy]benzamide

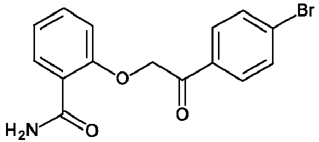

The title compound was synthesized from 2-bromo-1-(4-bromophenyl)ethanone and salicylamide using method B.
MS m/z: 333.17 [M-H]⁻
¹H NMR (400 MHz, DMSO) δ 8.40 (s, 1H), 8.05-8.00 (m, 3H), 7.75 (d, *J* = 9.0 Hz, 2H), 7.60-7.40 (m, 2H), 7.25 (d, *J* = 8.0 Hz, 1H), 7.10 (t, *J* = 8.0 Hz, 1H), 5.70 (s, 2H).
Purity 93% (HPLC)

### BTY_A3.46

### 2-({[4-(3,4-Dichlorobenzyl)-1-piperazinyl]acetyl}amino)benzamide

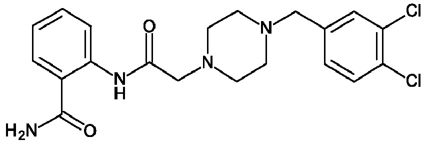

The title compound was synthesized starting from 3,4-dichlorobenzyl bromide through reaction with piperazine using method E to give 1-(3,4-dichlorobenzyl)piperazine, which was then reacted with 2-(2-chloroacetamido)benzamide using method E to give the final compound. MS m/z: 422.33 [M+H]⁺
¹H NMR (400 MHz, DMSO) δ 12.20 (s, 1H), 8.60 (d, *J* = 2.3 Hz, 1H), 8.00 (s, 1H), 7.75 (d, *J* = 2.3 Hz, 1H), 7.60-7.25 (m, 5H), 7.10 (t, *J* = 7.0 Hz, 1H), 3.50 (s, 2H), 3.30-3.20 (m, 6H), 2.60-2.40 (m, 4H).
Purity 95% (HPLC)

### BTY_A3.47

### 2-({[4-(2,4-Dichlorobenzyl)-1-piperazinyl]acetyl}amino)benzamide

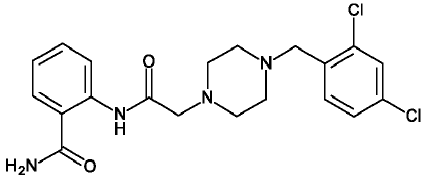

The title compound was synthesized starting from 2,4-dichlorobenzyl bromide through reaction with piperazine using general E to give 1-(2,4-dichlorobenzyl)piperazine, which was then reacted with 2-(2-chloroacetamido)benzamide using method E to give the final compound.
MS m/z: 422.33 [M+H]⁺
¹H NMR (400 MHz, DMSO) δ 12.10 (s, 1H), 8.70 (d, *J* = 2.3 Hz, 1H), 8.00 (s, 1H), 7.75 (d, *J* = 2.3 Hz, 1H), 7.50 (d, *J* = 8.0 Hz, 1H), 7.40-7.30 (m, 3H), 7.25 (dd, *J* = 8.0, 1.4 Hz, 1H), 7.10 (t, *J* = 7.0 Hz, 1H), 3.50 (s, 2H), 3.30-3.20 (m, 6H), 2.60-2.40 (m, 4H).
Purity 95% (HPLC)

### BTY_A3.49

### 2-({[4-(2,6-Dichlorobenzyl)-1-piperazinyl]acetyl}amino)benzamide

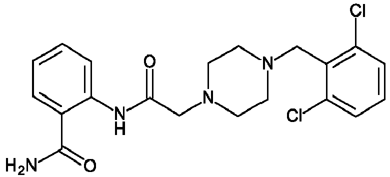

The title compound was synthesized starting from 2,6-dichlorobenzyl bromide through reaction with piperazine using method E to give 1-(2,6-dichlorobenzyl)piperazine, which was then reacted with 2-(2-chloroacetamido)benzamide using method E to give the final compound.
MS m/z: 422.33 [M+H]⁺
¹H NMR (400 MHz, DMSO) δ 12.10 (s, 1H), 8.60 (d, *J* = 8.0 Hz, 1H), 8.00 (s, 1H), 7.75 (d, *J* = 8.0 Hz, 1H), 7.50-7.25 (m, 5H), 7.05 (t, *J* = 8.0 Hz, 1H), 3.60 (s, 2H), 3.00 (s, 2H), 3.50-2.40 (m, 8H).
Purity 95% (HPLC)

### BTY_A3.50

### 2-({[4-(4-Chlorobenzyl)-1-piperazinyl]acetyl}amino)benzamide

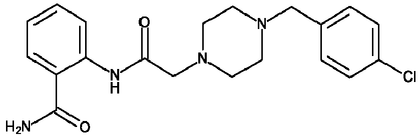

The title compound was synthesized starting from 4-chlorobenzyl bromide through reaction with piperazine using method E to give 1-(4-chlorobenzyl)piperazine, which was then reacted with 2-(2-chloroacetamido)benzamide using method E to give the final compound.
MS m/z: 387.88 [M+H]⁺
¹H NMR (400 MHz, DMSO) δ 12.00 (s, 1H), 8.55 (d, *J* = 8.0 Hz, 1H), 8.00 (s, 1H), 7.70 (d, *J* = 8.0 Hz, 1H), 7.50-7.30 (m, 6H), 7.05 (t, *J* = 8.0 Hz, 1H), 3.60 (s, 2H), 3.00 (s, 2H), 3.50-2.40 (m, 8H).
Purity 95% (HPLC)

### BTY_A3.51

### 2-({[4-(2-Chlorobenzyl)-1-piperazinyl]acetyl}amino)benzamide

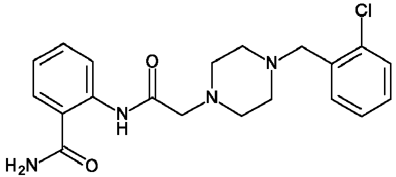

The title compound was synthesized starting from 2-chlorobenzyl bromide through reaction with piperazine using method E to give 1-(2-chlorobenzyl)piperazine, which was then reacted with 2-(2-chloroacetamido)benzamide using method E to give the final compound.
MS m/z: 387.88 [M+H]⁺
¹H NMR (400 MHz, DMSO) δ 12.05 (s, 1H), 8.60 (d, *J* = 8.0 Hz, 1H), 8.05 (s, 1H), 7.75 (d, *J* = 8.0 Hz, 1H), 7.50-7.30 (m, 6H), 7.05 (t, *J* = 8.0 Hz, 1H), 3.60 (s, 2H), 3.00 (s, 2H), 3.50-2.40 (m, 8H).
Purity 95% (HPLC)

### BTY_A3.52

### 2-({[4-(2-Chloro-4-fluorobenzyl)-1-piperazinyl]acetyl}amino)benzamide

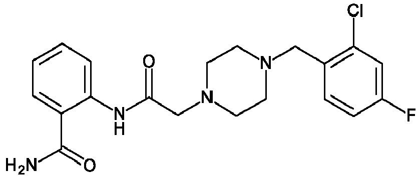

The title compound was synthesized starting from 2-chloro-4-fluorobenzyl bromide through reaction with piperazine using method E to give 1-(2-chloro-4-fluorobenzyl)piperazine, which was then reacted with 2-(2-chloroacetamido)benzamide using method E to give the final compound.
MS m/z: 405.88 [M+H]⁺
¹H NMR (400 MHz, DMSO) δ 12.10 (s, 1H), 8.60 (d, *J* = 8.0 Hz, 1H), 8.05 (s, 1H), 7.75 (d, *J* = 8.0 Hz, 1H), 7.50-7.10 (m, 6H), 3.60 (s, 2H), 3.00 (s, 2H), 3.50-2.40 (m, 8H).
Purity 95% (HPLC)

### BTY_A3.54

### 2-{3-[(2-Chlorophenoxy)methyl]-4-methoxybenzamido}benzamide

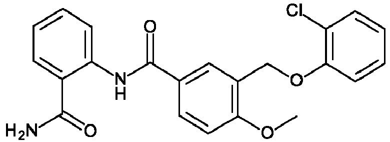

The title compound was synthesized starting from methyl 3-(bromomethyl)-4-methoxybenzoate which reacted with 2-chlorophenol using method B followed by hydrolysis of the ester product using method C to afford 3-[(2-chlorophenoxy)methyl]-4-methoxybenzoic acid. This acid was reacted with anthranilamide using method A to give the final compound.
MS m/z: 409.32 [M-H]⁻
¹H NMR (400 MHz, DMSO) δ 12.85 (d, *J* = 16.7 Hz, 1H), 8.66 (dd, *J* = 8.4, 1.0 Hz, 1H), 8.37 (s, 1H), 8.08 (d, *J* = 2.3 Hz, 1H), 7.93 (dd, *J* = 8.7, 2.4 Hz, 1H), 7.87 (dd, *J* = 8.0, 1.4 Hz, 1H), 7.79 (s, 1H), 7.57 - 7.50 (m, 3H), 7.43 (dd, *J* = 7.9, 1.6 Hz, 1H), 7.33 - 7.20 (m, 1H), 7.13 (dt, *J* = 12.5, 2.9 Hz, 1H), 6.96 (td, *J* = 7.8, 1.5 Hz, 1H), 5.19 (s, 2H), 3.91 (s, 3H).
Purity 95% (HPLC)

### BTY_A3.55

### 2-{3-[(2-Chlorophenoxy)methyl]-4-methoxybenzamido}thiophene-3-carboxamide

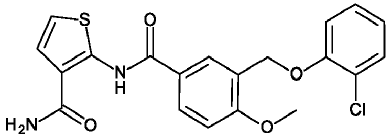

The title compound was synthesized starting from methyl 3-(bromomethyl)-4-methoxybenzoate which reacted with 2-chlorophenol using method B followed by hydrolysis of the ester product using method C to afford 3-[(2-chlorophenoxy)methyl]-4-methoxybenzoic acid. This acid was reacted with 2-aminothiophene-3-carboxamide using method A to give the final compound.
MS m/z: 415.22 [M-H]⁻
¹H NMR (400 MHz, DMSO) δ 13.32 (d, *J* = 12.7 Hz, 1H), 8.08 (d, *J* = 2.3 Hz, 1H), 8.00 (s, 1H), 7.89 (dd, *J* = 8.7, 2.4 Hz, 1H), 7.58 (d, *J* = 2.4 Hz, 1H), 7.48 - 7.41 (m, 2H), 7.30 (td, *J* = 7.3, 1.4 Hz, 2H), 7.23 (dd, *J* = 8.3, 1.5 Hz, 1H), 7.01 - 6.94 (m, 2H), 5.21 (s, 2H), 3.93 (s, 3H).
Purity 96% (HPLC)

### BTY_A3.57 (CB 6890496)

### 2-({3-[4-(Benzyloxy)-3-methoxyphenyl]acryloyl}amino)benzamide

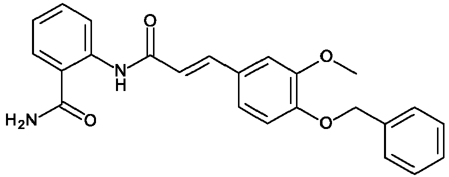

This compound was purchased from Chembridge Inc, San Diego, USA.
MS m/z: 401.45 [M-H]⁻
¹H NMR (400 MHz, DMSO) δ 12.00 (s, 1H), 8.60 (d, *J* = 2.3 Hz, 1H), 8.20 (s, 1H), 7.80 (d, *J* = 8.0 Hz, 1H), 7.60-7.30 (m, 7H), 7.20-7.00 (m, 5H), 6.60 (d, *J* = 15 Hz, 1H), 5.19 (s, 2H), 3.90 (s, 3H).
Purity 96% (HPLC)

### BTY_A3.63 (CB 9085496)

### 2-{[(5-Chloro-2-thienyl)carbonyl]amino}-5,6-dihydro-4H-cyclopenta[b]thiophene-3-carboxamide

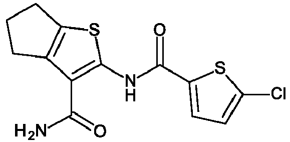

This compound was purchased from Chembridge Inc, San Diego, USA.
MS m/z: 325.83 [M-H]⁻
¹H NMR (400 MHz, DMSO) δ 13.20 (s, 1H), 7.60-7.50 (m, 2H), 7.20 (d, *J* = 1.0 Hz, 1H), 6.75 (s, 1H), 2.90-2.80 (m, 4H), 2.45-2.30 (m, 2H).
Purity 95% (HPLC)

### BTY_A3.64 (CB 9085855)

### 2-{[(5-Chloro-2-thienyl)carbonyl]amino}-4,5,6,7-tetrahydro-1-benzothiophene-3-carboxamide

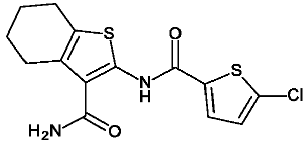

This compound was purchased from Chembridge Inc, San Diego, USA.
MS m/z: 339.85 [M-H]⁻
¹H NMR (400 MHz, DMSO) δ 13.10 (s, 1H), 7.60-7.50 (m, 2H), 7.20-6.90 (m, 2H), 2.90-2.80 (m, 4H), 1.80-1.60 (m, 4H).
Purity 95% (HPLC)

### BTY_A3.14

### N-[3-(aminocarbonyl)-5-benzyl-2-thienyl]-2-thiophenecarboxamide

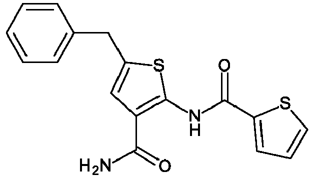

The title compound was synthesized from thiophene-2-carboxylic acid and 2-amino-5-benzylthiophene-3-carboxamide using method A.
MS m/z: 341.26 [M-H]⁻
¹H NMR (400 MHz, DMSO) δ 13.23 (s, 1H), 7.98-7.90 (m, 2H), 7.66 (dd, J = 3.8, 1.1 Hz, 1H), 7.58 (s, 1H), 7.35 - 7.15 (m, 7H), 4.05 (s, 2H).
Purity 99% (HPLC)

### BTY_A3.14.2

### 5-Benzyl-2-{[(5-ethyl-3-thienyl)carbonyl]amino}-3-thiophenecarboxamide

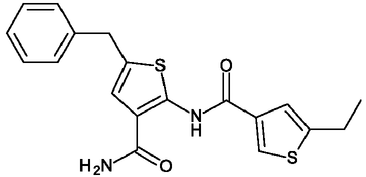

The title compound was synthesized from 5-ethyl-3-thiophenecarboxylic acid and 2-amino-5-benzylthiophene-3-carboxamide using method A.
MS m/z: 369.50 [M-H]⁻
¹H NMR (400 MHz, DMSO) δ 13.10 (s, 1H), 7.90-7.80 (m, 2H), 7.40-6.90 (m, 8H), 4.00 (s, 2H), 2.85 (q, *J* = 7.0 Hz, 2H), 1.32 (t, J = 7.0 Hz, 3H).
Purity 95% (HPLC)

### BTY_A3.14.3

### 5-Benzyl-2-{[(4-ethyl-5-methyl-3-thienyl)carbonyl]amino}-3-thiophenecarboxamide

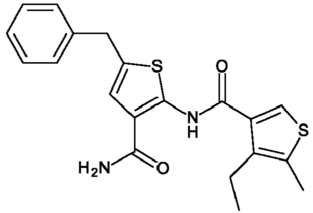

The title compound was synthesized from 4-ethyl-5-methyl-3-thiophenecarboxylic acid and 2-amino-5-benzylthiophene-3-carboxamide using method A.
MS m/z: 383.52 [M-H]⁻
¹H NMR (400 MHz, DMSO) δ 12.90 (s, 1H), 7.80-7.70 (m, 2H), 7.50-7.20 (m, 7H), 4.05 (s, 2H), 2.85 (q, *J* = 7.0 Hz, 2H), 2.30 (s, 3H), 1.10 (t, J = 7.0 Hz, 3H).
Purity 95% (HPLC)

### BTY_A3.14.4

### 5-Benzyl-2-[(2-thienylacetyl)amino]-3-thiophenecarboxamide

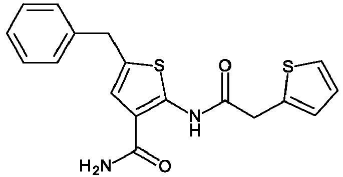

The title compound was synthesized from 2-thienylacetic acid and 2-amino-5-benzylthiophene-3-carboxamide using method A.
MS m/z: 383.47 [M-H]⁻
¹H NMR (400 MHz, DMSO) δ 12.20 (s, 1H), 7.70 (s, 1H), 7.50-7.20 (m, 8H), 7.10-7.00 (m, 2H), 4.10-4.00 (2s, each 2H).
Purity 95% (HPLC)

### BTY_A3.14.5

### N-[3-(Aminocarbonyl)-5-benzyl-2-thienyl]-5-methyl-2-furamide

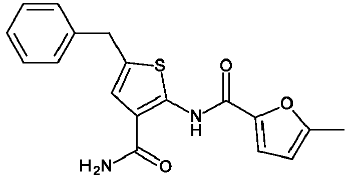

The title compound was synthesized from 5-methyl-2-furoic acid and 2-amino-5-benzylthiophene-3-carboxamide using method A.
MS m/z: 339.40 [M-H]⁻
¹H NMR (400 MHz, DMSO) δ 13.10 (s, 1H), 7.80 (s, 1H), 7.40-7.20 (m, 7H), 7.10 (d, *J* = 8.0 Hz, 1H), 6.30 (d, *J* = 1.3 Hz, 1H), 4.10 (s, 2H), 2.40 (s, 3H).
Purity 95% (HPLC)

### BTY_A3.14.6

### N-[3-(Aminocarbonyl)-5-benzyl-2-thienyl]-5-chloro-2-thiophenecarboxamide

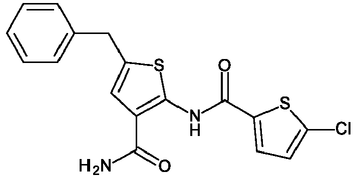

The title compound was synthesized from 5-chloro-2-furoic acid and 2-amino-5-benzylthiophene-3-carboxamide using method A.
MS m/z: 375.89 [M-H]⁻
¹H NMR (400 MHz, DMSO) δ 13.30 (s, 1H), 7.85 (s, 1H), 7.55-7.40 (m, 2H), 7.35-7.20 (m, 7H), 4.05 (s, 2H).
Purity 96% (HPLC)

### BTY_A3.14.8

### 2-(Benzoylamino)-5-benzyl-3-thiophenecarboxamide

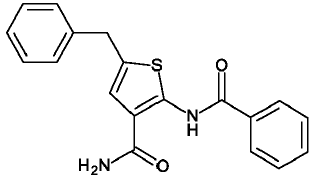

The title compound was synthesized from benzoic acid and 2-amino-5-benzylthiophene-3-carboxamide using method A.
MS m/z: 335.42 [M-H]⁻
¹H NMR (400 MHz, DMSO) δ 13.40 (s, 1H), 7.95-7.85 (m, 3H), 7.65-7.55 (m, 3H), 7.45-7.20 (m, 7H), 4.05 (s, 2H).
Purity 92% (HPLC)

### BTY_A3.14.9

### N-[3-(aminocarbonyl)-5-benzyl-2-thienyl]-1,3-benzodioxole-5-carboxamide

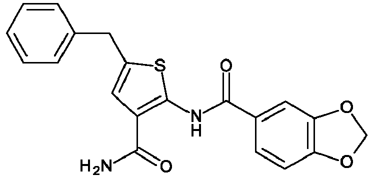

The title compound was synthesized from piperonylic acid and 2-amino-5-benzylthiophene-3-carboxamide using method A.
MS m/z: 379.43 [M-H]⁻
¹H NMR (400 MHz, DMSO) δ 13.20 (s, 1H), 7.85 (s, 1H), 7.45-7.40 (m, 2H), 7.35-7.20 (m, 7H), 7.05 (d, *J* = 8.0 Hz, 1H), 6.10 (s, 2H), 4.10 (s, 2H).
Purity 92% (HPLC)

### BTY_A3.14.10

### 5-Benzyl-2-[(3-phenylpropanoyl)amino]-3-thiophenecarboxamide

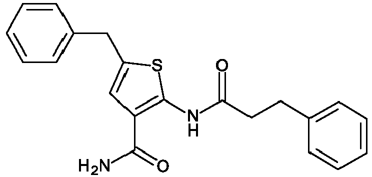

The title compound was synthesized from hydrocinnamic acid and 2-amino-5-benzylthiophene-3-carboxamide using method A.
MS m/z: 363.47 [M-H]⁻
¹H NMR (400 MHz, DMSO) δ 12.10 (s, 1H), 7.70 (s, 1H), 7.35-7.15 (m, 12H), 4.15 (s, 2H), 2.80-2.75 (m, 2H), 2.65-2.60 (m, 2H).
Purity 94% (HPLC)

### BTY_A3.14.11

### 5-Benzyl-2-[(phenoxyacetyl)amino]-3-thiophenecarboxamide

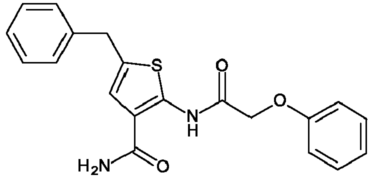

The title compound was synthesized from phenoxyacetic acid and 2-amino-5-benzylthiophene-3-carboxamide using method A.
MS m/z: 365.44 [M-H]⁻
¹H NMR (400 MHz, DMSO) δ 12.75 (s, 1H), 7.80 (s, 1H), 7.50-7.30 (m, 9H), 7.05-6.95 (m, 3H), 4.60 (s, 2H), 4.00 (s, 2H).
Purity 95% (HPLC)

### BTY_A3.14.12

### 5-Benzyl-2-[(4-methoxybenzoyl)amino]-3-thiophenecarboxamide

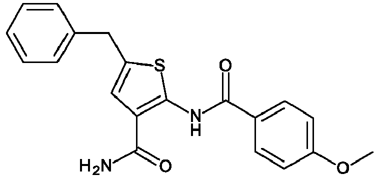

The title compound was synthesized from 4-methoxybenzoic acid and 2-amino-5-benzylthiophene-3-carboxamide using method A.
MS m/z: 365.43 [M-H]⁻
¹H NMR (400 MHz, DMSO) δ 13.20 (s, 1H), 7.90-7.80 (m, 3H), 7.50-7.30 (m, 7H), 7.10-7.05 (m, 2H), 4.10 (s, 2H), 3.85 (s, 3H).
Purity 95% (HPLC)

### BTY_A3.14.14 (CB 8803228)

### 2-{[(3,4-Dichlorophenyl)acetyl]amino}-5-isopropyl-3-thiophenecarboxamide

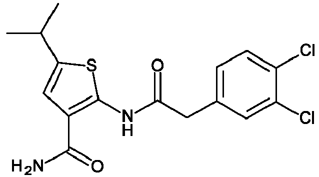

This compound was purchased from Chembridge Inc, San Diego, USA.
MS m/z: 418.33 [M-H]⁻
¹H NMR (400 MHz, DMSO) δ 12.10 (s, 1H), 7.65-7.50 (m, 3H), 7.30-7.20 (m, 2H), 7.00 (s, 1H), 3.80 (s, 2H), 3.10-3.05 (m, 1H), 1.30 (m, 6H).
Purity 94% (HPLC)

### BTY_A3.14.15

### 5-Benzyl-2-({4-[(4-bromophenoxy)methyl]benzoyl}amino)-3-thiophenecarboxamide

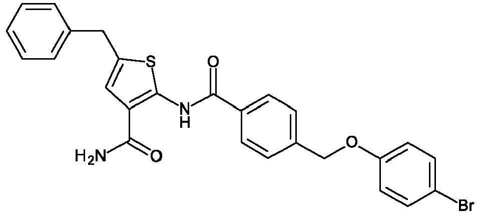

The title compound was synthesized starting from methyl 4-(bromomethyl)benzoate which reacted with 4-bromophenol using method B followed by hydrolysis of the ester product using method C to afford 4-[(4-bromophenoxy)methyl]benzoic acid. This acid was reacted with 2-amino-5-benzylthiophene-3-carboxamide using method A to give the final compound.
MS m/z: 520.44 [M-H]⁻
¹H NMR (400 MHz, DMSO) δ 13.40 (s, 1H), 8.00-7.90 (m, 2H), 7.60 (d, *J* = 8.0 Hz, 1H), 7.40-7.20 (m, 11H), 6.95 (d, *J* = 8.0 Hz, 2H), 5.20 (s, 2H), 3.95 (s, 2H).
Purity 95% (HPLC)

### BTY_A3.14.16

### 5-Benzyl-2-({3-[(2-chlorophenoxy)methyl]-4-methoxybenzoyl}amino)-3-thiophenecarboxamide

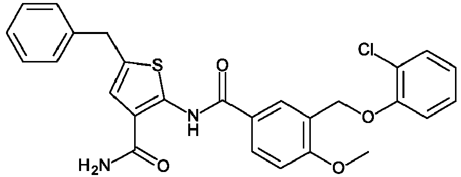

The title compound was synthesized starting from methyl 3-(bromomethyl)-4-methoxybenzoate which reacted with 2-chlorophenol using method B followed by hydrolysis of the ester product using method C to afford 3-[(2-chlorophenoxy)methyl]-4-methoxybenzoic acid. This acid was reacted with 2-amino-5-benzylthiophene-3-carboxamide using method A to give the final compound.
MS m/z: 505.35 [M-H]⁻
¹H NMR (400 MHz, DMSO) δ 13.26 (s, 1H), 8.04 (d, *J* = 2.3 Hz, 1H), 7.92 (s, 1H), 7.84 (dd, *J* = 8.7, 2.3 Hz, 1H), 7.52 (s, 1H), 7.43 (dd, *J* = 7.9, 1.6 Hz, 1H), 7.34 - 7.19 (m, 9H), 6.99 - 6.93 (m, 1H), 5.19 (s, 2H), 4.04 (s, 2H), 3.92 (s, 2H).
Purity 98% (HPLC)

### BTY_A3.14.17

### 5-Benzyl-2-({3-[(2,3-dichlorophenoxy)methyl]-4-methoxybenzoyl}amino)-3-thiophenecarboxamide

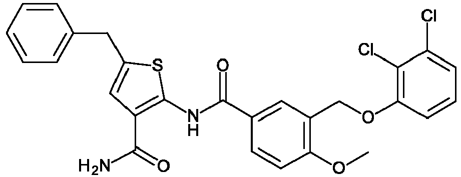

The title compound was synthesized starting from methyl 3-(bromomethyl)-4-methoxybenzoate which reacted with 2,3-dichlorophenol using method B followed by hydrolysis of the ester product using method C to afford 3-[(2,3-dichlorophenoxy)methyl]-4-methoxybenzoic acid. This acid was reacted with 2-amino-5-benzylthiophene-3-carboxamide using method A to give the final compound.
MS m/z: 539.19 [M-H]⁻
¹H NMR (400 MHz, DMSO) δ 13.26 (s, 1H), 8.03 (d, J = 2.3 Hz, 1H), 7.92 (s, 1H), 7.86 (dd, J = 8.7, 2.4 Hz, 1H), 7.52 (s, 1H), 7.36 - 7.19 (m, 10H), 5.23 (s, 2H), 4.05 (s, 2H), 3.92 (s, 3H).
Purity 97% (HPLC)

### BTY_A3.14.21

### 2-({3-[(2-Chlorophenoxy)methyl]-4-methoxybenzoyl}amino)-5-isopropyl-3-thiophenecarboxamide

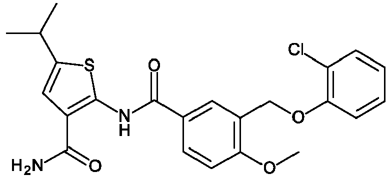

The title compound was synthesized starting from methyl 3-(bromomethyl)-4-methoxybenzoate which reacted with 2-chlorophenol using method B followed by hydrolysis of the ester product using method C to afford 3-[(2-chlorophenoxy)methyl]-4-methoxybenzoic acid. This acid was reacted with 2-amino-5-isopropyl-3-thiophenecarboxamide using method A to give the final compound.
MS m/z: 459.97 [M-H]⁻
¹H NMR (400 MHz, DMSO) δ 13.20 (s, 1H), 8.05 (s, 1H), 7.90 (d, *J* = 1.4 Hz, 1H), 7.80 (s, 1H), 7.50-7.00 (m, 7H), 5.20 (s, 2H), 4.00 (s, 3H), 3.10-3.00 (m, 1H), 1.40-1.30 (m, 6H).
Purity 95% (HPLC)

### BTY_A3.14.22

### 5-Benzyl-2-{3-[(2,4-dichlorophenoxy)methyl]-4-methoxybenzamido}thiophene-3-carboxamide

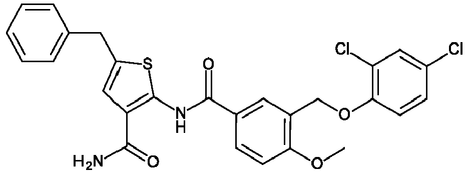

The title compound was synthesized starting from methyl 3-(bromomethyl)-4-methoxybenzoate which reacted with 2,4-dichlorophenol using method B followed by hydrolysis of the ester product using method C to afford 3-[(2,4-dichlorophenoxy)methyl]-4-methoxybenzoic acid. This acid was reacted with 2-amino-5-benzylthiophene-3-carboxamide using method A to give the final compound.
MS m/z: 539.30 [M-H]⁻
¹H NMR (400 MHz, DMSO) δ 13.25 (s, 1H), 8.01 (d, *J* = 2.3 Hz, 1H), 7.92 (s, 1H), 7.85 (dd, *J* = 8.7, 2.4 Hz, 1H), 7.57 (d, *J* = 2.6 Hz, 1H), 7.52 (s, 1H), 7.38 - 7.19 (m, 9H), 5.20 (s, 2H), 4.04 (s, 2H), 3.91 (s, 3H).
Purity 99% (HPLC)

### BTY_A3.14.23

### 2-[3-(2-Chlorobenzyloxy)-4-methoxybenzamido]-5-benzylthiophene-3-carboxamide

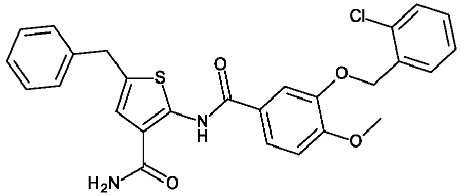

The title compound was synthesized from 3-hydroxy-4-methoxybenzoic acid which was converted to its methyl ester using method D. Methyl 3-hydroxy-4-methoxybenzoate reacted with 1-(bromomethyl)-2-chlorobenzene using method B followed by hydrolysis of the ester product using method C to afford 3-(2-chlorobenzyloxy)-4-methoxybenzoic acid. This acid was reacted with 2-amino-5-benzylthiophene-3-carboxamide using method A to give the final compound.
MS m/z: 505.22 [M-H]⁻
¹H NMR (400 MHz, DMSO) δ 13.27 (s, 1H), 7.92 (s, 1H), 7.62 - 7.45 (m, 5H), 7.41 - 7.36 (m, 2H), 7.34 - 7.18 (m, 7H), 5.20 (s, 2H), 4.04 (s, 2H), 3.85 (s, 3H).
Purity 96% (HPLC)

### BTY_A3.14.24

### N-(5-Benzyl-3-carbamoylthiophen-2-yl)-5-[(2,4-dichlorophenoxy)methyl]thiophene-2-carboxamide

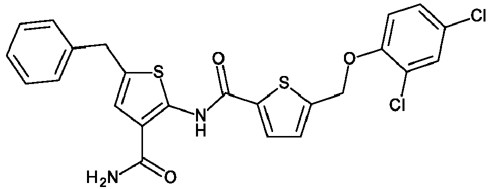

The title compound was synthesized from methyl 5-(bromomethyl)thiophene-2-carboxylate ¹ which reacted with 2,4-dichlorophenol using method B followed by hydrolysis of the ester product using method C to afford 5-[(2,4-dichlorophenoxy)methyl]thiophene-2-carboxylic acid. This acid was reacted with 2-amino-5-benzylthiophene-3-carboxamide using method A to give the final compound.
MS m/z: 515.08 [M-H]⁻
¹H NMR (400 MHz, DMSO) δ 13.23 (s, 1H), 7.95 (s, 1H), 7.58 (dd, *J* = 5.7, 3.2 Hz, 2H), 7.42 - 7.18 (m, 9H), 5.47 (s, 2H), 4.04 (s, 2H).
Purity 100% (HPLC)

### BTY_A3.14.25

### 5-Benzyl-2-{3-[(2,4-dichlorophenoxy)methyl]benzamido}thiophene-3-carboxamide

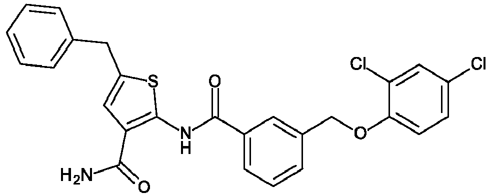

The title compound was synthesized starting from methyl 3-(bromomethyl)benzoate which reacted with 2,4-dichlorophenol using method B followed by hydrolysis of the ester product using method C to afford 3-[(2,4-dichlorophenoxy)methyl]benzoic acid. This acid was reacted with 2-amino-5-benzylthiophene-3-carboxamide using method A to give the final compound. MS m/z: 509.39 [M-H]⁻
¹H NMR (400 MHz, DMSO) δ 13.37 (s, 1H), 8.01-7.92 (m, 2H), 7.81 (d, *J* = 7.8 Hz, 1H), 7.72 (d, *J* = 7.8 Hz, 1H), 7.66-7.52 (m, 3H), 7.41 - 7.19 (m, 8H), 5.32 (s, 2H), 4.06 (s, 2H).
Purity 99% (HPLC)

### BTY_A3.14.27

### 5-Benzyl-2-{3-[(3-chlorophenoxy)methyl]-4-methoxybenzamido}thiophene-3-carboxamide

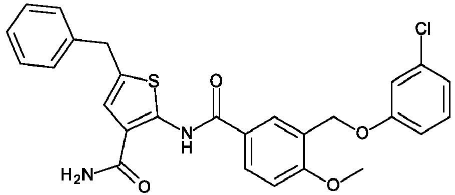

The title compound was synthesized starting from methyl 3-(bromomethyl)-4-methoxybenzoate which reacted with 3-chlorophenol using method B followed by hydrolysis of the ester product using method C to afford 3-[(3-chlorophenoxy)methyl]-4-methoxybenzoic acid. This acid was reacted with 2-amino-5-benzylthiophene-3-carboxamide using method A to give the final compound.
MS m/z: 505.54 [M-H]⁻
¹H NMR (400 MHz, DMSO) δ 13.29 (s, 1H), 7.97 - 7.83 (m, 3H), 7.52 (d, *J* = 8.9 Hz, 1H), 7.34 - 7.19 (m, 8H), 7.11 (t, *J* = 2.2 Hz, 1H), 7.00 (dd, *J* = 8.2, 2.2 Hz, 2H), 5.13 (s, 2H), 4.04 (s, 2H), 3.91 (s, 3H).
Purity 94% (HPLC)

### BTY_A3.14.28

### 5-Benzyl-2-{3-[(4-chlorophenoxy)methyl]-4-methoxybenzamido}thiophene-3-carboxamide

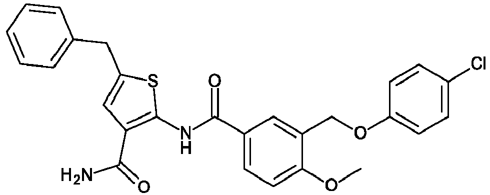

The title compound was synthesized starting from methyl 3-(bromomethyl)-4-methoxybenzoate which reacted with 4-chlorophenol using method B followed by hydrolysis of the ester product using method C to afford 3-[(4-chlorophenoxy)methyl]-4-methoxybenzoic acid. This acid was reacted with 2-amino-5-benzylthiophene-3-carboxamide using method A to give the final compound.
MS m/z: 505.46 [M-H]⁻
¹H NMR (400 MHz, DMSO) δ 13.29 (s, 1H), 7.97 - 7.83 (m, 3H), 7.55 (s, 1H), 7.36 - 7.19 (m, 9H), 7.08 - 7.02 (m, 2H), 5.10 (s, 2H), 4.04 (s, 2H), 3.91 (s, 3H).
Purity 99% (HPLC)

### BTY_A3.14.30

### 5-Benzyl-2-{3-[(2,6-dichlorophenoxy)methyl]-4-methoxybenzamido}thiophene-3-carboxamide

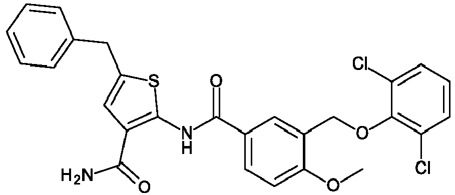

The title compound was synthesized starting from methyl 3-(bromomethyl)-4-methoxybenzoate which reacted with 2,6-dichlorophenol using method B followed by hydrolysis of the ester product using method C to afford 3-[(2,6-dichlorophenoxy)methyl]-4-methoxybenzoic acid. This acid was reacted with 2-amino-5-benzylthiophene-3-carboxamide using method A to give the final compound.
MS m/z: 539.41 [M-H]⁻
¹H NMR (400 MHz, DMSO) δ 13.27 (d, *J* = 28.1 Hz, 1H), 8.09 (d, *J* = 2.3 Hz, 1H), 7.95 - 7.86 (m, 2H), 7.56 (s, 1H), 7.51 - 7.48 (m, 2H), 7.35 - 7.15 (m, 8H), 5.09 (s, 2H), 4.05 (s, 2H), 3.85 (s, 3H).
Purity 95% (HPLC)

### BTY_A3.14.33

### 2-{2-[4-(2-Chlorobenzyl)piperazin-l-yl]acetamido}-5-benzylthiophene-3-carboxamide

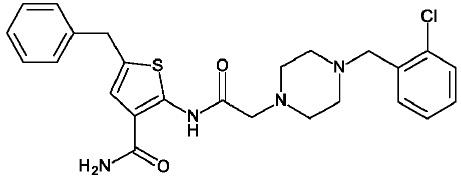

The title compound was synthesized starting from 2-chlorobenzyl bromide through reaction with piperazine using method E to give 1-(2-chlorobenzyl)piperazine, which was then reacted with 5-benzyl-2-(2-chloroacetamido)thiophene-3-carboxamide using method E to give the final compound.
MS m/z: 483.50 [M+H]⁺
¹H NMR (400 MHz, DMSO) δ 12.72 (s, 1H), 7.72 (s, 1H), 7.49 - 7.38 (m, 3H), 7.34 - 7.16 (m, 8H), 4.00 (s, 2H), 3.54 (s, 2H), 3.17 (s, 2H), 2.60-2.40 (m, 8H).
Purity 98% (HPLC)

### BTY_A3.14.34

### 2-{2-[4-(3,4-Dichlorobenzyl)piperazin-1-yl]acetamido}-5-benzylthiophene-3-carboxamide

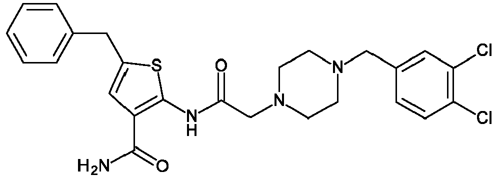

The title compound was synthesized starting from 3,4-dichlorobenzyl bromide through reaction with piperazine using method E to give 1-(3,4-dichlorobenzyl)piperazine, which was then reacted with 5-benzyl-2-(2-chloroacetamido)thiophene-3-carboxamide using method E to give the final compound.
MS m/z: 517.40 [M+H]⁺
¹H NMR (400 MHz, DMSO) δ 12.71 (s, 1H), 7.71 (s, 1H), 7.58 - 7.49 (m, 3H), 7.42 (s, 1H), 7.33 - 7.16 (m, 6H), 4.00 (s, 2H), 3.45 (s, 2H), 3.16 (s, 2H), 2.60-2.40 (m, 8H).
Purity 98% (HPLC)

### BTY_A3.14.35

### 2-{2-[4-(3,4-Dichlorobenzyl)piperazin-1-yl]acetamido}thiophene-3-carboxamide

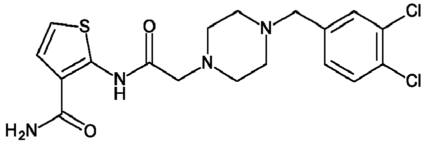

The title compound was synthesized starting from 3,4-dichlorobenzyl bromide through reaction with piperazine using method E to give 1-(3,4-dichlorobenzyl)piperazine, which was then reacted with 2-(2-chloroacetamido)thiophene-3-carboxamide using method E to give the final compound.
MS m/z: 427.30 [M+H]⁺
¹H NMR (400 MHz, DMSO) δ 12.78 (s, 1H), 7.79 (s, 1H), 7.56 - 7.53 (m, 2H), 7.50 (s, 1H), 7.38 (d, *J* = 5.8 Hz, 1H), 7.31 (dd, *J* = 8.2, 2.0 Hz, 1H), 6.95 - 6.91 (m, 1H), 3.46 (s, 2H), 3.21 (s, 2H), 2.60-2.40 (m, 8H).
Purity 99% (HPLC)

### BTY_A3.14.37

### 2-({4-[(2-Bromophenoxy)methyl]benzoyl}amino)-5-isopropyl-3-thiophenecarboxamide

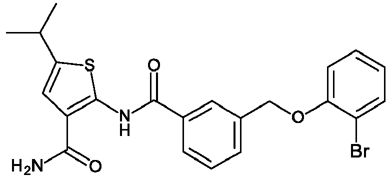

The title compound was synthesized starting from methyl 3-(bromomethyl)benzoate which reacted with 2-bromophenol using method B followed by hydrolysis of the ester product using method C to afford 3-[(2-bromophenoxy)methyl]benzoic acid. This acid was reacted with 2-amino-5-isopropyl-3-thiophenecarboxamide using method A to give the final compound.
MS m/z: 472.39 [M-H]⁻
¹H NMR (400 MHz, DMSO) δ 13.20 (s, 1H), 8.00 (d, *J* = 8.0 Hz, 2H), 7.90-7.55 (m, 3H), 7.45-6.90 (m, 6 H), 5.30 (s, 2H), 3.10-2.90 (m, 1H), 1.20 (s, 6H).
Purity 94% (HPLC)

### BTY_A3.14.39

### 2-({3-[(4-Bromophenoxy)methyl]-4-methoxybenzoyl}amino)-5-isopropyl-3-thiophenecarboxamide

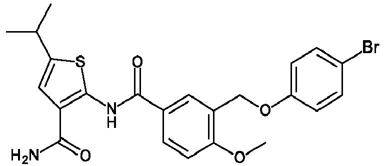

The title compound was synthesized starting from methyl 3-(bromomethyl)-4-methoxybenzoate which reacted with 4-bromophenol using method B followed by hydrolysis of the ester product using method C to afford 3-[(4-bromophenoxy)methyl]-4-methoxybenzoic acid. This acid was reacted with 2-amino-5-isopropyl-3-thiophenecarboxamide using method A to give the final compound.
MS m/z: 502.42 [M-H]⁻
¹H NMR (400 MHz, DMSO) δ 13.10 (s, 1H), 8.00 (s, 1H), 7.90 (d, *J* = 1.0 Hz, 1H), 7.60-7.45 (m, 4H), 7.30-6.90 (m, 4H), 5.10 (s, 2H), 4.10 (s, 3H), 3.10-2.90 (m, 1H), 1.20 (s, 6H).
Purity 93% (HPLC)

### Biological efficacy

In view of recent studies revealing a conserved inhibitory activity of BTYNB on tumor cell growth and metastasis in tumor cell and mouse models (15), novel IGF2BP1 inhibitors were synthesized, analytically characterized and tested in cellulo. All novel inhibitors were explored for their cytotoxic activity in ovarian cancer-derived ES-2 cells. In these cell models, BTYNB was prior shown to impair cell vitality and metastatic potential *in vitro* and cancer mouse models (15). For the respective studies ES-2 cells were plated and exposed to increasing concentration of the compounds and cell vitality was monitored by determining cell confluency relative to DMSO controls using an IncuCyte cell imaging system, essentially as described recently (15).
For determining the impact of the developed compounds on ES-2 cell vitality, cells were cultured in Dulbecco's modified eagle medium (DMEM) containing phenol red, 4.5 g/L D-Glucose, 0.11 g/L sodium pyruvate, 10% (v/v) fetal bovine serum (FBS) and 2 mM GlutaMAX (Sigma-Aldrich, Darmstadt, Germany). Cells were maintained in under humidified conditions at 37 °C, 5% CO₂. For cytotoxicity studies, cells were seeded in 96-well flat-bottom plates at 2 x 10³ cells per well in 100 µL supplemented DMEM and incubated for 24 h prior to addition of BTYNB derivatives. Compounds were analyzed at eleven concentrations using a two-fold dilution with an initial compound concentration of 20µM in DMSO (solvent). Cells treated with 0.002% (v/v) DMSO served as controls. Cytotoxicity of compound concentrations was determined 72 h post compound application by monitoring cell confluency using an IncuCyte Systems for Live-Cell Imaging. Cell confluency was determined with the IncuCyte 2019B Rev2 software. Confluency of controls was set to 1 (100%) and altered confluency at distinct compound concentrations was calculated as relative confluency. The mean relative confluency at compound concentrations was analyzed in three independent studies with three technical replicates each. The mean values of three analyses were plotted against the logarithmic concentration of compounds and fitted by the non-linear module [Sigmoidal, 4PL, X is log(concentration)] using GraphPad Prism V9(86) for macOS. In Table 1 compounds with EC₅₀ values, indicating the potency of the 58 developed compounds to impair cell vitality, are listed. Out of the 58 compounds, potency higher than observed for BTYNB (average EC₅₀ = 5.9 µM; determined in 11 analyses with three technical replicates each) was observed for 51 derivatives (Table 2).

**Table 2: EC-values of the synthesized compounds of formula (I)**

| **Ex. No.** | **Code** | **EC₅₀, µM** | **Ex. No.** | **Code** | **EC₅₀, µM** |
|---|---|---|---|---|---|
| 1 | BTY_A2 | 5.612 | 30 | BTY_A3.64 | 1.467 |
| 2 | BTY_A3 | 2.059 | 31 | BTY_A3.14 | 0.671 |
| 3 | BTY_A8 | 0.476 | 32 | BTY_A3.14.2 | 1.904 |
| 4 | BTY_A10 | 0.250 | 33 | BTY_A3.14.3 | 5.063 |
| 5 | BTY_A13 | 1.117 | 34 | BTY_A3.14.4 | 1.160 |
| 6 | BTY_A14 | 4.046 | 35 | BTY_A3.14.5 | 1.343 |
| 7 | BTY_A18 | 4.841 | 36 | BTY_A3.14.6 | 1.661 |
| 8 | BTY_A3.2 | 2.827 | 37 | BTY_A3.14.36 | 2.270 |
| 9 | BTY_A3.5 | 1.329 | 38 | BTY_A3.14.9 | 1.304 |
| 10 | BTY_A3.11 | 3.234 | 39 | BTY_A3.14.10 | 6.210 |
| 11 | BTY_A3.22 | 5.183 | 40 | BTY_A3.14.11 | 2.421 |
| 12 | BTY_A3.26 | 1.877 | 41 | BTY_A3.14.12 | 1.936 |
| 13 | BTY_A3.29 | 2.296 | 42 | BTY_A3.14.14 | 2.449 |
| 14 | BTY_A3.30 | 10.500 | 43 | BTY_A3.14.15 | 1.193 |
| 15 | BTY_A3.34 | 2.954 | 44 | BTY_A3.14.16 | 0.439 |
| 16 | BTY_A3.35 | 5.844 | 45 | BTY_A3.14.17 | 0.401 |
| 17 | BTY_A3.39 | 0.675 | 46 | BTY_A3.14.21 | 2.227 |
| 18 | BTY_A3.40 | 5.145 | 47 | BTY_A3.14.22 | 0.293 |
| 19 | BTY_A3.44 | 2.947 | 48 | BTY_A3.14.23 | 1.095 |
| 20 | BTY_A3.46 | 1.223 | 49 | BTY_A3.14.24 | 0.560 |
| 21 | BTY_A3.47 | 0.993 | 50 | BTY_A3.14.25 | 0.047 |
| 22 | BTY_A3.49 | 6.458 | 51 | BTY_A3.14.27 | 0.355 |
| 23 | BTY_A3.50 | 2.992 | 52 | BTY_A3.14.28 | 1.022 |
| 24 | BTY_A3.51 | 9.582 | 53 | BTY_A3.14.30 | 2.596 |
| 25 | BTY_A3.52 | 3.168 | 54 | BTY_A3.14.33 | 7.107 |
| 26 | BTY_A3.54 | 1.098 | 55 | BTY_A3.14.34 | 4.093 |
| 27 | BTY_A3.55 | 1.662 | 56 | BTY_A3.14.35 | 0.781 |
| 28 | BTY_A3.57 | 1.876 | 57 | BTY_A3.14.37 | 3.743 |
| 29 | BTY_A3.63 | 6.272 | 58 | BTY_A3.14.39 | 8.345 |

### References

1. Roos, M., Pradere, U., Ngondo, R.P., Behera, A., Allegrini, S., Civenni, G., Zagalak, J.A., Marchand, J.R., Menzi, M., Towbin, H. et al. (2016) A Small-Molecule Inhibitor of Lin28. ACS Chem Biol, 11, 2773-2781.
2. Wang, L., Rowe, R.G., Jaimes, A., Yu, C., Nam, Y., Pearson, D.S., Zhang, J., Xie, X., Marion, W., Heffron, G.J. et al. (2018) Small-Molecule Inhibitors Disrupt let-7 Oligouridylation and Release the Selective Blockade of let-7 Processing by LIN28. Cell Rep, 23, 3091-3101.
3. Mahapatra, L., Andruska, N., Mao, C., Le, J. and Shapiro, D.J. (2017) A Novel IMP1 Inhibitor, BTYNB, Targets c-Myc and Inhibits Melanoma and Ovarian Cancer Cell Proliferation. Transl Oncol, 10, 818-827.
4. Minuesa, G., Albanese, S.K., Xie, W., Kazansky, Y., Worroll, D., Chow, A., Schurer, A., Park, S.M., Rotsides, C.Z., Taggart, J. et al. (2019) Small-molecule targeting of MUSASHI RNA-binding activity in acute myeloid leukemia. Nat Commun, 10, 2691.
5. Bell, J.L., Wachter, K., Muhleck, B., Pazaitis, N., Kohn, M., Lederer, M. and Huttelmaier, S. (2013) Insulin-like growth factor 2 mRNA-binding proteins (IGF2BPs): post-transcriptional drivers of cancer progression? Cell Mol Life Sci, 70, 2657-2675.
6. Muller, S., Glass, M., Singh, A.K., Haase, J., Bley, N., Fuchs, T., Lederer, M., Dahl, A., Huang, H., Chen, J. et al. (2019) IGF2BP1 promotes SRF-dependent transcription in cancer in a m6A- and miRNA-dependent manner. Nucleic Acids Res, 47, 375-390.
7. Muller, S., Bley, N., Glass, M., Busch, B., Rousseau, V., Misiak, D., Fuchs, T., Lederer, M. and Huttelmaier, S. (2018) IGF2BP1 enhances an aggressive tumor cell phenotype by impairing miRNA-directed downregulation of oncogenic factors. Nucleic Acids Res, 46, 6285-6303.
8. Gutschner, T., Hammerle, M., Pazaitis, N., Bley, N., Fiskin, E., Uckelmann, H., Heim, A., Grobeta, M., Hofmann, N., Geffers, R. et al. (2014) Insulin-like growth factor 2 mRNA-binding protein 1 (IGF2BP1) is an important protumorigenic factor in hepatocellular carcinoma. Hepatology, 59, 1900-1911.
9. Zirkel, A., Lederer, M., Stohr, N., Pazaitis, N. and Huttelmaier, S. (2013) IGF2BP1 promotes mesenchymal cell properties and migration of tumor-derived cells by enhancing the expression of LEF1 and SNAI2 (SLUG). Nucleic Acids Res, 41, 6618-6636.
10. Bell, J.L., Turlapati, R., Liu, T., Schulte, J.H. and Huttelmaier, S. (2015) IGF2BP1 harbors prognostic significance by gene gain and diverse expression in neuroblastoma. J Clin Oncol, 33, 1285-1293.
11. Stohr, N., Kohn, M., Lederer, M., Glass, M., Reinke, C., Singer, R.H. and Huttelmaier, S. (2012) IGF2BP1 promotes cell migration by regulating MK5 and PTEN signaling. Genes Dev, 26, 176-189.
12. Stohr, N. and Huttelmaier, S. (2012) IGF2BP1: a post-transcriptional "driver" of tumor cell migration. Cell Adh Migr, 6, 312-318.
13. Kobel, M., Weidensdorfer, D., Reinke, C., Lederer, M., Schmitt, W.D., Zeng, K., Thomssen, C., Hauptmann, S. and Huttelmaier, S. (2007) Expression of the RNA-binding protein IMP1 correlates with poor prognosis in ovarian carcinoma. Oncogene, 26, 7584-7589.
14. Busch, B., Bley, N., Muller, S., Glass, M., Misiak, D., Lederer, M., Vetter, M., Strauss, H.G., Thomssen, C. and Huttelmaier, S. (2016) The oncogenic triangle of HMGA2, LIN28B and IGF2BP1 antagonizes tumor-suppressive actions of the let-7 family. Nucleic Acids Res, 44, 3845-3864.
15. Muller, S., Bley, N., Busch, B., Glass, M., Lederer, M., Misiak, C., Fuchs, T., Wedler, A., Haase, J., Bertoldo, J.B. et al. (2020) The oncofetal RNA-binding protein IGF2BP1 is a druggable, post-transcriptional super-enhancer of E2F-driven gene expression in cancer. Nucleic Acids Res, 48, 8576-8590.
16. Kumar, B. R. P.; Nanjan, M. J. Novel glitazones: Design, synthesis, glucose uptake and structure-activity relationships. Bioorg. Med. Chem. Lett. 2010, 20, 1953-1956.
17. Tynebor, R. M.; Chen, M.-H.; Natarajan, S. R.; O'Neill, E. A.; Thompson, J. E.; Fitzgerald, C. E.; O'Keefe, S. J.; Doherty, J. B. Synthesis and biological activity of pyridopyridazin-6-one p38α MAP kinase inhibitors. Part 2. Bioorg. Med. Chem. Lett. 2012, 22, 5979-5983.
18. Rückle, T.; Biamonte, M.; Grippi-Vallotton, T.; Arkinstall, S.; Cambet, Y.; Camps, M.; Chabert, C.; Church, D. J.; Halazy, S.; Jiang, X.; Martinou, I.; Nichols, A.; Sauer, W.; Gotteland, J.-P. Design, Synthesis, and Biological Activity of Novel, Potent, and Selective (Benzoylaminomethyl)thiophene Sulfonamide Inhibitors of c-Jun-N-Terminal Kinase. J. Med. Chem. 2004, 47, 6921-6934.

## Claims

1. A compound of formula (I) 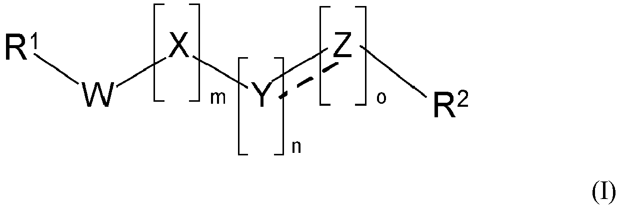 or a pharmaceutically acceptable salt, solvate or polymorph thereof, including all tautomers and stereoisomers thereof,
wherein:
W is selected from the group consisting of NH, O, and CR³R⁴;
Y is selected from the group consisting of C=O, CR³R⁴ and CR³;
Z is CR³ or CR³R⁴,
any of m, n, and o is independently selected from 0 and 1;
R¹ is selected from the group consisting of aryl, heteroaryl and heteroaryl fused to carbocyclyl;
wherein R¹ may be optionally substituted by one or more groups selected from the group consisting of alkyl, alkoxy, haloalkyl, thioalkyl, carbamoyl, aryl, alkylaryl, heteroaryl fused to aryl, heteroaryl fused to heteroaryl and halo; wherein said carbamoyl group may optionally be further substituted by alkyl and wherein said thioalkyl and heteroaryl groups may optionally be further substituted by one or more groups selected from oxo, alkylaryl, and heteroaryl.
R² is selected from the group consisting of alkyl, heteroalkyl, aryl, aryl fused to heterocyclyl, heteroaryl and heterocyclyl;
wherein R² may be optionally substituted by one or more groups selected from the group consisting of alkyl, alkoxy, aryl, alkylaryl, alkoxy-aryl, aryl-amido, alkoxy-heteroaryl, halo and nitro, wherein said aryl and heteroaryl groups may optionally be further substituted by one or more groups selected from halo and alkoxy;
R³ is hydrogen;
R⁴ is hydrogen or alkyl, such as methyl;
with the proviso that
a) when R¹ is aryl or heteroaryl, R1 is substituted by one or more substituents independently selected from the group consisting of benzyl, alkyl, and amido;
b) the bond between Y and Z is a double bond, when m, n and o are each 1;
c) the compound of formula (I) is not a compound selected from the group consisting of:
N-(5-{[1-(4-oxo-3,4-dihydro-2-quinazolinyl)ethyl]thio}-1,3,4-thiadiazol-2-yl)benzamide;
2-benzyl-6-methylthieno[2,3-d]pyrimidin-4(3H)-one;
N-[3-(aminocarbonyl)-2-thienyl]-2-thiophenecarboxamide;
2-{[(5-ethyl-3-thienyl)carbonyl]amino}-3-thiophenecarboxamide;
2-({3-[4-(benzyloxy)-3-methoxyphenyl]acryloyl}amino)-benzamide;
2-{[(5-chloro-2-thienyl)carbonyl]amino}-5,6-dihydro-4H-cyclopenta[b]thiophene-3-carboxamide;
2-{[(5-chloro-2-thienyl)carbonyl]amino}-4,5,6,7-tetrahydro-1-benzothiophene-3-carboxamide;
2-{[(3,4-dichlorophenyl)acetyl]amino}-5-isopropyl-3-thiophenecarboxamide;
2-[(5-chlorothiophen-2-yl)methoxy]benzamide;
N-[2-(aminocarbonyl)phenyl]-5-methyl-2- thiophenecarboxamide;
5-bromo-N-(2-nitrophenyl)-2-thiophenecarboxamide; and
N-[3-(aminocarbonyl)-5-benzyl-4-methyl-2-thienyl]-2-thiophenecarboxamide;
d) the compound o formula (I) is not the compound

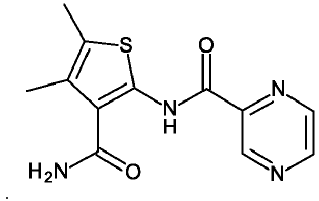

2. The compound of formula (I) according to claim 1, wherein the compound of formula (I) is one of formulae (Ia) to (Ih):

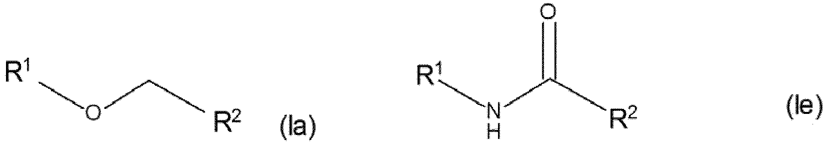

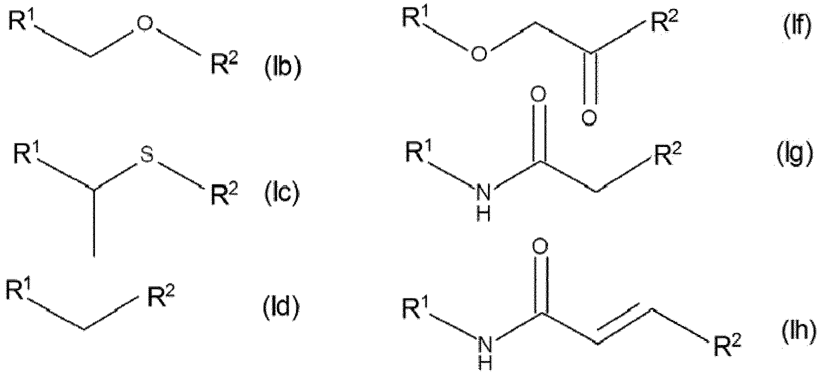

3. The compound of formula (I) of claim 1 or 2, wherein R¹ is selected from the group consisting of phenyl, thiophenyl, thiadiazolyl, such as 1,3,4-thiadiazolyl, 4,5,6,7-tetrahydro-1-benzothiophene, thieno[2,3-d]pyrimidin-4(3H)-one and 5,6-dihydro-4H-cyclopenta[b]thiophene, which groups may optionally be further substituted as claimed in claim 1.

4. The compound according to any one of claims 1 to 3, wherein R¹ is thiophenyl, phenyl, 4,5,6,7-tetrahydro-1-benzothiophene, any of which may optionally be further substituted by one or more groups selected from isopropyl, carbamoyl, methylcarbamoyl, chloro, benzyl and 4(3H)-quinazolinone, thieno[2,3-d]pyrimidinone which may further be substituted by one or more of methoxy and benzyl.

5. The compound formula (I) according to any one of the preceding claims, wherein R¹ is selected from the group consisting of benzamide, 5-chlorobenzamide, thiophene-3-carboxamide, 3-(aminocarbonyl)-5-benzyl-2-thienyl, phenyl-4(3H)-quinazolinone, 3-methoxyphenyl-4(3H)-quinazolinone, 6-benzyl-2-(4-methoxyphenyl)thieno[2,3-d]pyrimidin-4(3H)-one, 4,5,6,7-tetrahydro-1-benzothiophene-3-carboxamide, 5-isopropyl-3-thiophenecarboxamide, 2-(methylcarbamoyl)phenyl and 6-methylthieno[2,3-d]pyrimidin-4(3H)-one.

6. The compound of formula (I) according to any one of the preceding claims, wherein
i. W is O, X is CH₂, m is 1, n is 0 and o is 0; or
ii. W is CH₂, X is O, m is 1, n is 0 and o is 0;
iii. W is CR³R⁴, X is S, m is 1, n is 0 and o is 0, wherein R³ is hydrogen and R⁴ is methyl; or
iv. W is CH₂, X is 0, m is 1, n is 0 and o is 0; or
v. W is NH, X is C=O, m is 1, n is 0 and o is 0; or
vi. W is O, X is CH₂, Y is C=O, m is 1, n is 1 and o is 0; or
vii. W is NH, X is C=O, Y is CH₂, m is 1, n is 1 and o is 0; or
viii. W is NH, X is C=O, Y is CH, Z is CH, m is 1, n is 1 and o is 1.

7. The compound formula (I) according to any one of the preceding claims, wherein R² is selected from the group consisting of phenyl, benozodioxole, piperazinyl, furanyl and thiophenyl, which groups may optionally be further substituted as claimed in claim 1.

8. The compound formula (I) according to any one of the preceding claims, wherein R² is selected from the group consisting of phenyl, 1,3- benzodioxole, piperazin-1-yl, furan-2-yl, thiophen-2-yl and thiophen-3-yl, which groups may optionally be substituted by one or more groups selected from alkoxy, aryl, halo, nitro, alkylaryl, alkoxyaryl, wherein said aryl groups may optionally be further substituted by one or more halo groups.

9. The compound formula (I) according to any one of the preceding claims, wherein said compound is a compound of formula (II) 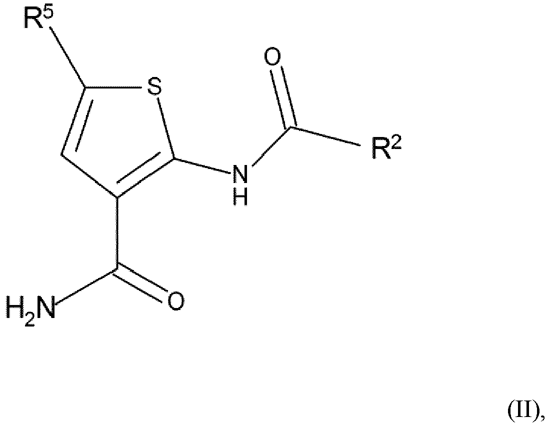 wherein R² is defined in one of the preceding claims and R⁵ is selected from hydrogen, benzyl and alkyl;
or wherein said compound is a compound of formula (IIa): 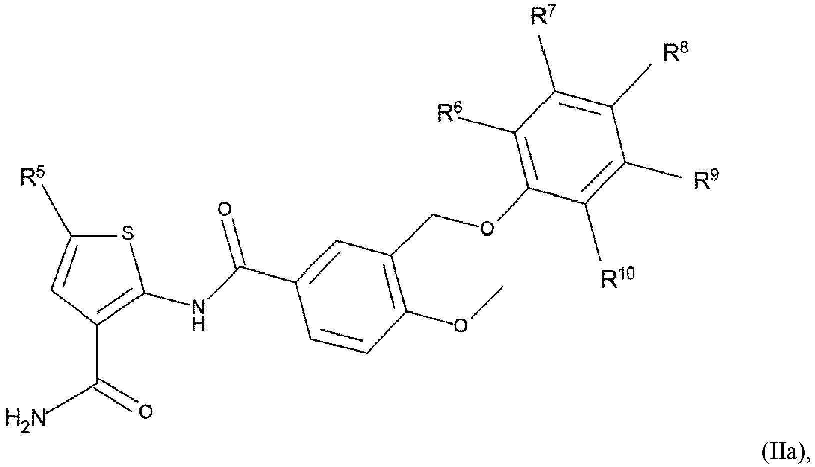 wherein
R⁵ is selected from hydrogen, benzyl and alkyl, such as isopropyl; and
R⁶, R⁷, R⁸, R⁹, and R¹⁰ are independently selected from the group consisting of hydrogen, nitro and halo, such as bromo, chloro or fluoro;
or wherein said compound is a compound of formula (III): 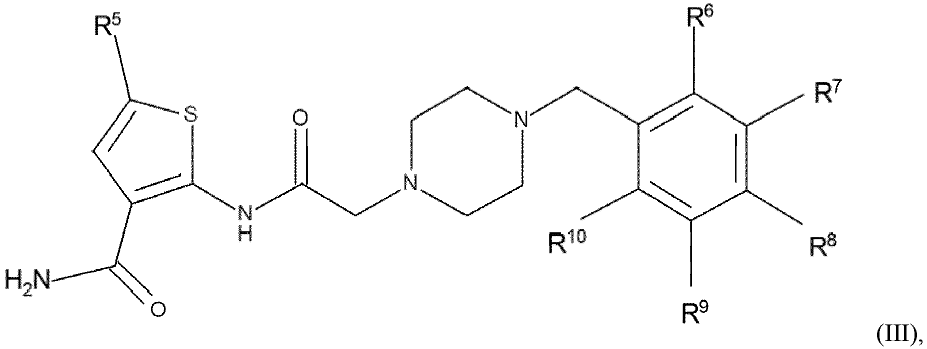 wherein
R⁵ is selected from hydrogen and benzyl; and
R⁶, R⁷, R⁸, R⁹, and R¹⁰ are independently selected from the group consisting of hydrogen and halo, such as bromo, chloro or fluoro;
or wherein said compound is a compound of formula (IV): 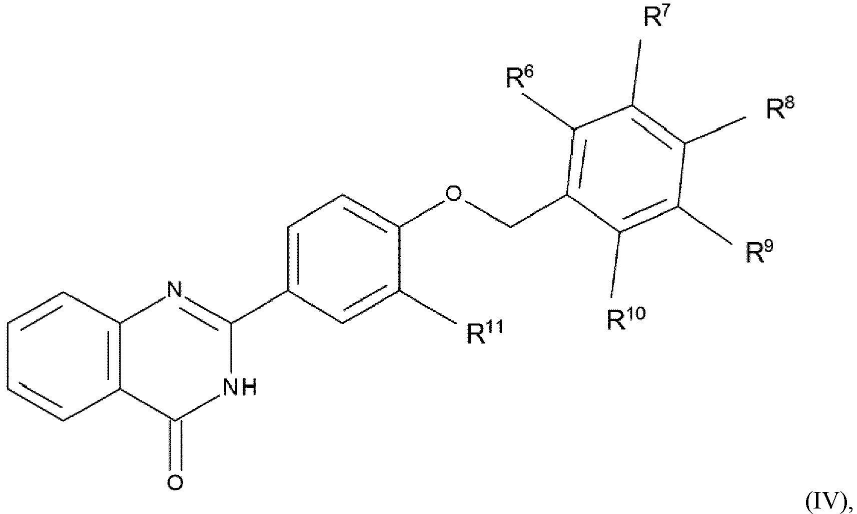 wherein
R⁶, R⁷, R⁸, R⁹, and R¹⁰ are independently selected from the group consisting of hydrogen, nitro and halo, such as bromo, chloro or fluoro; and
R¹¹ is selected from hydrogen and alkoxy;
or wherein said compound is a compound of formula (V): 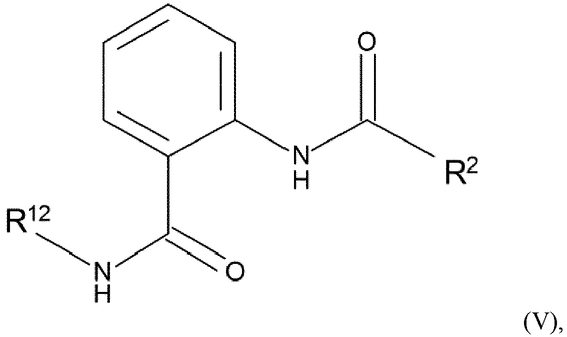 wherein
R² is as defined in claim 1; and
R¹² is hydrogen or alkyl;
or wherein said compound is a compound of formula (VI): 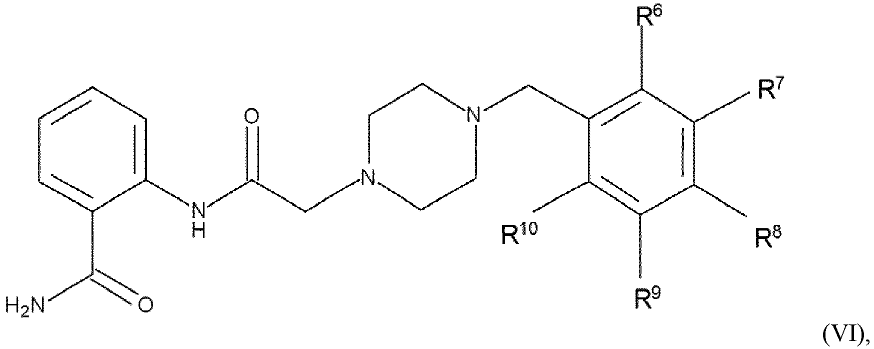 wherein
R⁶, R⁷, R⁸, R⁹, and R¹⁰ are independently selected from the group consisting of hydrogen and and halo, such as bromo, chloro or fluoro;
or wherein said compound is a compound of formula (VII): 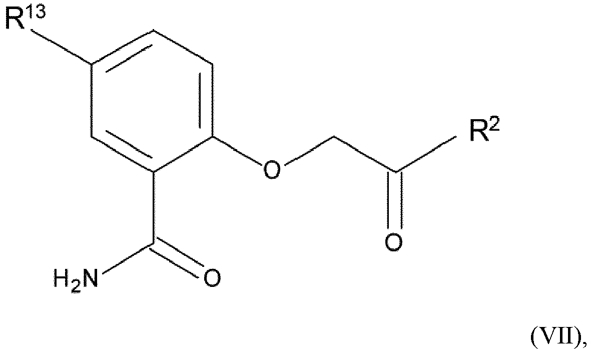 wherein
R² is as defined in claim 1; and
R¹³ is hydrogen or halo.

10. A pharmaceutical composition comprising a compound of formula (I) according to any one of the preceding claims, including the compounds of proviso c) of claim 1.

11. The compound of formula (I) or the pharmaceutical composition according to any one of the preceding claims for use in the prevention or treatment of cancer, tumor metastasis or tumor recurrence in a subject, including the compounds of provisos c) and d) of claim 1.

12. The compound of formula (I) or pharmaceutical composition for use according to any one of the preceding claims, wherein said proliferative disease is selected from malignant tumors of epithelial, mesenchymal, (neuro)-endocrine or hematopoietic origin.

13. The compound of formula (I) or pharmaceutical composition for use according to any one of the preceding claims, wherein said proliferative disease is cancer and is selected from the group consisting of malignant tumors of epithelial, mesenchymal or haemopoietic cells, breast cancer, prostate cancer, pancreatic cancer, adrenal cancer, melanoma, lung cancer, colon cancer, leukemia (a liquid or non-solid tumor), soft tissue and bone sarcomas, neuroendocrine tumors such as islet cell carcinoma or medullary carcinoma of the thyroid, squamous carcinomas (particularly of the head and neck), adenocarcinomas, gliosarcomas such as glioblastoma multiforme, liver cancer, kidney cancer, bladder cancer, stomach cancer, thyroid carcinomas of follicular origin, neuroblastoma, teratoma and ovarian carcinoma; preferably ovarian carcinoma, lung adenocarcinoma, melanoma, renal carcinoma, hepatocellular carcinoma, pancreas carcinoma and childhood-specific neuroblastoma.

14. The compound formula (I) for use or the pharmaceutical composition according to any one of the claims 10 to 13, wherein said compound of formula (I) is selected from the group consisting of:
2-[(5-chlorothiophen-2-yl)methoxy]benzamide;
N-[2-(aminocarbonyl)phenyl]-5-methyl-2-thiophenecarboxamide;
2-{3-methoxy-4-[(2-nitrobenzyl)oxy]phenyl}-4(3H)-quinazolinone;
2-{4-[(2-fluorobenzyl)oxy]phenyl}-4(3H)-quinazolinone;
6-benzyl-2-{4-methoxy-3-[(4-methoxyphenoxy)methyl]phenyl}thieno[2,3-d]pyrimidin-4(3H)-one;
N-(5-{[1-(4-oxo-3,4-dihydro-2-quinazolinyl)ethyl]thio}-1,3,4-thiadiazol-2-yl)benzamide;
2-benzyl-6-methylthieno[2,3-d]pyrimidin-4(3H)-one;
4,5-dimethyl-N-[2-(methylcarbamoyl)phenyl]thiophene-2-carboxamide;
5-chloro-2-[2-(5-chlorothiophen-2-yl)-2-oxoethoxy]benzamide;
N-[3-(aminocarbonyl)-2-thienyl]-2-thiophenecarboxamide;
N-[2-(aminocarbonyl)phenyl]-1-(2-thienylcarbonyl)-4-piperidinecarboxamide;
N-[2-(aminocarbonyl)phenyl]-3,4-dichlorobenzamide;
N-(2-carbamoylphenyl)-5-chlorothiophene-2-carboxamide;
N-(2-carbamoylphenyl)benzamide;
2-{[(5-ethyl-3-thienyl)carbonyl]amino}-3-thiophenecarboxamide;
N-[2-(aminocarbonyl)phenyl]-3-chloro-4-methoxybenzamide;
2-({3-[(3-chlorophenoxy)methyl]benzoyl}amino)benzamide;
N-[2-(aminocarbonyl)phenyl]-3,5-dichlorobenzamide;
2-[2-(4-bromophenyl)-2-oxoethoxy]benzamide;
2-({[4-(3,4-dichlorobenzyl)-1-piperazinyl]acetyl}amino)benzamide;
2-({[4-(2,4-dichlorobenzyl)-1-piperazinyl]acetyl}amino)benzamide;
2-({[4-(2,6-dichlorobenzyl)-1-piperazinyl]acetyl}amino)benzamide;
2-({[4-(4-chlorobenzyl)-1-piperazinyl]acetyl}amino)benzamide;
2-({[4-(2-chlorobenzyl)-1-piperazinyl]acetyl}amino)benzamide;
2-({[4-(2-chloro-4-fluorobenzyl)-1-piperazinyl]acetyl}amino)benzamide;
2-{3-[(2-chlorophenoxy)methyl]-4-methoxybenzamido}benzamide;
2-{3-[(2-chlorophenoxy)methyl]-4-methoxybenzamido}thiophene-3-carboxamide;
2-({3-[4-(benzyloxy)-3-methoxyphenyl]acryloyl}amino)benzamide;
2-{[(5-chloro-2-thienyl)carbonyl]amino}-5,6-dihydro-4H-cyclopenta[b]thiophene-3-carboxamide;
2-{[(5-chloro-2-thienyl)carbonyl]amino}-4,5,6,7-tetrahydro-1-benzothiophene-3-carboxamide;
N-[3-(aminocarbonyl)-5-benzyl-2-thienyl]-2-thiophenecarboxamide;
5-benzyl-2-{[(5-ethyl-3-thienyl)carbonyl]amino}-3-thiophenecarboxamide;
5-benzyl-2-{[(4-ethyl-5-methyl-3-thienyl)carbonyl]amino}-3-thiophenecarboxamide;
5-benzyl-2-[(2-thienylacetyl)amino]-3-thiophenecarboxamide;
N-[3-(aminocarbonyl)-5-benzyl-2-thienyl]-5-methyl-2-furamide;
N-[3-(aminocarbonyl)-5-benzyl-2-thienyl]-5-chloro-2-thiophenecarboxamide;
2-(benzoylamino)-5-benzyl-3-thiophenecarboxamide;
N-[3-(aminocarbonyl)-5-benzyl-2-thienyl]-1,3-benzodioxole-5-carboxamide;
5-benzyl-2-[(3-phenylpropanoyl)amino]-3-thiophenecarboxamide;
5-benzyl-2-[(phenoxyacetyl)amino]-3-thiophenecarboxamide;
5-benzyl-2-[(4-methoxybenzoyl)amino]-3-thiophenecarboxamide;
2-{[(3,4-dichlorophenyl)acetyl]amino}-5-isopropyl-3-thiophenecarboxamide;
5-benzyl-2-({4-[(4-bromophenoxy)methyl]benzoyl}amino)-3-thiophenecarboxamide;
5-benzyl-2-({3-[(2-chlorophenoxy)methyl]-4-methoxybenzoyl}amino)-3-thiophenecarboxamide;
5-benzyl-2-({3-[(2,3-dichlorophenoxy)methyl]-4-methoxybenzoyl}amino)-3-thiophenecarboxamide;
2-({3-[(2-chlorophenoxy)methyl]-4-methoxybenzoyl}amino)-5-isopropyl-3-thiophenecarboxamide;
5-benzyl-2-{3-[(2,4-dichlorophenoxy)methyl]-4-methoxybenzamido}thiophene-3-carboxamide;
2-[3-(2-chlorobenzyloxy)-4-methoxybenzamido]-5-benzylthiophene-3-carboxamide;
N-(5-benzyl-3-carbamoylthiophen-2-yl)-5-[(2,4-dichlorophenoxy)methyl]thiophene-2-carboxamide;
5-benzyl-2-{3-[(2,4-dichlorophenoxy)methyl]benzamido}thiophene-3-carboxamide;
5-benzyl-2-{3-[(3-chlorophenoxy)methyl]-4-methoxybenzamido}thiophene-3-carboxamide;
5-benzyl-2-{3-[(4-chlorophenoxy)methyl]-4-methoxybenzamido}thiophene-3-carboxamide;
5-benzyl-2-{3-[(2,6-dichlorophenoxy)methyl]-4-methoxybenzamido}thiophene-3-carboxamide;
2-{2-[4-(2-Chlorobenzyl)piperazin-1-yl]acetamido}-5-benzylthiophene-3-carboxamide;
2-{2-[4-(3,4-Dichlorobenzyl)piperazin-1-yl]acetamido}-5-benzylthiophene-3-carboxamide;
2-{2-[4-(3,4-Dichlorobenzyl)piperazin-1-yl]acetamido}thiophene-3-carboxamide;
2-({4-[(2-bromophenoxy)methyl]benzoyl}amino)-5-isopropyl-3-thiophenecarboxamide; and
2-({3-[(4-bromophenoxy)methyl]-4-methoxybenzoyl}amino)-5-isopropyl-3-thiophenecarboxamide.

15. A process for preparing a compound of formula (I) comprising:
(a) Preparing a compound of formula (IIa): 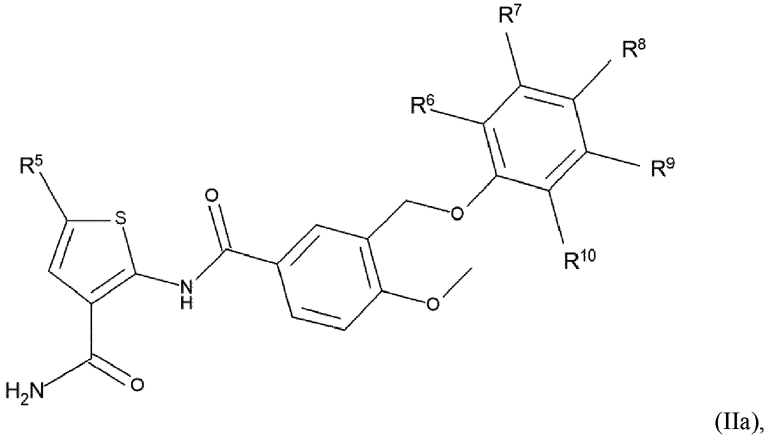 wherein R⁵, R⁶, R⁷, R⁸, R⁹, and R¹⁰ are as defined herein for compounds of formula (IIa), wherein said process comprises reacting the suitable phenol with the suitable alkyl bromide in presence of potassium carbonate followed by hydrolysis of the resulting ester using lithium hydroxide, and wherein the resulting acid is coupled with the suitable amine using thionyl chloride.
(b) Preparing a compound of formula (III): 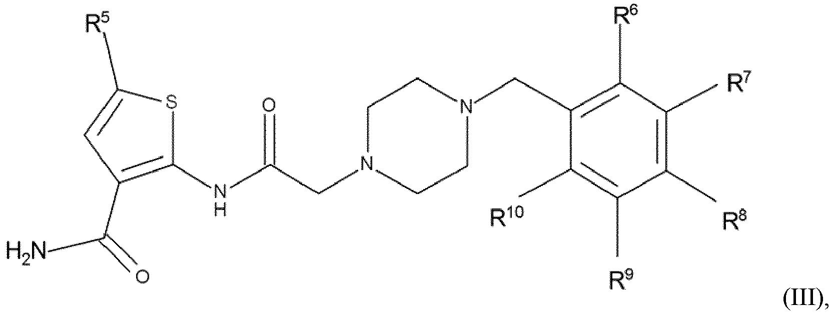 wherein
R⁵, R⁶, R⁷, R⁸, R⁹, and R¹⁰ are as defined herein for compounds of formula (III), wherein said process comprises reacting the suitable amine with chloroacetyl chloride in presence of triethylamine followed by reacting the product with the suitable piperazine derivative using potassium carbonate..
(c) Preparing a compound of formula (IV): 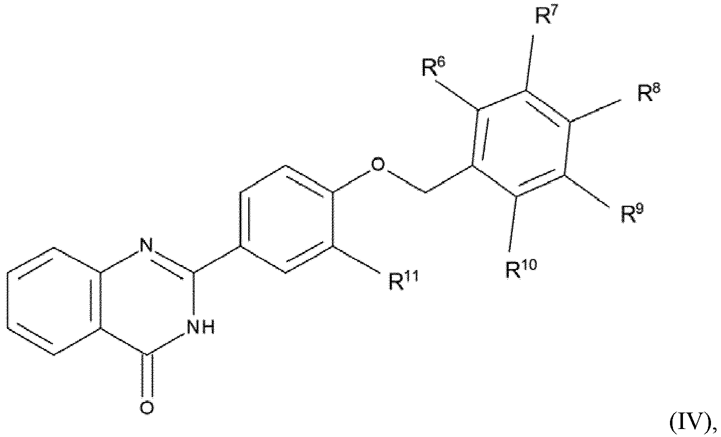 wherein R⁶, R⁷, R⁸, R⁹, and R¹⁰ and R¹¹ are as defined herein for compounds of formula (IV), wherein said process comprises reacting the suitable phenol with the suitable alkyl bromide in presence of potassium carbonate followed by reacting the resulting aldehyde with the suitable amine in dimethylsulfoxide to form the quinazolinone ring.
(d) Preparing a compound of formula (V): 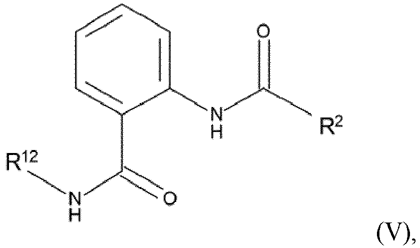 wherein
R², R¹², R¹² are as defined herein for compounds of formula (V),
Wherein said process comprises reacting the suitable carboxylic acid with the suitable amine using thionyl chloride.
(e) Preparing a compound of formula (VI): 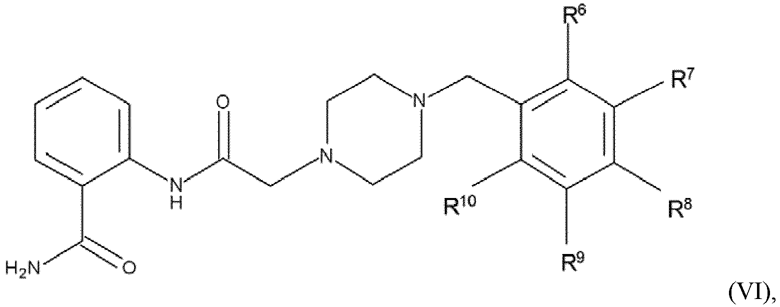 wherein
R⁶, R⁷, R⁸, R⁹, and R¹⁰ are as defined herein for compounds of formula (VI),
wherein said process comprises reacting 2-aminobenzamide with chloroacetyl chloride in presence of triethylamine followed by reacting the product with the suitable piperazine derivative using potassium carbonate..
(f) Preparing a compound of formula (VII): 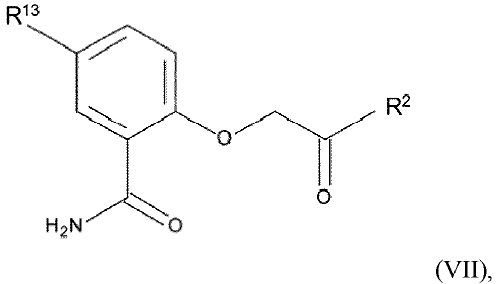 wherein
R² and R¹³ are as defined herein for compounds of formula (VII),
wherein said process comprises reacting the suitable phenol with the suitable alkyl bromide in presence of potassium carbonate..
